# EUROPÄISCHE PATENTSCHRIFT

(11) **EP 1 135 386 B1**
(45) Veröffentlichungstag und Bekanntmachung des Hinweises auf die Patenterteilung: **02.04.2003**
(21) Anmeldenummer: 99966912.0
(22) Anmeldetag: 02.12.1999
(51) Int. Cl.: C07D 409/06, C07D 401/06, C07D 417/06, A01N 43/56

(54) **VERFAHREN ZUR HERSTELLUNG VON PYRAZOLYLBENZOYLDERIVATEN SOWIE NEUE PYRAZOLYLBENZOYLDERIVATE**
METHOD FOR PRODUCING PYRAZOLYLBENZOYL DERIVATIVES AND NOVEL PYRAZOLYLBENZOYL DERIVATIVES
PROCEDE POUR LA PRODUCTION DE DERIVES DE PYRAZOLYLBENZOYLE, AINSI QUE NOUVEAUX DERIVES DE PYRAZOLYLBENZOYLE

(30) Priorität: 04.12.1998 DE 19856048
(43) Veröffentlichungstag der Anmeldung: 26.09.2001
(73) Patentinhaber: BASF AKTIENGESELLSCHAFT, 67056 Ludwigshafen (DE)
(72) Erfinder: NEIDLEIN, Ulf, D-68165 Mannheim (DE); GÖTZ, Norbert, D-67547 Worms (DE); MISSLITZ, Ulf, D-67433 Neustadt (DE); BAUMANN, Ernst, D-67373 Dudenhofen (DE); VON DEYN, Wolfgang, D-67435 Neustadt (DE); KUDIS, Steffen, D-68167 Mannheim (DE); LANGEMANN, Klaus, D-67551 Worms (DE); MAYER, Guido, D-67433 Neustadt (DE); WITSCHEL, Matthias, D-67061 Ludwigshafen (DE); OTTEN, Martina, D-67069 Ludwigshafen (DE); WESTPHALEN, Karl-Otto, D-67346 Speyer (DE); WALTER, Helmut, D-67283 Obrigheim (DE)
(86) Internationale Anmeldenummer: EP9909414
(87) Internationale Veröffentlichungsnummer: WO00034271

(56) Entgegenhaltungen:
- WO-A-97/30993
- DE-A- 19 532 312
- DE-A- 19 638 484

## Beschreibung

Die vorliegende Erfindung betrifft ein Verfahren zur Herstellung von Pyrazolylbenzoylderivaten, neue Pyrazolyl-benzoylderivate, herbizide Mittel welche diese enthalten und die Verwendung der pyrazolyl-benzoylderivate oder sie enthaltende Mittel zur Unkrautbekämpfung.

Aus der Literatur sind Verfahren zur Herstellung von herbizidwirksamen Pyrazolylbenzoylderivaten bekannt, beispielsweise aus der WO 93/18031, WO 94/01431, WO 98/12192 und WO 98/28291.

Gemäß der WO 98/12192 wird ein 5-Hydroxypyrazol mit einem Benzoylhalogenid acyliert und der gebildete Pyrazolester mit einem Katalysator zu den Pyrazolyl-benzoylderivaten umgelagert.

Ist das 5-Hydroxypyrazol an beiden Stickstoffatomen unsubstituiert, läßt sich der entsprechende Pyrazolester nicht zu den gewünschten Pyrazolyl-benzoylderivaten umlagern.

Somit läßt sich nach diesen Verfahren kein Pyrazolyl-benzoylderivat herstellen, bei dem der Pyrazolyl-Rest an den beiden Stickstoffatomen unsubstituiert ist.

Aufgabe der vorliegenden Erfindung war es, ein Verfahren zur Verfügung zu stellen, nach dem sich auch an den beiden Stickstoffatomen unsubstituierte Pyrazolyl-benzoylderivate in guter Ausbeute herstellen lassen.

Weitere Aufgabe der vorliegenden Erfindung war es, neue Pyrazolyl-benzoylderivate herzustellen, die an den beiden Stickstoffatomen unsubstituiert sind.

Gelöst wurde die Aufgabe durch ein Verfahren zur Herstellung von Pyrazolylbenzoylderivaten der Formel I 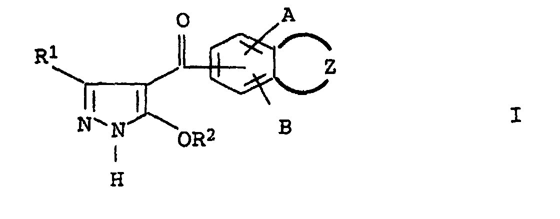 in der die Substituenten die folgende Bedeutung haben:
- R¹: Wasserstoff, C₁-C₄-Alkyl oder C₁-C₄-Halogenalkyl;
- R²: Wasserstoff;
C₁-C₆-Alkyl, C₂-C₆-Alkenyl, C₂-C₆-Alkinyl, Benzyl, Benzoyl, C₁-C₄-Alkylcarbonyl, Hydroxycarbonyl-C₁-C₄-alkyl, C₁-C₄-Alkoxycarbonyl-C₁-C₄-alkyl, C₁-C₄-Alkylsulfonyl oder Phenylsulfonyl, wobei diese Gruppen durch C₁-C₄-Alkyl, C₁-C₄-Alkoxy, C₁-C₄-Alkylthio, Halogen, Hydroxy, Amino, Nitro oder Cyano substituiert sein können;
- A,B: unabhängig voneinander Wasserstoff;
C₁-C₆-Alkyl, C₂-C₆-Alkenyl, C₂-C₆-Alkinyl, C₁-C₄-Alkoxy, wobei diese Gruppen durch Halogen, Hydroxy, C₁-C₄-Alkoxy oder Cyano substituiert sein können;
Halogen, Hydroxy, Cyano, Nitro, eine Gruppe -(Y)ₙ-S(O)ₘR³ oder eine Gruppe -(Y)ₙ-CO-R^{4;}
- Z: ein 5- oder 6-gliedriger heterocyclischer, gesättigter oder ungesättigter ankondensierter Ring, enthaltend ein bis drei Heteroatome, ausgewählt aus der Gruppe Sauerstoff, Schwefel oder Stickstoff, der gegebenenfalls durch Halogen, Cyano, Nitro, eine Gruppe -CO-R⁴, C₁-C₄-Alkyl, C₁-C₄-Halogenalkyl, C₃-C₈-Cycloalkyl, C₁-C₄-Alkoxy, C₁-C₄-Halogenalkoxy, C₁-C₄-Alkylthio, C₁-C₄-Halogenalkylthio, Di-C₁-C₄-Alkylamino, gegebenenfalls durch Halogen, Cyano, Nitro, C₁-C₄-Alkyl oder C₁-C₄-Halogenalkyl substituiertes Phenyl oder eine Oxogruppe, die gegebenenfalls auch in der tautomeren Form als Hydroxygruppe vorliegen kann, substituiert ist oder der mit einem ankondensierten, gegebenenfalls durch Halogen, Cyano, Nitro, C₁-C₄-Alkyl oder C₁-C₄-Halogenalkyl substituierten Phenylring, einem ankondensierten Carbocyclus oder einem ankondensierten, gegebenenfalls durch Halogen, Cyano, Nitro, C₁-C₄-Alkyl, Di-C₁-C₄-Alkylamino, C₁-C₄-Alkoxy, C₁-C₄-Halogenalkoxy, oder C₁-C₄-Halogenalkyl substituierten zweiten Heterocyclus ein tricyclisches System bildet;
- Y: O, NR⁵
- n: null oder eins
- m: null, eins oder zwei
- R³: C₁-C₄-Alkyl, C₁-C₄-Halogenalkyl oder NR⁵R⁶
- R⁴: C₁-C₄-Alkyl, C₁-C₄-Halogenalkyl, C₁-C₄-Alkoxy, oder NR⁵R⁶
- R⁵: Wasserstoff oder C₁-C₄-Alkyl
- R⁶: C₁-C₄-Alkyl indem man ein Pyrazolylbenzoylderivat der Formel II 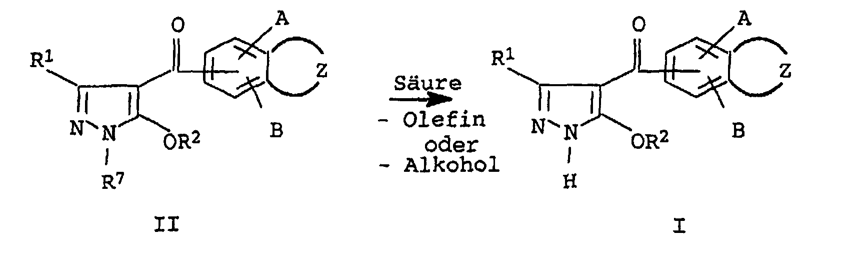 in der die Substituenten R¹, R², A, B und Z die oben angegebene Bedeutung haben und
- R⁷: in α-Stellung verzweigtes C₃-C₁₂-Alkyl, C₃-C₁₂-Alkenyl, C₄-C₁₂-Alkinyl, die gegebenenfalls durch Halogen oder C₁-C₄-Alkoxy substituiert sind oder Benzyl, das gegebenenfalls durch Halogen, Cyano, Nitro, C₁-C₄-Halogenalkyl, C₁-C₄-Alkylsulfonyl oder C₁-C₄-Alkylcarbonyl substituiert ist, bedeutet, mit einer anorganischen oder organischen Säure bei einem pH-Wert < 2 behandelt und unter Abspaltung eines Olefins oder eines Alkohols umsetzt.

Weiterhin betrifft die vorliegende Erfindung neue Pyrazol-4-ylbenzoylderivate der Formel I gemäß Anspruch 1 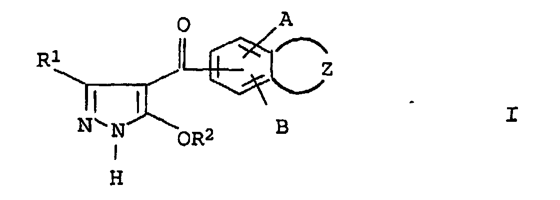 in der die Substituenten die folgende Bedeutung haben:
- R¹: Wasserstoff, C₁-C₄-Alkyl oder C₁-C₄-Halogenalkyl;
- R²: Wasserstoff;
C₁-C₆-Alkyl, C₂-C₆-Alkenyl, C₂-C₆-Alkinyl, Benzyl, Benzoyl, C₁-C₄-Alkylcarbonyl, Hydroxycarbonyl-C₁-C₄-alkyl, C₁-C₄-Alkoxycarbonyl-C₁-C₄-alkyl, C₁-C₄-Alkylsulfonyl oder Phenylsulfonyl, wobei diese Gruppen durch C₁-C₄-Alkyl,
C₁-C₄-Alkoxy, C₁-C₄-Alkylthio, Halogen, Hydroxy, Amino, Nitro oder Cyano substituiert sein können;
- A,B: unabhängig voneinander Wasserstoff;
C₁-C₆-Alkyl, C₂-C₆-Alkenyl, C₂-C₆-Alkinyl, C₁-C₄-Alkoxy, wobei diese Gruppen durch Halogen, Hydroxy, C₁-C₄-Alkoxy oder Cyano substituiert sein können;
Halogen, Hydroxy, Cyano, Nitro, eine Gruppe -(Y)ₙ-S(O)ₘR³ oder eine Gruppe -(Y)ₙ-CO-R^{4;}
- Z: ein 5- oder 6-gliedriger heterocyclischer, gesättigter oder ungesättigter ankondensierter Ring, enthaltend ein bis drei Heteroatome, ausgewählt aus der Gruppe Sauerstoff, Schwefel oder Stickstoff, der gegebenenfalls durch Halogen, Cyano, Nitro, eine Gruppe -CO-R⁴, C₁-C₄-Alkyl, C₁-C₄-Halogenalkyl, C₃-C₈-Cycloalkyl, C₁-C₄-Alkoxy, C₁-C₄-Halogenalkoxy, C₁-C₄-Alkylthio, C₁-C₄-Halogenalkylthio, Di-C₁-C₄-Alkylamino, gegebenenfalls durch Halogen, Cyano, Nitro, C₁-C₄-Alkyl oder C₁-C₄-Halogenalkyl substituiertes Phenyl oder eine Oxogruppe, die gegebenenfalls auch in der tautomeren Form als Hydroxygruppe vorliegen kann, substituiert ist oder der mit einem ankondensierten, gegebenenfalls durch Halogen, Cyano, Nitro, C₁-C₄-Alkyl oder C₁-C₄-Halogenalkyl substituierten Phenylring, einem ankondensierten Carbocyclus oder einem ankondensierten, gegebenenfalls durch Halogen, Cyano, Nitro, C₁-C₄-Alkyl, Di-C₁-C₄-Alkylamino, C₁-C₄-Alkoxy, C₁-C₄-Halogenalkoxy, oder C₁-C₄-Halogenalkyl substituierten zweiten Heterocyclus ein tricyclisches System bildet,
- Y: O, NR^{5;}
- n: null oder eins;
- m: null, eins oder zwei;
- R³: C₁-C₄-Alkyl, C₁-C₄-Halogenalkyl oder NR⁵R^{6;}
- R⁴: C₁-C₄-Alkyl, C₁-C₄-Halogenalkyl, C₁-C₄-Alkoxy, oder NR⁵R^{6;}
- R⁵: Wasserstoff oder C₁-C₄-Alkyl;
- R⁶: C₁-C₄-Alkyl;
sowie landwirtschaftlich übliche Salze der Verbindungen I.

Bei dem erfindungsgemäßen Verfahren wird ein Pyrazolylbenzoylderivat der Formel II mit einer anorganischen oder organischen Säure bei einem pH-Wert < 2 behandelt, wobei im Fall daß R⁷ verzweigtes C₃-C₁₂-Alkyl, C₃-C₁₂-Alkenyl oder C₄-C₁₂-Alkinyl bedeutet ein entsprechendes C₃-C₁₂-Olefin abgespalten wird, für den Fall, daß R⁷ gegebenenfalls substituiertes Benzyl bedeutet, der entsprechende Benzylalkohol abgespalten wird.

Die Reaktion wird bevorzugt in einem organischen Lösungsmittel durchgeführt. Geeignete Lösungsmittel sind beispielsweise Acetonitril, DMF, Dioxan, THF, Toluol, oder Chlorbenzol. Bevorzugte Lösungsmittel sind Acetonitril, THF oder Dioxan. Ein besonders bevorzugtes Lösungsmittel ist Acetonitril.

Geeignete Säuren sind beispielsweise Trifluormethansulfonsäure, Trichloressigsäure, Schwefelsäure, Salpetersäure, Salzsäure und Bromwasserstoffsäure. Bevorzugte Säuren sind Trifluormethansulfonsäure und Trichloressigsäure. Besonders bevorzugt ist Trifluormethansulfonsäure.

Die Reaktion wird bei einer Temperatur von 20 bis 150°C bzw. bis zum Siedepunkt des jeweiligen Lösungsmittels durchgeführt. Bevorzugt ist eine Temperatur von 50 bis 100°C bzw. der Siedetemperatur des verwendeten Lösungsmittels, falls diese unter 100°C liegt.

Die Reaktion kann bei Normaldruck, Überdruck und Unterdruck durchgeführt werden.

Wird beispielsweise ein Pyrazolylbenzoylderivat der Formel II, in der R⁷ für tert.-Butyl steht mit einer Säure behandelt, so wird Butylen abgespalten und das entsprechende Pyrazolylbenzoylderivat der Formel I gebildet. Steht R⁷ beispielsweise für sec.-Butyl wird Butylen abgespalten. Steht R⁷ beispielsweise für Benzyl, wird Benzylalkohol abgespalten.

Die Herstellung des Pyrazolylbenzoylderivates der Formel II erfolgt durch Umsetzung der Pyrazole der Formel III, in der die substituierten R¹ und R⁷ die eingangs angegeben Bedeutung haben, mit einem Benzoylderivat der Formel IV, in der T Halogen bedeutet und A, B und Z die eingangs angegeben Bedeutung haben, acyliert und den gebildeten Pyrazolester V in Gegenwart eines Katalysators zum Pyrazolylbenzoylderivat II mit R² = Wasserstoff umlagert.

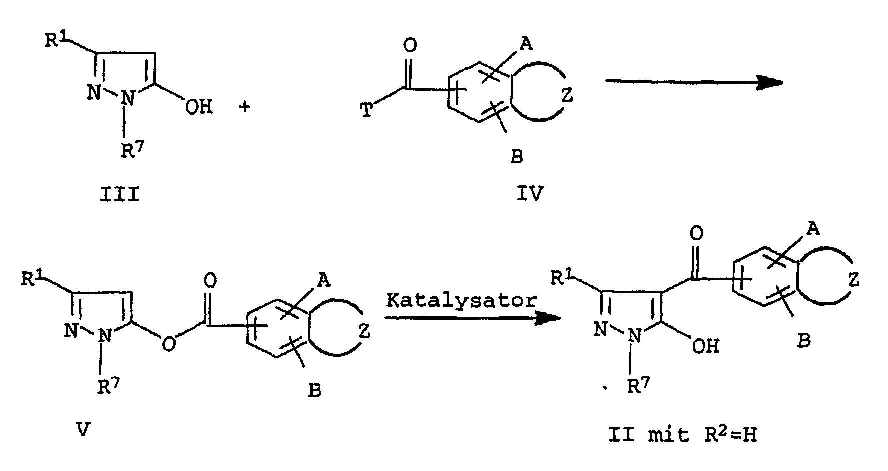

In den oben genannten Formeln haben T = Halogen und A, B und Z die eingangs angegebene Bedeutung.

Der erste Schritt der Reaktionsabfolge, die Acylierung, erfolgt in allgemein bekannter Weise, z. B. durch Zugabe eines Benzoylderivates der Formel IV (T = Cl) zur Lösung oder Suspension eines 5-Hydroxypyrazols III in Gegenwart einer Hilfsbase. Die Reaktanden und die Hilfsbase werden dabei zweckmäßigerweise in etwa äquimolaren Mengen eingesetzt. Ein geringer Überschuß der Hilfsbase, z.B. 1,2 bis 1,5 Moläquivalente, bezogen auf III, kann u.U. von Vorteil sein.

Als Hilfsbase eignen sich z.B. tertiäre Alkylamine, Pyridin oder Alkalicarbonate, während als Lösungsmittel Methylenchlorid, Diethylether, Toluol oder Essigsäureethylester verwendet werden können. Während der Zugabe des Säurechlorids wird die Reaktionsmischung vorteilhaft auf 0 -10°C gekühlt, danach wird bei höherer Temperatur, z.B. bei einer Temperatur von 25 - 50°C gerührt, bis die Umsetzung beendet ist.

Die Aufarbeitung erfolgt in üblicher Weise, z.B. wird das Reaktionsgemisch in Wasser gegossen und mit Methylenchlorid extrahiert. Nach Trocknen der organischen Phase und Entfernung des Lösungsmittels kann der rohe 5-Hydroxypyrazolester ohne weitere Reinigung zur Umlagerung eingesetzt werden. Herstellungsbeispiele für Benzoesäureester von 5-Hydroxy-pyrazolen findet man z. B. in EP-A-282 944 oder US 4,643,757.

Die Umlagerung der 5-Hydroxypyrazolester zu den Verbindungen der Formel I erfolgt in einem Lösungsmittel bei Temperaturen von 50°C bis 90°C oder beim Siedepunkt des Lösungsmittels und in Gegenwart einer Hilfsbase sowie mit Hilfe einer Cyanoverbindung als Katalysator. Als Lösungsmittel können z.B. Acetonitril, Methylenchlorid, 1,2-Dichlorethan, Essigsäureethylester oder Toluol verwendet werden. Bevorzugtes Lösungsmittel ist Acetonitril. Als Hilfsbase eignen sich tertiäre Alkylamine, Pyridin oder Alkalicarbonate, die in äquimolarer Menge oder bis zu vierfachem Überschuß eingesetzt werden. Bevorzugte Hilfsbase ist Triethylamin in doppelter Menge. Als Katalysator eignen sich Cyanidverbindungen, wie Kaliumcyanid oder Aceton-cyanhydrin, z.B. in einer Menge von 1 bis 50, insbesondere 5-20 Molprozent, bezogen auf den 5-Hydroxypyrazolester. Bevorzugt setzt man Acetoncyanhydrin z.B. in Mengen von 10 Molprozent zu.

Beispiele zur Umlagerung von Benzoesäureestern von 5-Hydroxypyrazolen findet man z. B. in WO 93/18031, WO 98/28291 oder WO 98/12192.

Die Aufarbeitung erfolgt in üblicher Weise, z.B. wird das Reaktionsgemisch mit verdünnten Mineralsäuren wie 5 % Salzsäure oder Schwefelsäure angesäuert und extrahiert z.B. mit Methylenchlorid oder Essigsäureethylester . Zur Reinigung wird der Extrakt mit kalter 5 - 10 % Alkalicarbonatlösung extrahiert, wobei das Endprodukt in die wäßrige Phase übergeht. Durch Ansäuern der wäßrigen Lösung wird das Produkt der Formel Ic ausgefällt, oder erneut mit Methylenchlorid extrahiert, getrocknet und anschließend vom Lösungsmittel befreit.

Die als Ausgangsmaterial verwendeten 5-Hydroxypyrazole der Formel III sind bekannt und können nach an sich bekannten Verfahren hergestellt werden (vgl. EP- A 240 001 und J. Prakt. Chem. 315, 382 (1973)). 1,3-Dimethyl-5-hydroxypyrazol ist eine käufliche Verbindung.

Benzoesäurederivate der Formel IV sind beispielsweise aus der WO 93/18031, WO 98/28291 oder WO 98/12192 bekannt.

Für das erfindungsgemäße Verfahren sind Pyrazolylbenzoylderivate der Formel II geeignet, in denen R⁷ in α-Stellung verzweigtes C₃-C₁₂-Alkyl, C₃-C₁₂-Alkenyl oder C₄-C₁₂-Alkinyl bedeutet, wobei diese Reste durch Halogen oder C₁-C₄-Alkoxy substituiert sein können, oder Benzyl, das durch Halogen, Cyano, Nitro, C₁-C₄-Halogenalkyl, SO₂-C₁-C₄-Alkyl oder C₁-C₄-Alkylcarbonyl substituiert sein kann.

Bevorzugt ist R⁷in α-Stellung verzweigtes C₃-C₆-Alkyl, das durch Halogen oder C₁-C₄-Alkoxy substituiert sein kann.

In α-Stellung verzweigtes C₃-C₆-Alkyl bedeutet beispielsweise 1-Methylethyl, 1,1-Dimethylethyl, 1-Methylpropyl, 1-Ethylpropyl, 1,1-Dimethylpropyl, 1-Methyl-1-ethylpropyl, 1-Methylbutyl, 1-Ethylbutyl, 1,1-Dimethylbutyl und 1-Methylpentyl.

In α-Stellung verzweigtes C₃-C₁₂-Alkyl bedeutet beispielsweise in α-Stellung verzweigtes C₃-C₆-Alkyl wie vorstehend genannt, sowie 1,1-Diethylpropyl, 1-Propylbutyl, 1-Methyl-1-ethylbutyl, 1,1-Diethylbutyl, 1-Methyl-1-propylbutyl, 1-Ethyl-1-propylbutyl, 1,1-Dipropyl-butyl, 1-Ethylpentyl, 1,1-Di-methylpentyl, 1,1-Diethylpentyl, 1-Methylhexyl.

1-Ethylhexyl, 1-Propylhexyl, 1,1-Dimethylhexyl, 1-1-Dipropylhexyl, 1-Methylheptyl, 1-Ethylheptyl, 1-Propylheptyl, 1-Methyloctyl, 1-Ethyloctyl, 1,1-Dimethyloctyl und 1,1-Diethyloctyl.

Bevorzugt sind 1-Methylethyl, 1,1-Dimethylethyl, 1-Methylpropyl, 1-Ethylpropyl, 1,1-Dimethylpropyl, 1-Methylbutyl und 2-Methylpentyl.

Besonders bevorzugt sind 1,1-Dimethylethyl, 1-Methylpropyl und 1-Methylbutyl.

Unter in α-Stellung verzweigtes C₃-C₁₂-Alkenyl ist mindestens eine Doppelbindung enthaltendes in α-Stellung verzweigtes C₃-C₁₂-Alkyl, wie oben aufgeführt zu verstehen.

Unter in α-Stellung verzweigtes C₄-C₁₂-Alkinyl ist mindestens eine Dreifachbindung enthaltendes in α-Stellung verzweigtes C₄-C₁₂-Alkyl, wie oben unter C₃-C₁₂-Alkyl aufgeführt, zu verstehen.

Gegebenenfalls substituiertes Benzyl bedeutet Benzyl, 4-Chlorbenzyl, 4-Cyanobenzyl, 4-Nitrobenzyl, 4-Trifluormethylbenzyl, 4-Methylsulfonylbenzyl oder 4-Acylbenzyl.

Bevorzugt sind Benzyl, 4-Chlorbenzyl und 4-Nitrobenzyl.

Besonders bevorzugt ist Benzyl.

Weiterhin sind für das erfindungsgemäße Verfahren Pyrazolylbenzoylderivate der Formel II geeignet, in denen bedeutet
- R¹ und R²: unabhängig voneinander Wasserstoff, Benzoyl, 4-F-Benzoyl, Benzyl, C₁-C₄-Alkyl, bevorzugt Wasserstoff und C₁-C₄-Alkyl;
- A, B: unabhängig voneinander Wasserstoff, C₁-C₄-Alkyl, C₁-C₄-Alkoxy, C₁-C₄-Alkylthio, C₁-C₄-Alkylsulfonyl, C₁-C₄-Halogenalkyl, Halogen, Hydroxy, Cyano oder Nitro,
bevorzugt Wasserstoff, Methyl, Methoxy, Methylthio, Methylsulfonyl, Trifluormethyl, Chlor, Cyano oder Nitro, besonders bevorzugt Wasserstoff, Methyl, Methoxy, Methylthio, Methylsulfonyl oder Chlor,
weiterhin bevorzugt
- A: Methyl oder Chlor,
- B: Methylthio oder Methylsulfonyl,
- Z: ein 5- oder 6-gliedriger heterocyclischer, gesättigter oder ungesättigter ankondensierter Ring,
so daß mit dem ankondensierten Phenylring beispielsweise folgende Heterocyclen resultieren:

Benzofuranyl, Isobenzofuranyl, Benzothienyl, Isobenzothienyl, Thionaphthalenyl, Isothionaphthalenyl, Indolyl, Isoindolyl, Indazolyl, Indoleninyl, Isobenzazolyl, Pyranopyrrolyl, Isoindazolyl, Indoxazinyl, Benzoxazolyl, Benzothiazolyl, Benzothiazolyl-S,S-dioxid, Benzopyranyl, Chinolinyl, Isochinolinyl, Phthalazinyl, Chinoxalinyl, Chinazolinyl, Naphthyridinyl, Pyridopyridinyl, Pyranyl, Thiopyranyl, Chromanyl, Thiachromanyl, Chromenyl, Thiachromenyl, Benzoxazinyl und Benzisoxazinyl.

Im Hinblick auf die bestimmungsgemäße Verwendung der Pyrazol-4-yl-benzoylderivate der allgemeinen Formel I kommen als Substituenten folgende Reste in Betracht:
- R¹: Wasserstoff,
C₁-C₄-Alkyl wie Methyl, Ethyl, n-Propyl, 1-Methylethyl, Butyl, 1-Methylpropyl, 2-Methylpropyl und 1,1-Dimethylethyl;
C₁-C₄-Halogenalkyl: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
bevorzugt ist Wasserstoff und Methyl;
- R²: Wasserstoff;
C₁-C₆-Alkyl; C₁-C₄-Alkyl wie voranstehend genannt sowie Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 1,1,2-Trimethylpropyl, 1,2,2-Trimethylpropyl, 1-Ethyl-1-methylpropyl oder 1-Ethyl-2-methyl-propyl,
bevorzugt Methyl, Ethyl, 1-Methylethyl, 1-Methylpropyl, 2-Methylpropyl, 1,1-Dimethylethyl und 1,1-Dimethylpropyl;
besonders bevorzugt Methyl und Ethyl;
C₂-C₆-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 3-Methyl-2-butenyl, 1-Methyl-2-butenyl, 2-Methyl-2-butenyl, 1-Methyl-3-butenyl, 2-Methyl-4-butenyl, 3-Methyl-3 butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-penteny, 2-Methyl-2-pentenyl, 3-Methyl-2-pentenyl, 4-Methyl-2-pentenyl, 1-Methyl-3 pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3 butenyl, 2-Ethyl-2-butenyl, 2-Ethyl-3-butenyl, 1,1,2-Trimethyl-2-propenyl, 1-Ethyl-1-methyl-2-propenyl und Ethyl-2-methyl-2-propenyl,
bevorzugt 1-Methyl-2-propenyl, 1-Methyl-2-butenyl, 1,1-Dimethyl-2-propenyl und 1,1-Dimethyl-2-butenyl;
C₂-C₆-Alkinyl wie Propargyl, 2-Butinyl, 3-Butenyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2 propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;
bevorzugt Propargyl;
Benzyl, Benzoyl;
C₁-C₄-Alkylcarbonyl wie Methylcarbonyl, Ethylcarbonyl, n-Propylcarbonyl, 1-Methylethylcarbonyl, n-Butylcarbonyl, 1-Methylpropylcarbonyl, 2-Methylpropylcarbonyl und 1,1-Dimethylethylcarbonyl, insbesondere Methylcarbonyl;
Hydroxycarbonyl-C₁-C₄-alkyl mit C₁-C₄-alkyl wie vorstehend genannt;
C₁-C₄-Alkoxycarbonyl-C₁-C₄-alkyl mit C₁-C₄-Alkoxy wie Methoxy, Ethoxy, n-Propoxy, 1-Methylethoxy, n-Butoxy, 1-Methylpropoxy, 2-Methylpropoxy und 1,1-Dimethylethoxy,
bevorzugt C₁-C₃-Alkoxy wie Methoxy, Ethoxy, i-Propoxy, besonders bevorzugt Methoxy;
C₁-C₄-Alkylsulfonyl, wie Methylsulfonyl, Ethylsulfonyl, Propylsulfonyl, 1-Methylethylsulfonyl, Butylsulfonyl, 1-Methylpropylsulfonyl, 2-Methylpropylsulfonyl und 1,1-Dimethylethylsulfonyl;
Phenylsulfonyl;
bevorzugt ist Wasserstoff und C₁-C₄-Alkylsulfonyl;
besonders bevorzugt ist Wasserstoff, Methylsulfonyl und Ethylsulfonyl;
wobei diese Gruppen durch C₁-C₄-Alkyl, C₁-C₄-Alkoxy; C₁-C₄-Alkylthio wie Methylthio, Ethylthio, n-Propylthio, 1-Methylethylthio, n-Butylthio, 1-Methylpropylthio, 2-Methylpropylthio und 1,1-Dimethylethylthio, insbesondere Methylthio;
Halogen, Hydroxy, Amino, Nitro und Cyano substituiert sein können;
- A,B: unabhängig voneinander Wasserstoff;
C₁-C₆-Alkyl wie vorstehend genannt;
C₁-C₆-Alkenyl wie vorsthend genannt;
C₁-C₆-Alkinyl wie vorstehend genannt;
C₁-C₄-Alkoxy wie vorstehend genannt, wobei diese Gruppen durch Halogen, wie Fluor, Chlor, Brom und Jod, Hydroxy, C₁-C₄-Alkoxy wie vorstehend genannt oder Cyano substituiert sein können;
Halogen, Hydroxy, Cyano, Nitro, eine Gruppe -(Y)ₙ-S(O)ₘ-R³ oder eine Gruppe -(Y)ₙ-CO-R⁴.

Die vorstehend definierte Gruppe -(Y)ₙ-S(O)ₘR³ steht beispielsweise für
C₁-C₄-Alkylthio wie vorstehend genannt;
C₁-C₄-Alkylsulfinyl wie Methylsulfinyl, Ethylsulfinyl, n-Propylsulfinyl, 1-Methylethylsulfinyl, n-Butylsulfinyl, 1-Methylpropylsulfinyl, 2-Methylpropylsulfinyl und 1,1-Dimethylethylsulfinyl, insbesondere Methylsulfinyl;
C₁-C₄-Alkylsulfonyl wie Methylsulfonyl, Ethylsulfonyl, n-Propylsulfonyl, 1-Methylethylsulfonyl, n-Butylsulfonyl, 1-Methylpropylsulfonyl, 2-Methylpropylsulfonyl und 1,1-Dimethylethylsulfonyl, insbesondere Methylsulfonyl;
C₁-C₄-Alkoxysulfonyl wie Methoxysulfonyl, Ethoxysulfonyl, n-Propoxysulfonyl, 1-Methylethoxysulfonyl, n-Butoxysulfonyl, 1-Methylpropoxysulfonyl, 2-Methylpropoxysulfonyl und 1,1-Dimethylethoxysulfonyl, insbesondere Methoxysulfonyl;
N-C₁-C₄-Alkylsulfamoyl wie N-Methylsulfamoyl, N-Ethylsulfamoyl, N-n-Propylsulfamoyl, N-1-Methylethylsulfamoyl, N-n-Butylsulfamoyl, N-1-Methylpropylsulfamoyl, N-2-Methylpropylsulfamoyl und N-1,1-Dimethylethylsulfamoyl, insbesondere N-Methylsulfamoyl;
N-C₁-C₄-Alkylsulfinamoyl wie N-Methylsulfinamoyl, N-Ethylsulfinamoyl, N-n-Propylsulfinamoyl, N-1-Methylethylsulfinamoyl, N-n-Butylsulfinamoyl, N-1-Methylpropylsulfinamoyl, N-2-Methylpropylsulfinamoyl und N-1,1-Dimethylethylsulfinamoyl, insbesondere N-Methylsulfinamoyl;
Di-C₁-C₄-Alkylsulfamoyl wie Dimethylsulfamoyl, Diethylsulfamoyl, Dipropylsulfamoyl, Dibutylsulfamoyl, N-Methyl-N-ethylsulfamoyl, N-Methyl-N-propylsulfamoyl, N-Methyl-N-l-methylethylsulfamoyl, N-Methyl-N-1,1-Dimethylethylsulfamoyl, Di-1-Methylethylsulfamoyl, N-Ethyl-N-1-Methylethylsulfamoyl und N-Ethyl-N-1,1-dimethylethylsulfamoyl; insbesondere Dimethylsulfamoyl;
Di-C₁-C₄-Alkylsulfinamoyl wie Dimethylsulfinamoyl, Diethylsulfinamoyl, Dipropylsulfinamoyl, Dibutylsulfinamoyl, N-Methyl-N-ethylsulfinamoyl, N-Methyl-N-propylsulfinamoyl, N-Methyl-N-1-methylethylsulfinamoyl, N-Methyl-N-1,1-Dimethylethylsulfinamoyl, Di-1-Methylethylsulfinamoyl, N-Ethyl-N-1-Methylethylsulfinamoyl und N-Ethyl-N-1,1-dimethylethylsulfinamoyl; insbesondere Dimethylsulfinamoyl,
C₁-C₄-Alkylsulfinyloxy wie Methylsulfinyloxy, Ethylsulfinyloxy, n-Propylsulfinyloxy, 1-Methylethylsulfinyloxy, n-Butylsulfinyloxy, 1-Methylpropylsulfinyloxy, 2-Methylpropylsulfinyloxy und 1,1-Dimethylethylsulfinyloxy, insbesondere Methylsulfinyloxy;
C₁-C₄-Alkylsulfonyloxy wie Methylsulfonyloxy, Ethylsulfonyloxy, n-Propylsulfonyloxy, 1-Methylethylsulfonyloxy, n-Butylsulfonyloxy, 1-Methylpropylsulfonyloxy, 2-Methylpropylsulfonyloxy und 1,1-Dimethylethylsulfonyloxy, insbesondere Methylsulfonyloxy;
C₁-C₄-Alkylsulfinylamino wie Methylsulfinylamino, Ethylsulfinylamino, n-Propylsulfinylamino, 1-Methylethylsulfinylamino, n-Butylsulfinylamino, 1-Methylpropylsulfinylamino, 2-Methylpropylsulfinylamino und 1,1-Dimethylethylsulfinylamino, insbesondere Methylsulfinylamino;
C₁-C₄-Alkylsulfonylamino wie Methylsulfonylamino, Ethylsulfonylamino, n-Propylsulfonylamino, 1-Methylethylsulfonylamino, n-Butylsulfonylamino, 1-Methylpropylsulfonylamino, 2-Methylpropylsulfonylamino und 1,1-Dimethylethylsulfonylamino, insbesondere Methylsulfonylamino;
N-C₁-C₄-Alkylsulfinyl-N-methyl-amino wie N-Methylsulfinyl-N-methyl-amino, N-Ethylsulfinyl-N-methyl-amino, N-n-Propylsulfinyl-N-methyl-amino, N-1-Methylethylsulfinyl-N-methyl-amino, N-n-Butylsulfinyl-N-methyl-amino, N-1-Methylpropylsulfinyl-N-methyl-amino, N-2-Methylpropylsulfinyl-N-methyl-amino und N-1,1-Dimethylethylsulfinyl-N-methyl-amino, insbesondere N-Methylsulfinyl-N-methyl-amino;
N-C₁-C₄-Alkylsulfinyl-N-ethyl-amino wie N-Methylsulfinyl-N-ethyl-amino, N-Ethylsulfinyl-N-ethyl-amino, N-n-Propylsulfinyl-N-ethyl-amino, N-1-Methylethylsulfinyl-N-ethyl-amino, N-n-Butylsulfinyl-N-ethyl-amino, N-1-Methylpropylsulfinyl-N-ethyl-amino, N-2-Methylpropylsulfinyl-N-ethyl-amino und N-1,1-Dimethylethylsulfinyl-N-ethyl-amino, insbesondere N-Methylsulfinyl-N-ethyl-amino;
N-C₁-C₄-Alkylsulfonyl-N-methyl-amino wie N-Methylsulfonyl-N-methyl-amino, N-Ethylsulfonyl-N-methyl-amino, N-n-Propylsulfonyl-N-methyl-amino, N-l-Methylethylsulfonyl-N-methyl-amino, N-n-Butylsulfonyl-N-methyl-amino, N-1-Methylpropylsulfonyl-N-methyl-amino, N-2-Methylpropylsulfonyl-N-methyl-amino und N-1,1-Dimethylethylsulfonyl-N-methyl-amino, insbesondere N-Methylsulfonyl-N-methyl-amino;
N-C₁-C₄-Alkylsulfonyl-N-ethyl-amino wie N-Methylsulfonyl-N-ethyl-amino, N-Ethylsulfonyl-N-ethyl-amino, N-n-Propylsulfonyl-N-ethyl-amino, N-1-Methylethylsulfonyl-N-ethyl-amino, N-n-Butylsulfonyl-N-ethyl-amino, N-1-Methylpropylsulfonyl-N-ethyl-amino, N-2-Methylpropylsulfonyl-N-ethyl-amino und N-1,1-Dimethylethylsulfonyl-N-ethyl-amino, insbesondere N-Methylsulfonyl-N-ethyl-amino;
C₁-C₄-Halogenalkylthio wie Chlormethylthio, Dichlormethylthio, Trichlormethylthio, Fluormethylthio, Difluormethylthio, Trifluormethylthio, Chlorfluormethylthio, Chlordifluormethylthio, 1-Fluorethylthio, 2-Fluorethylthio, 2,2-Difluorethylthio, 2,2,2-Trifluorethylthio, 2-Chlor-2,2-difluorethylthio, 2,2-Dichlor-2 fluorethylthio, 2,2,2-Trichlorethylthio und Pentafluorethylthio, insbesondere Trifluormethylthio.

Die vorstehend definierte Gruppe -(Y)ₙ-CO-R⁴ steht beispielsweise für
Hydroxycarbonyl, Hydroxycarbonyloxy und Hydroxycarbonylamido;
C₁-C₄-Alkylcarbonyl wie Methylcarbonyl, Ethylcarbonyl, n-Propylcarbonyl, 1-Methylethylcarbonyl, n-Butylcarbonyl, 1-Methylpropylcarbonyl, 2-Methylpropylcarbonyl und 1,1-Dimethylethylcarbonyl, insbesondere Methylcarbonyl;
C₁-C₄-Alkoxycarbonyl wie Methoxycarbonyl, Ethoxycarbonyl, n-Propoxycarbonyl, 1-Methylethoxycarbonyl, n-Butoxycarbonyl, 1-Methylpropoxycarbonyl, 2-Methylpropoxycarbonyl und 1,1-Dimethylethoxycarbonyl, insbesondere Methoxycarbonyl;
N-C₁-C₄-Alkylcarbamoyl wie N-Methylcarbamoyl, N-Ethylcarbamoyl, N-n-Propylcarbamoyl, N-1-Methylethylcarbamoyl, N-n-Butylcarbamoyl, N-1-Methylpropylcarbamoyl, N-2-Methylpropylcarbamoyl und N-1,1-Dimethylethylcarbamoyl, insbesondere N-Methylcarbamoyl;
Di-C₁-C₄-Alkylcarbamoyl wie Dimethylcarbamoyl, Diethylcarbamoyl, Dipropylcarbamoyl, Dibutylcarbamoyl, N-Methyl-N-ethylcarbamoyl, N-Methyl-N-propylcarbamoyl, N-Methyl-N-1-methylethylcarbamoyl, N-Methyl-N-1,1-Dimethylethylcarbamoyl, Di-1-Methylethylcarbamoyl, N-Ethyl-N-1-Methylethylcarbamoyl und N-Ethyl-N-1,1-dimethyl ethylcarbamoyl; insbesondere Dimethylcarbamoyl;
C₁-C₄-Alkylcarbonyloxy wie Methylcarbonyloxy, Ethylcarbonyloxy, n-Propylcarbonyloxy, 1-Methylethylcarbonyloxy, n-Butylcarbonyloxy, 1-Methylpropylcarbonyloxy, 2-Methylpropylcarbonyloxy und 1,1-Dimethylethylcarbonyloxy, insbesondere Methylcarbonyloxy; C₁-C₄-Alkylcarbonylamino wie Methylcarbonylamino, Ethylcarbonylamino, n-Propylcarbonylamino, 1-Methylethylcarbonylamino, n-Butylcarbonylamino, 1-Methylpropylcarbonylamino, 2-Methylpropylcarbonylamino und 1,1-Dimethylethylcarbonylamino, insbesondere Methylcarbonylamino;
N-C₁-C₄-Alkylcarbonyl-N-methyl-amino wie N-Methylcarbonyl-N-methyl-amino, N-Ethylcarbonyl-N-methyl-amino, N-n-Propylcarbonyl-N-methyl-amino, N-1-Methylethylcarbonyl-N-methyl-amino, N-n-Butylcarbonyl-N-methyl-amino, N-1-Methylpropylcarbonyl-N-methyl-amino, N-2-Methylpropylcarbonyl-N-methyl-amino und N-1,1-Dimethylethylcarbonyl-N-methyl-amino, insbesondere N-Methylcarbonyl-N-methylamino.

Weiterhin bevorzugt stehen die Reste A und B unabhängig voneinander für Wasserstoff, C₁-C₄-Alkyl, C₁-C₄-Alkoxy, C₁-C₄-Alkylthio, C₁-C₄-Alkylsulfonyl, C₁-C₄-Halogenalkyl, Halogen, Hydroxy, Cyano oder Nitro.

Darüber hinaus bevorzugt stehen die Reste A und B unabhängig voneinander für Wasserstoff, Methyl, Methoxy, Methylthio, Methylsulfonyl, Triflurmethyl, Chlor, Hydroxy, Cyano oder Nitro.

Auch bevorzugt stehen die Reste A und B unabhängig voneinander für Wasserstoff, Methyl, Methoxy, Methylthio, Methylsulfonyl oder Chlor oder Hydroxy.

Besonders bevorzugt stehen
- A: für Methyl oder Chlor und
- B: für Methylthio oder Methylsulfonyl.
- Z: ein 5- oder 6-gliedriger heterocyclischer, gesättigter oder ungesättigter ankondensierter Ring,
so daß mit dem ankondensierten Phenylring beispielsweise folgende Heterocyclen resultieren:

Benzofuranyl, Isobenzofuranyl, Benzothienyl, Isobenzothienyl, Thionaphthalenyl, Isothionaphthalenyl, Indolyl, Isoindolyl, Indazolyl, Indoleninyl, Isobenzazolyl, Pyranopyrrolyl, Isoindazolyl, Indoxazinyl, Benzoxazolyl, Benzothiazolyl, Benzothiazolyl-S,S-dioxid, Benzopyranyl, Chinolinyl, Isochinolinyl, Phthalazinyl, Chinoxalinyl, Chinazolinyl, Naphthyridinyl, Pyridopyridinyl, Pyranyl, Thiopyranyl, Chromanyl, Thiachromanyl, Chromenyl, Thiachromenyl, Benzoxazinyl und Benzisoxazinyl.

Bevorzugt sind auch Verbindungen der allgemeinen Formel VI 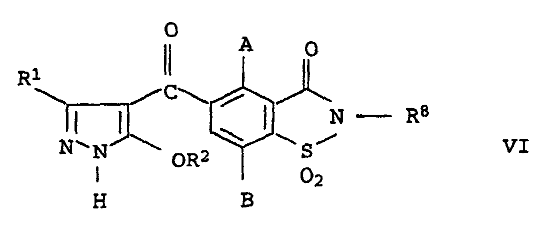 in der die Substituenten R¹, R², A und B die vorstehend angegebene Bedeutungen haben und R⁸ Wasserstoff, C₁-C₄-Alkyl, C₁-C₄-Alkoxy oder C₁-C₄-Halogenalkyl bedeutet.

Weiterhin bevorzugt sind Verbindungen der allgemeinen Formel VII 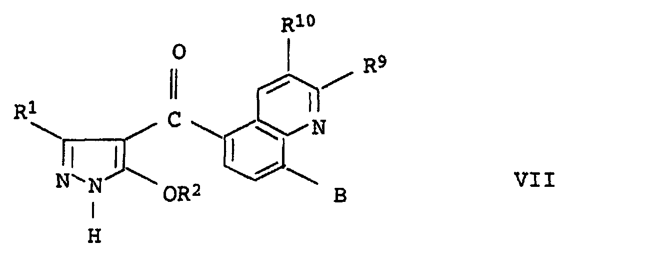 in der die Substituenten R¹, R² und B die vorstehend genannte Bedeutung haben und R⁹, R¹⁰ unabhängig voneinander Wasserstoff, C₁-C₄-Alkyl, C₁-C₄-Alkoxy oder C₁-C₄-Halogenalkyl bedeutet.

Bevorzugt sind auch Verbindungen der allgemeinen Formel VIII 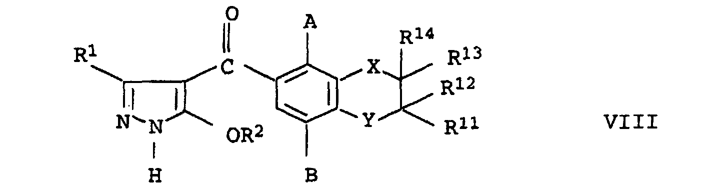 in der die Substituenten R¹, R² A und B die vorstehend genannte Bedeutung haben und
- R¹¹, R¹², R¹³, R¹⁴: unabhängig voneinander Wasserstoff, C₁-C₄-Alkyl, C₁-C₄-Alkoxy oder C₁-C₄ Halogenalkyl bedeutet;
- R¹¹, R¹³ oder R¹¹, R¹⁴ oder R¹², R¹³ oder R¹², R¹⁴: können eine Bindung bilden;
- X, Y: unabhängig voneinander S(O)ₙ, C=O, CR¹⁵R¹⁶, NR¹⁷;
- R¹⁵, R¹⁶: unabhängig voneinander Wasserstoff, C₁-C₄-Alkyl, C₁-C₄-Alkoxy, C₁-C₄-Halogenalkyl oder C₁-C₄-Halogenalkoxy;
- R¹⁵, R¹⁶: können zusammen einen 5-6 gliedrigen Carbocyclus bilden, der 2 Sauerstoffatome oder Schwefelatome enthalten kann;
- R¹⁷: Wasserstoff oder C₁-C₄-Alkyl;
- n: 0, 1 oder 2
bedeutet.

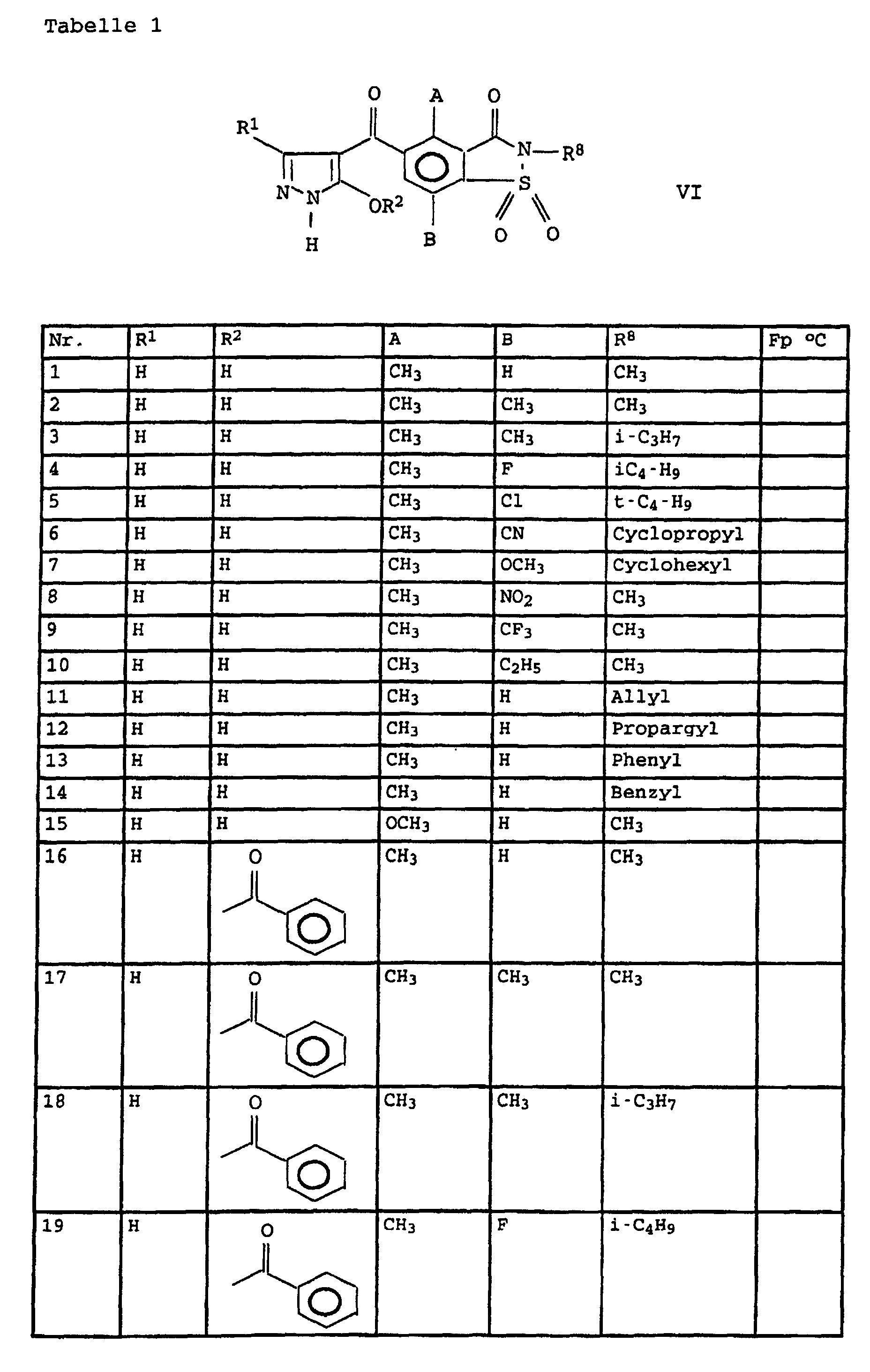

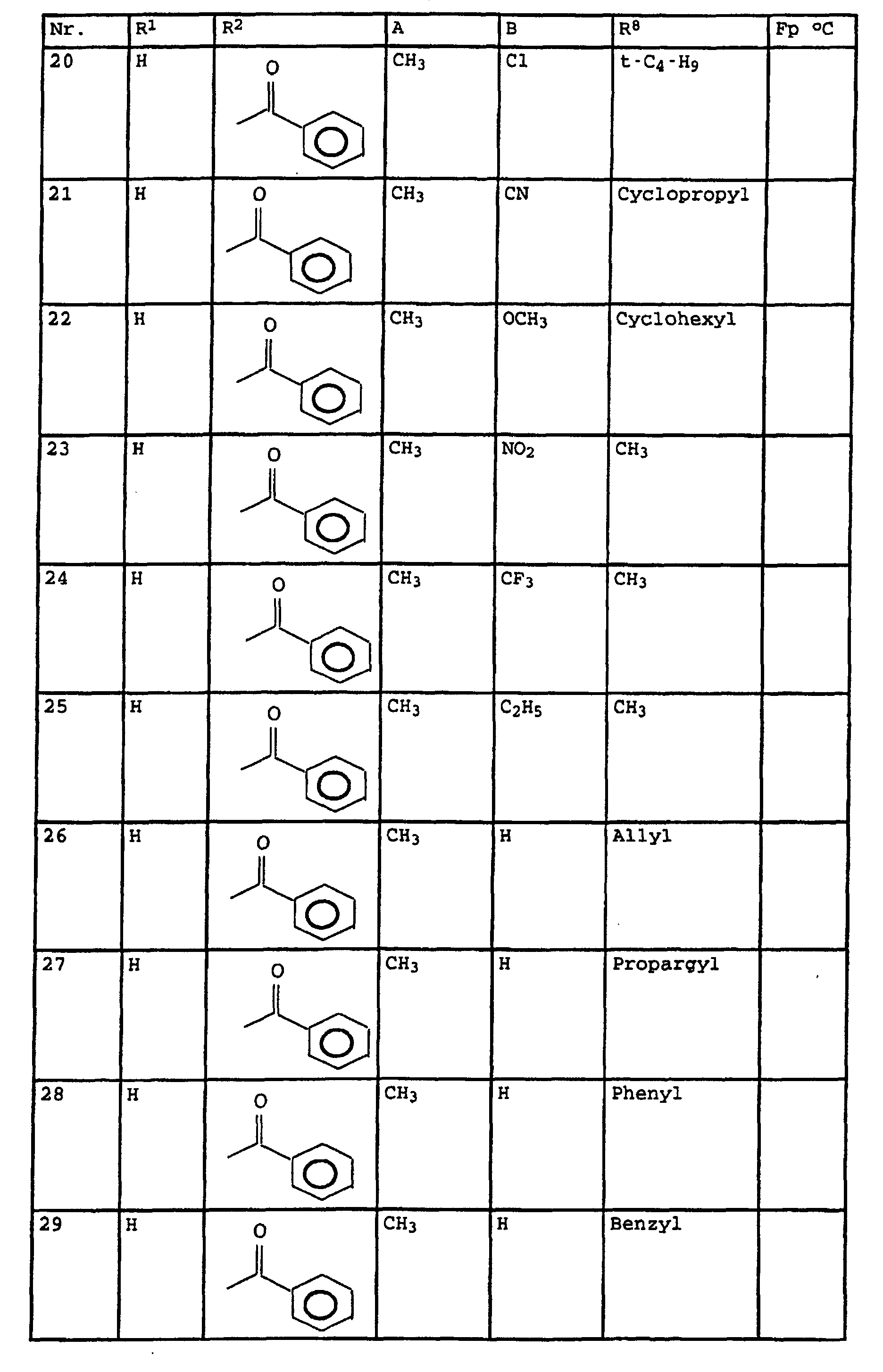

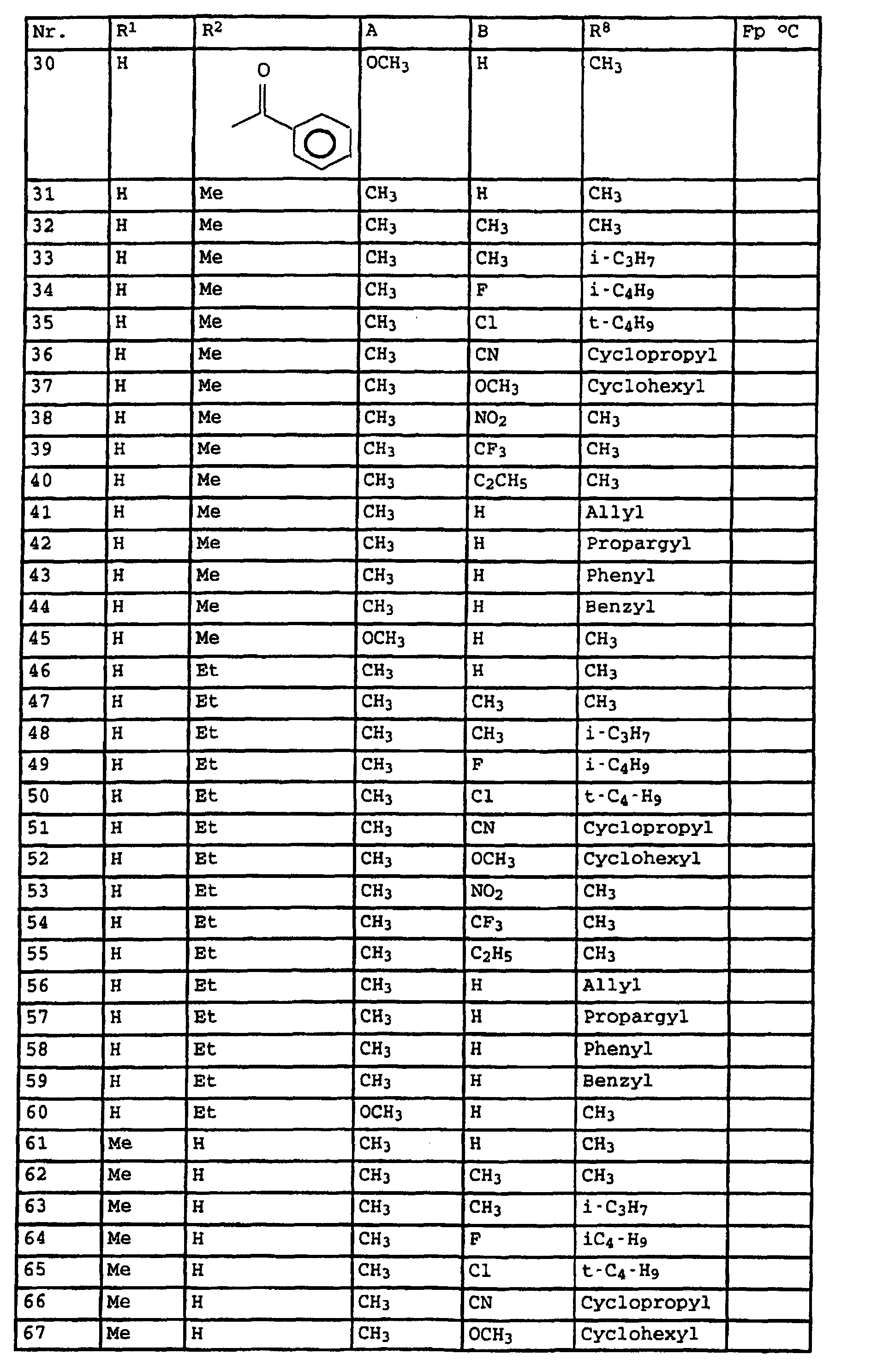

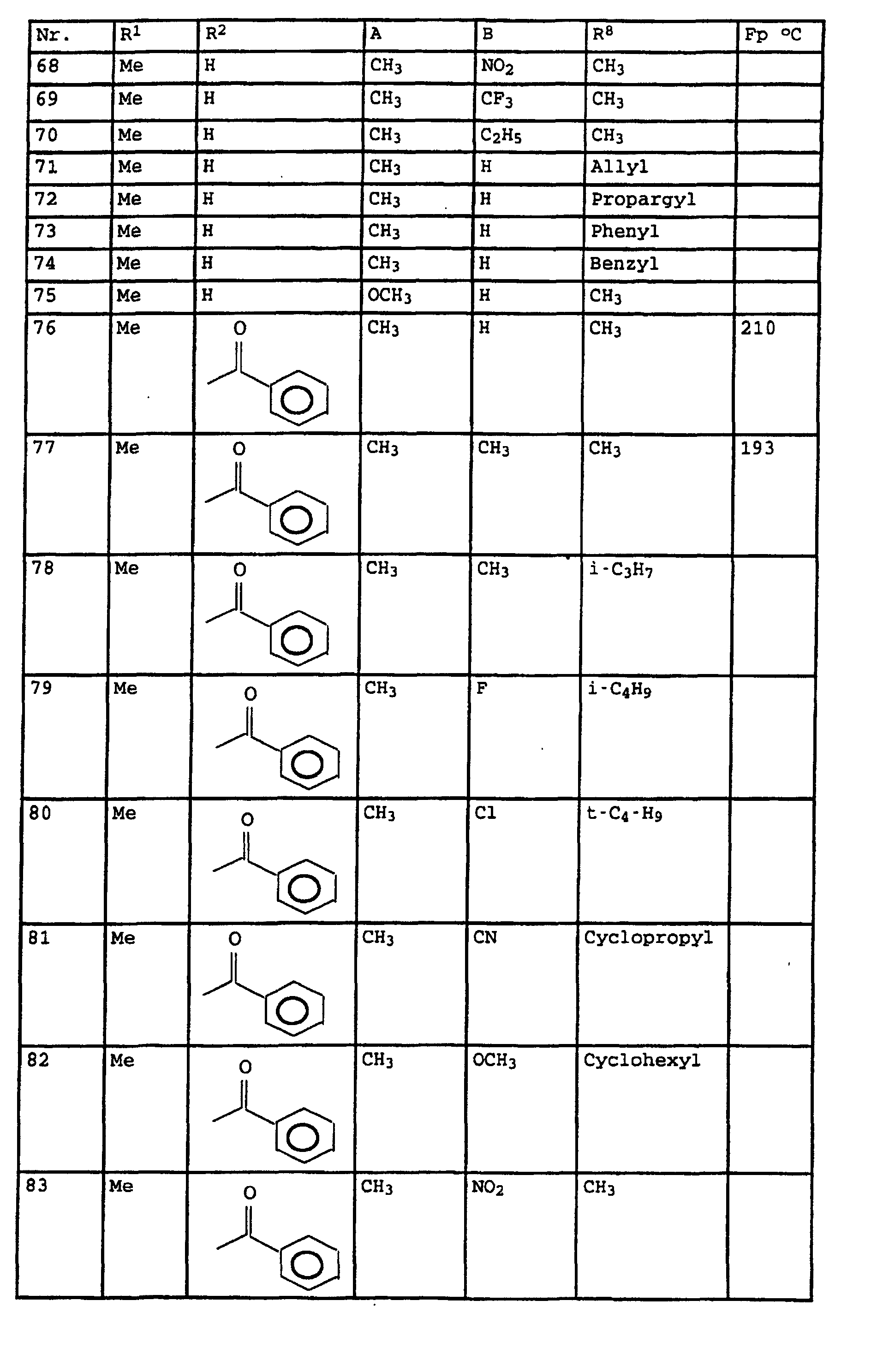

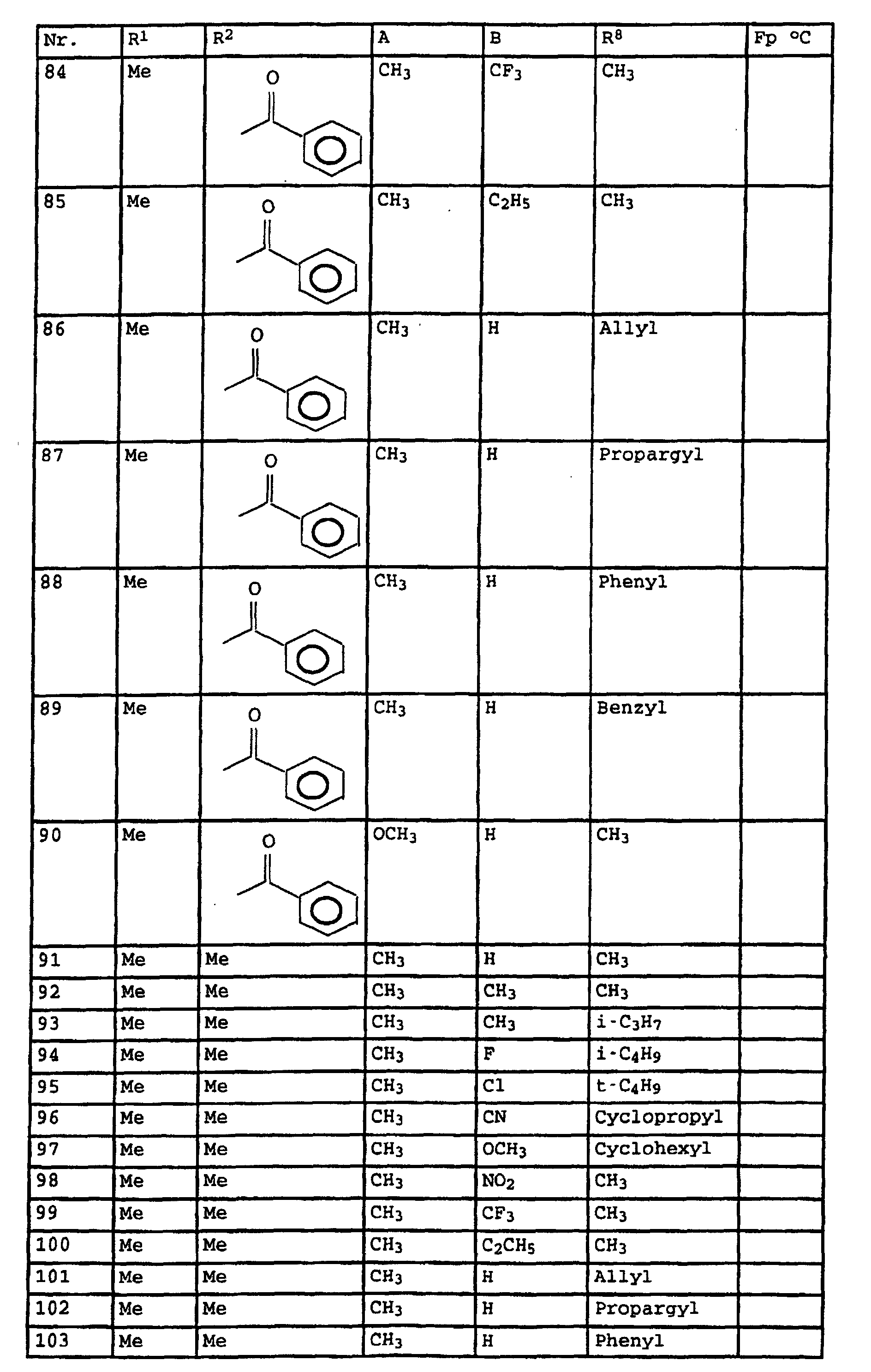

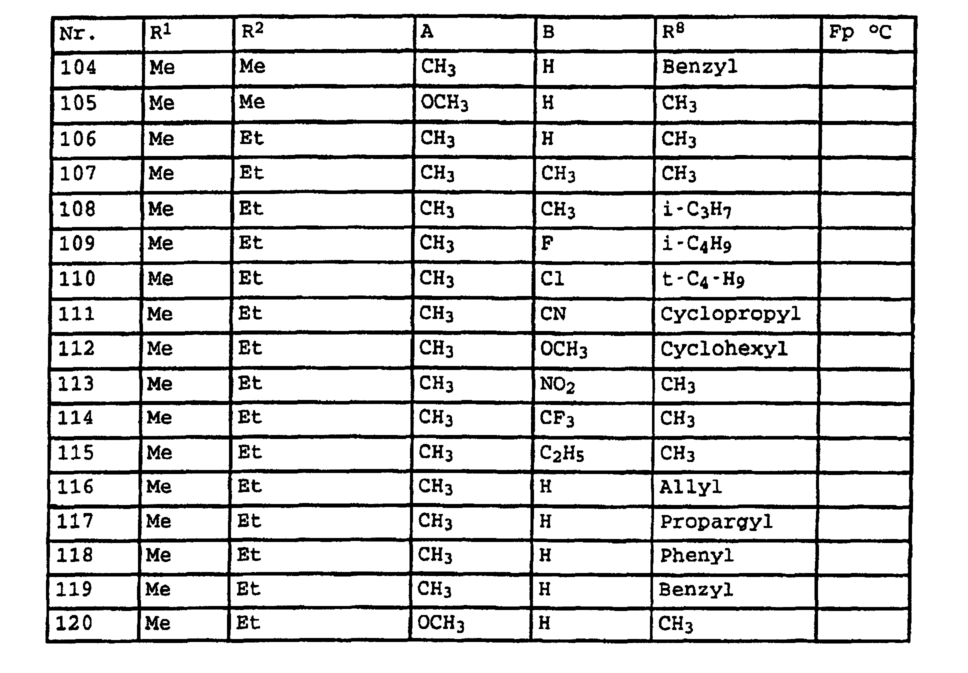

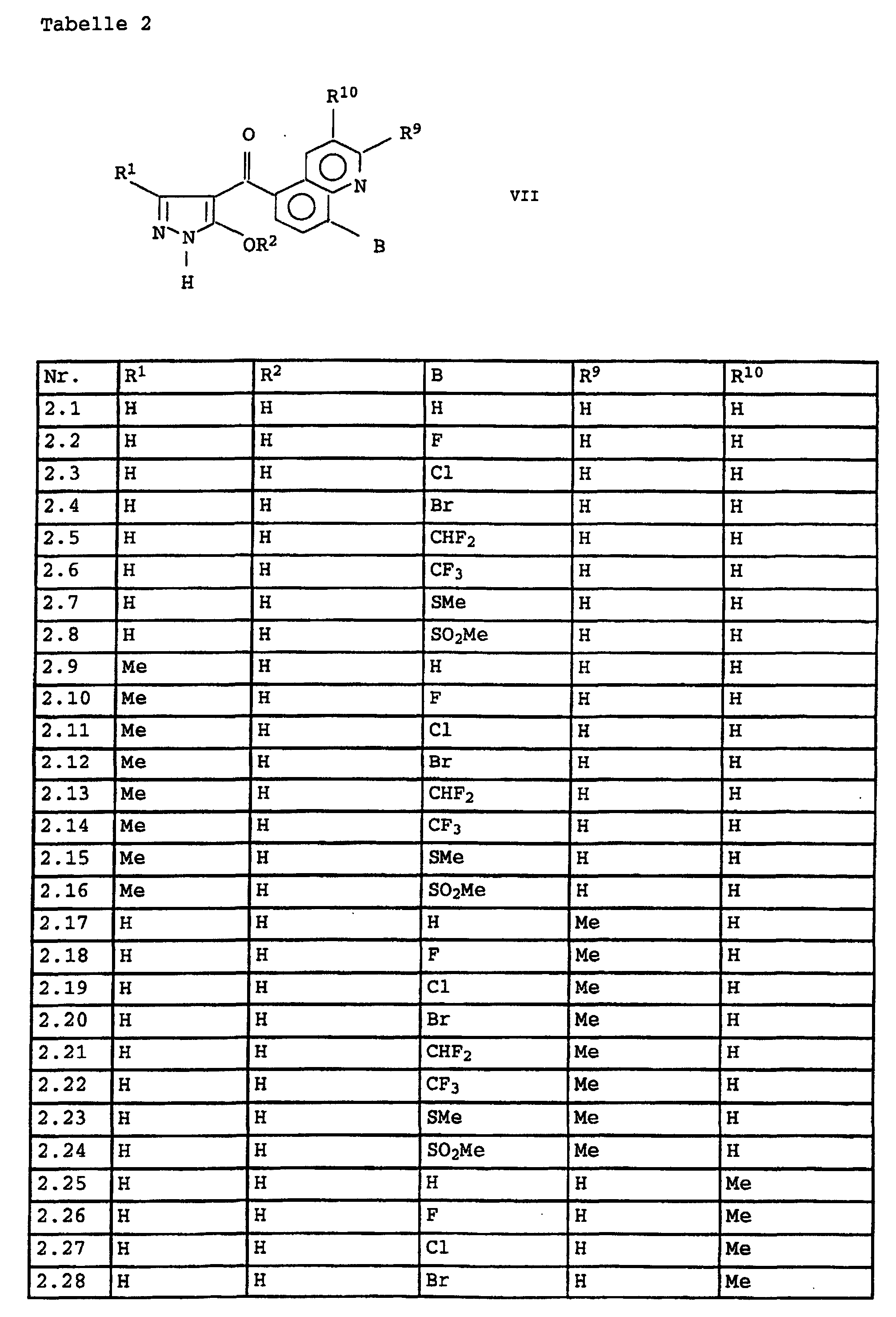

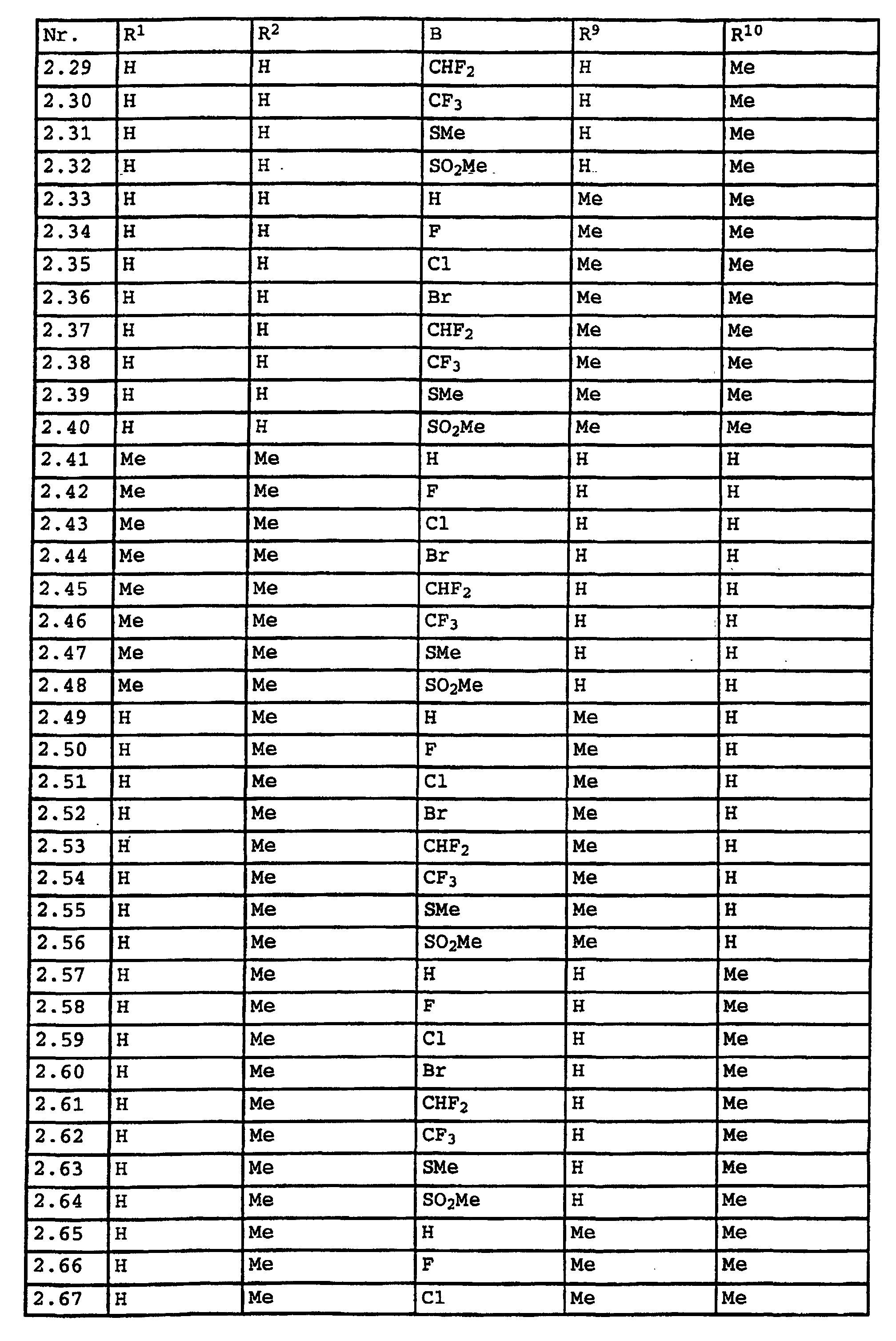

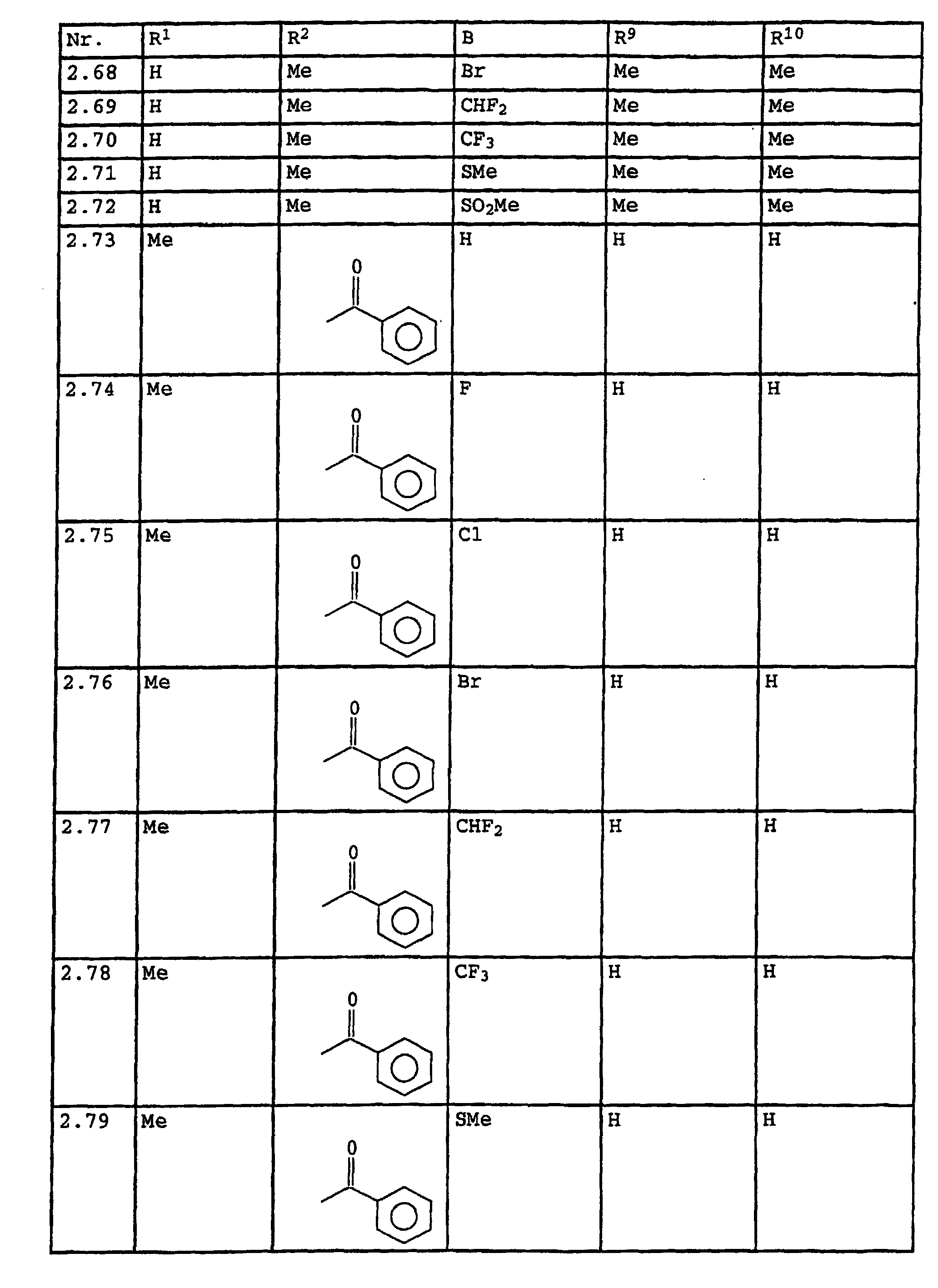

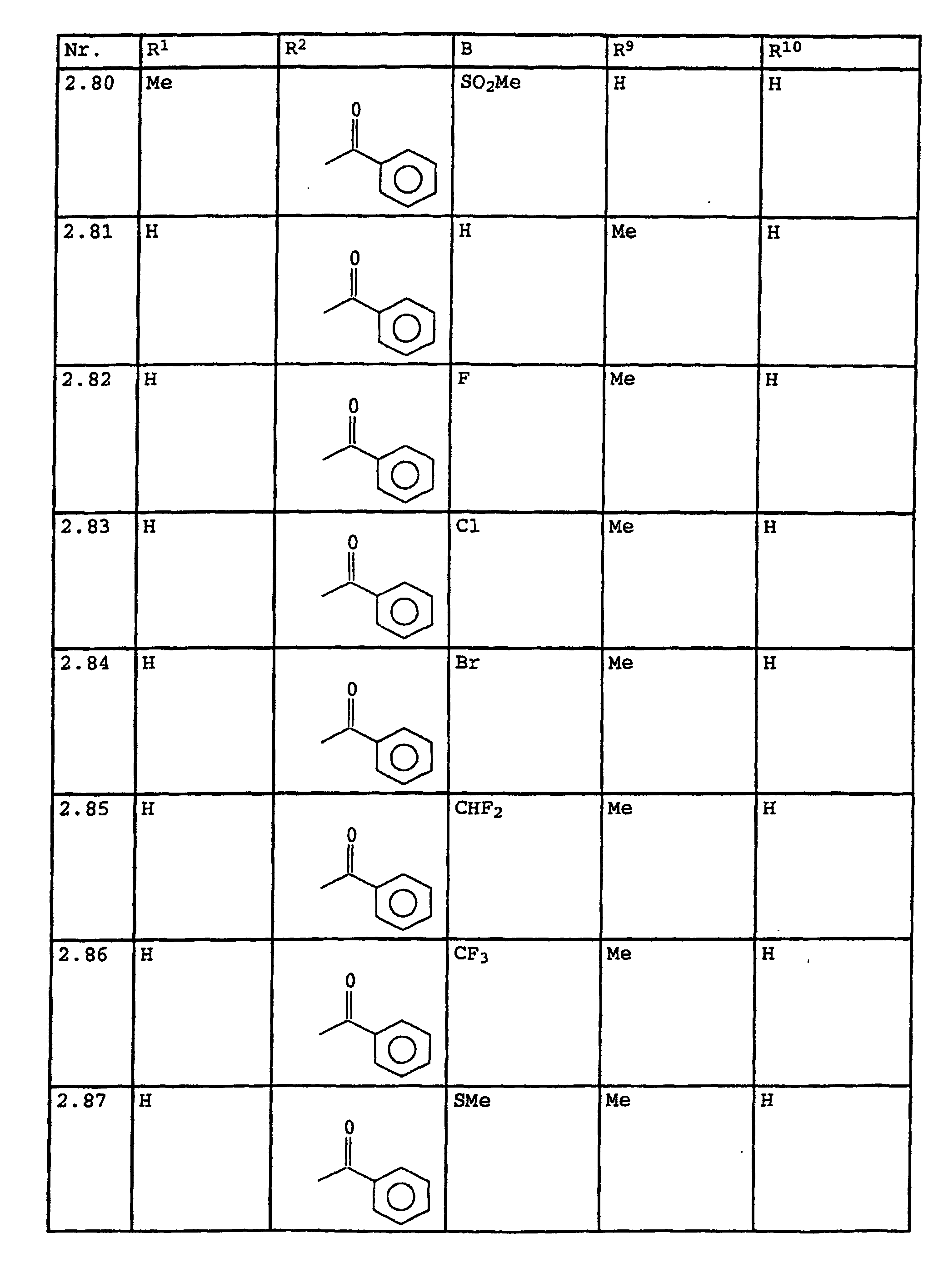

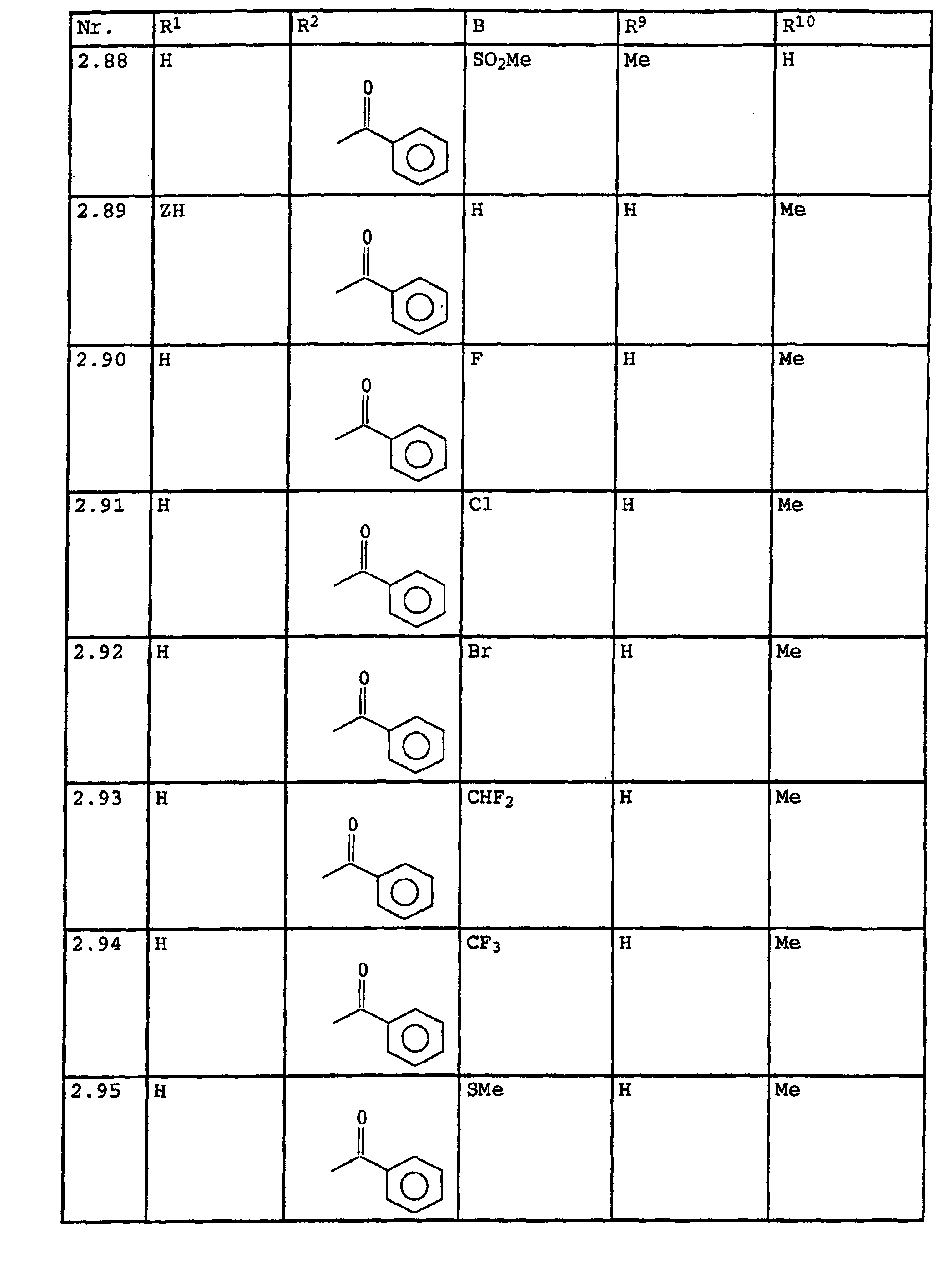

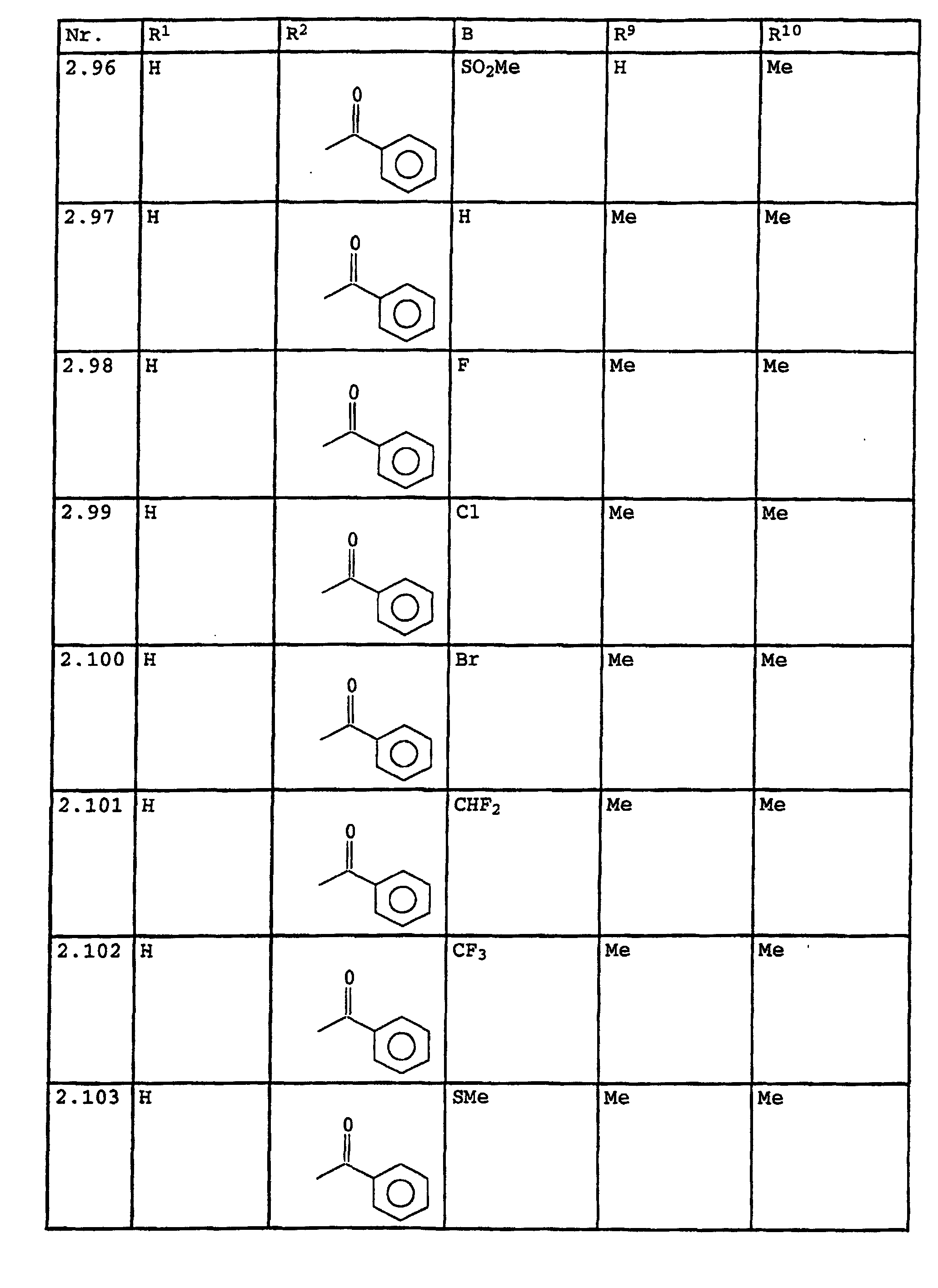

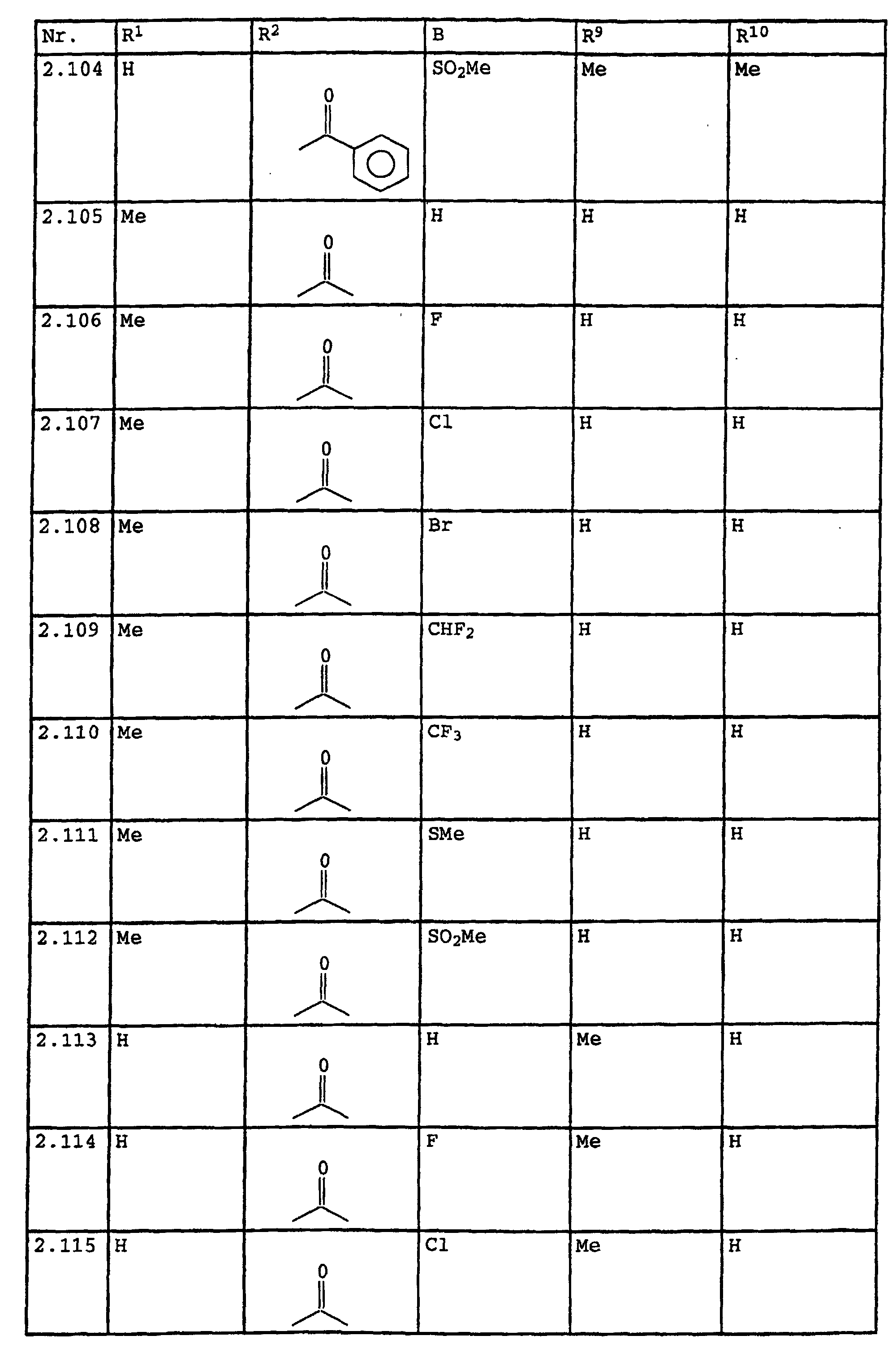

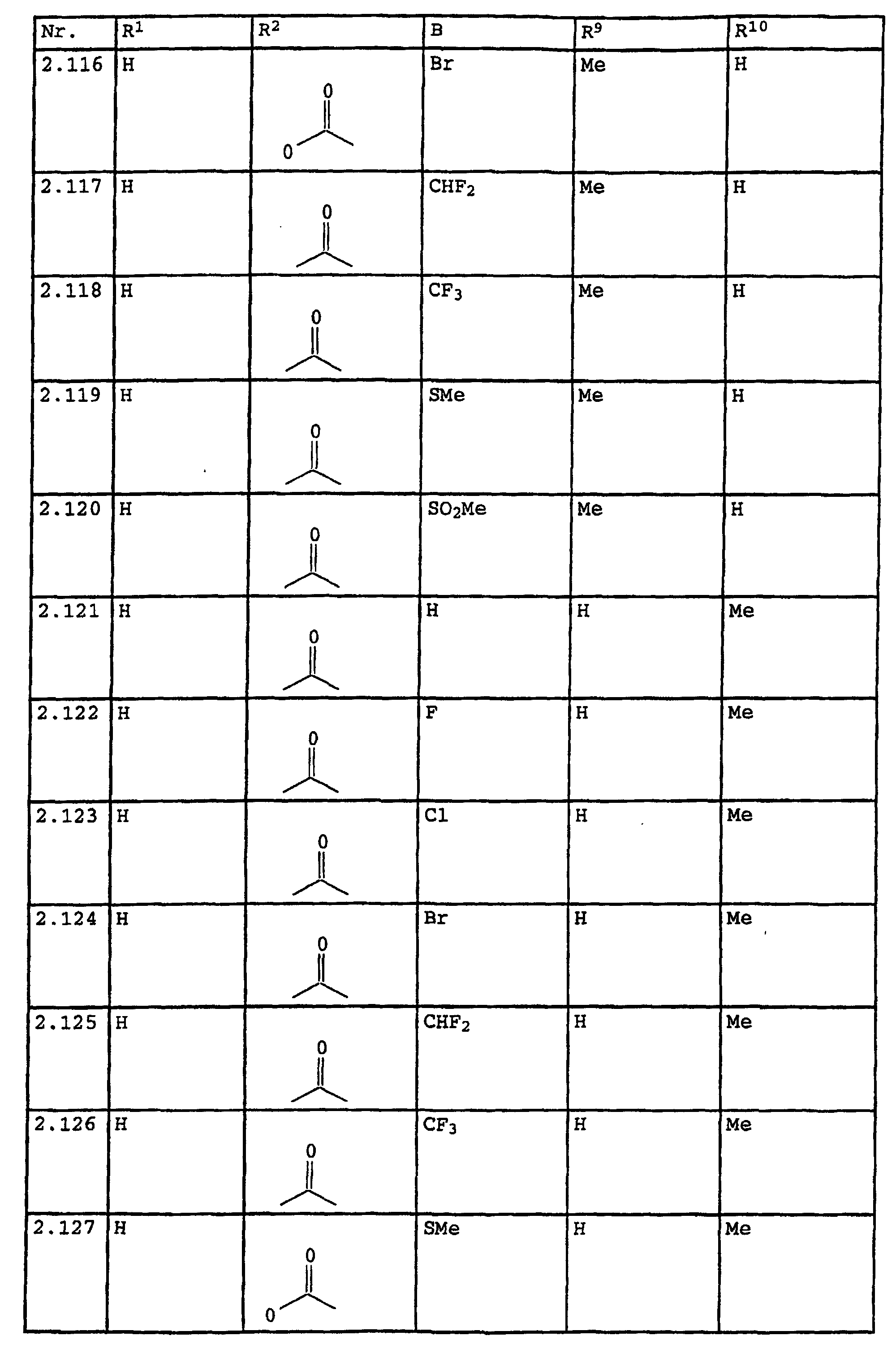

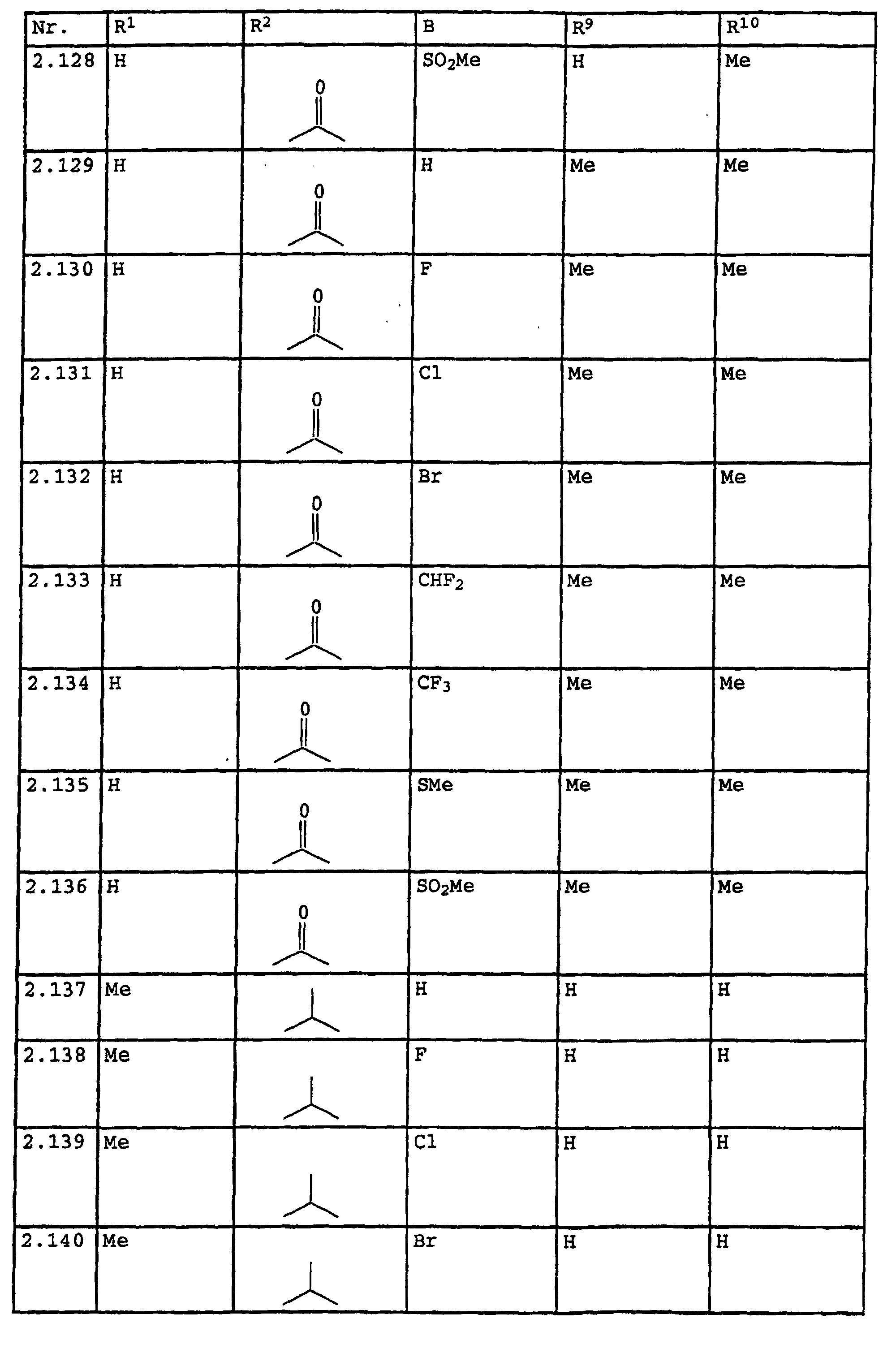

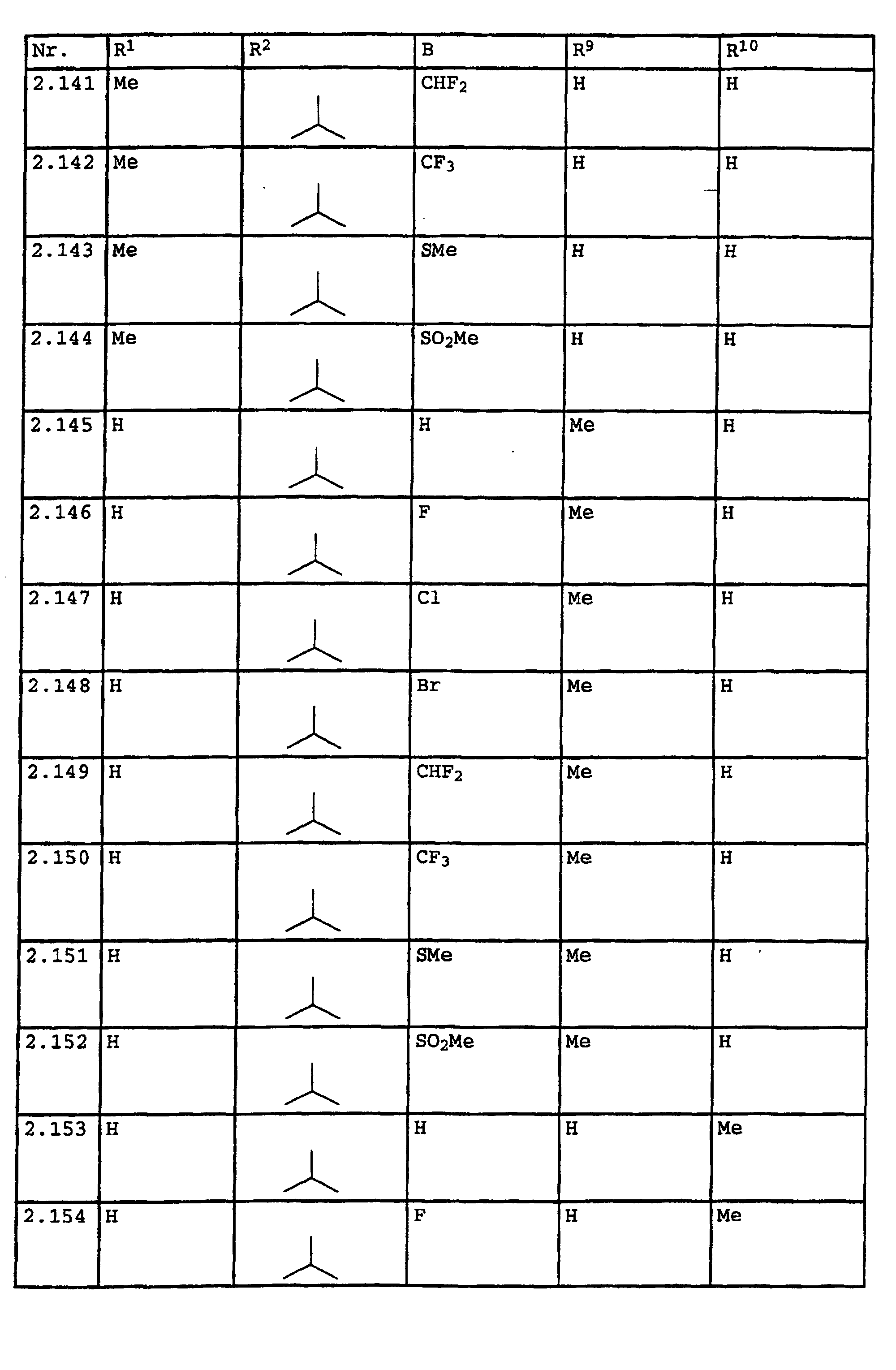

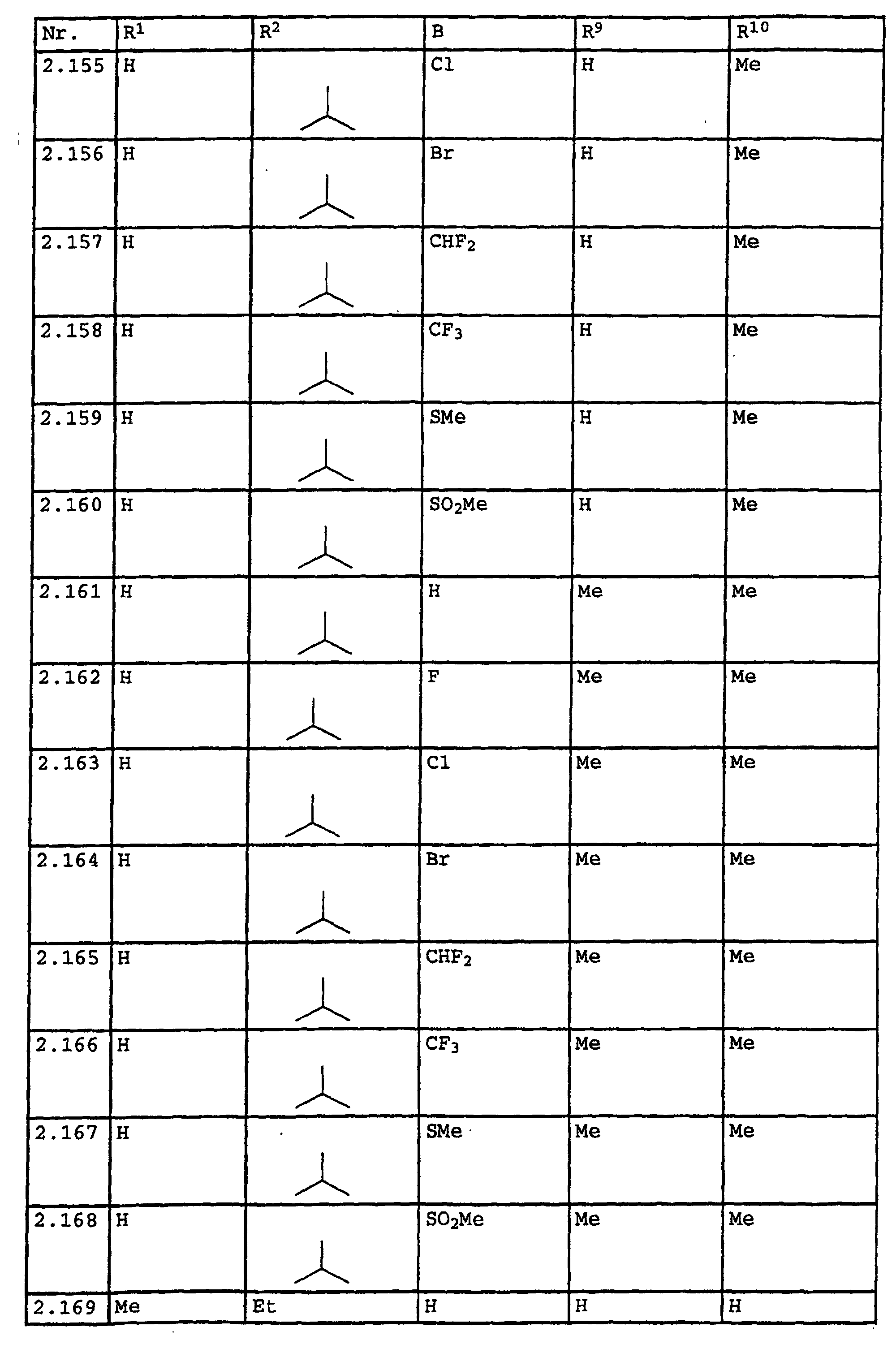

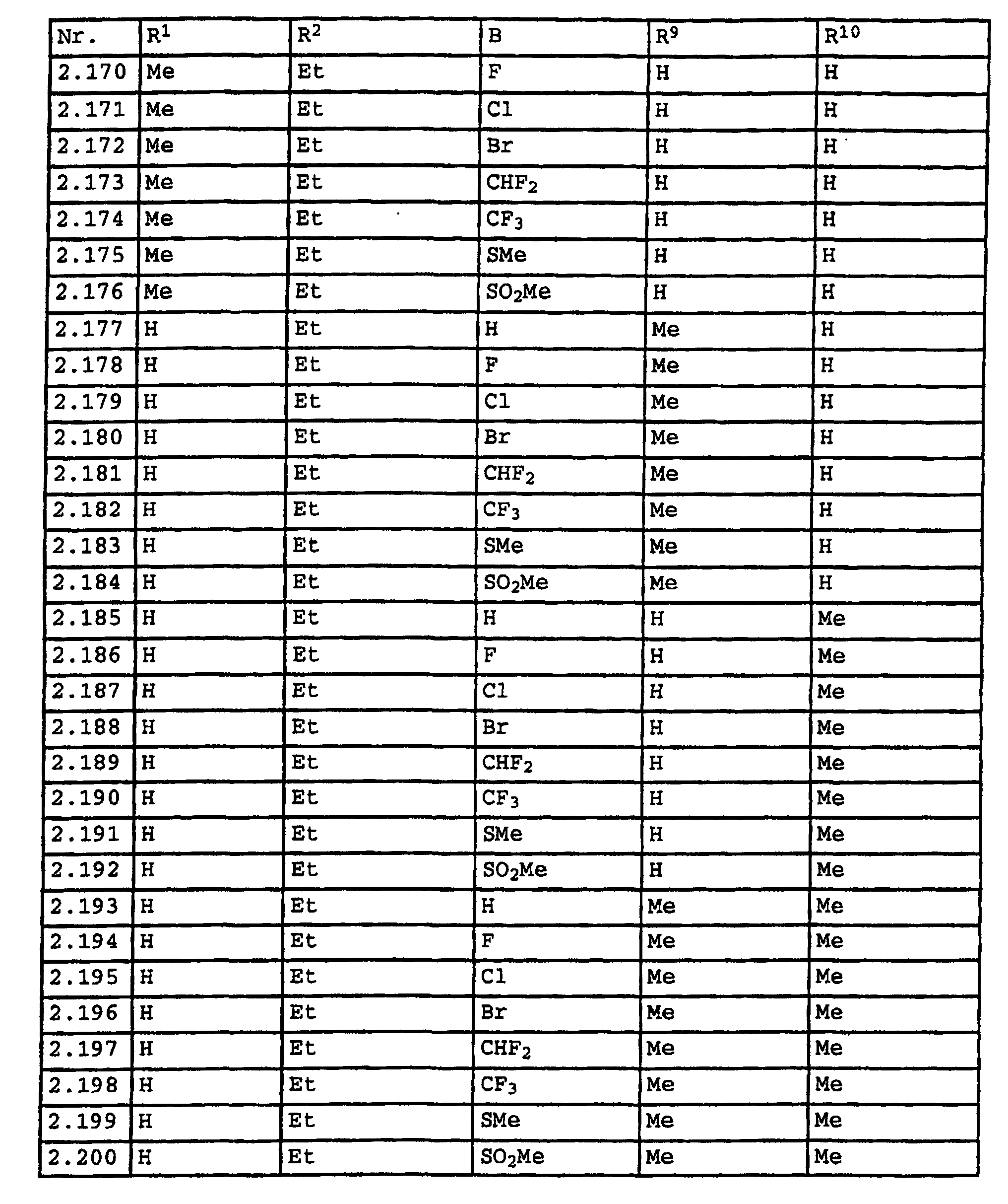

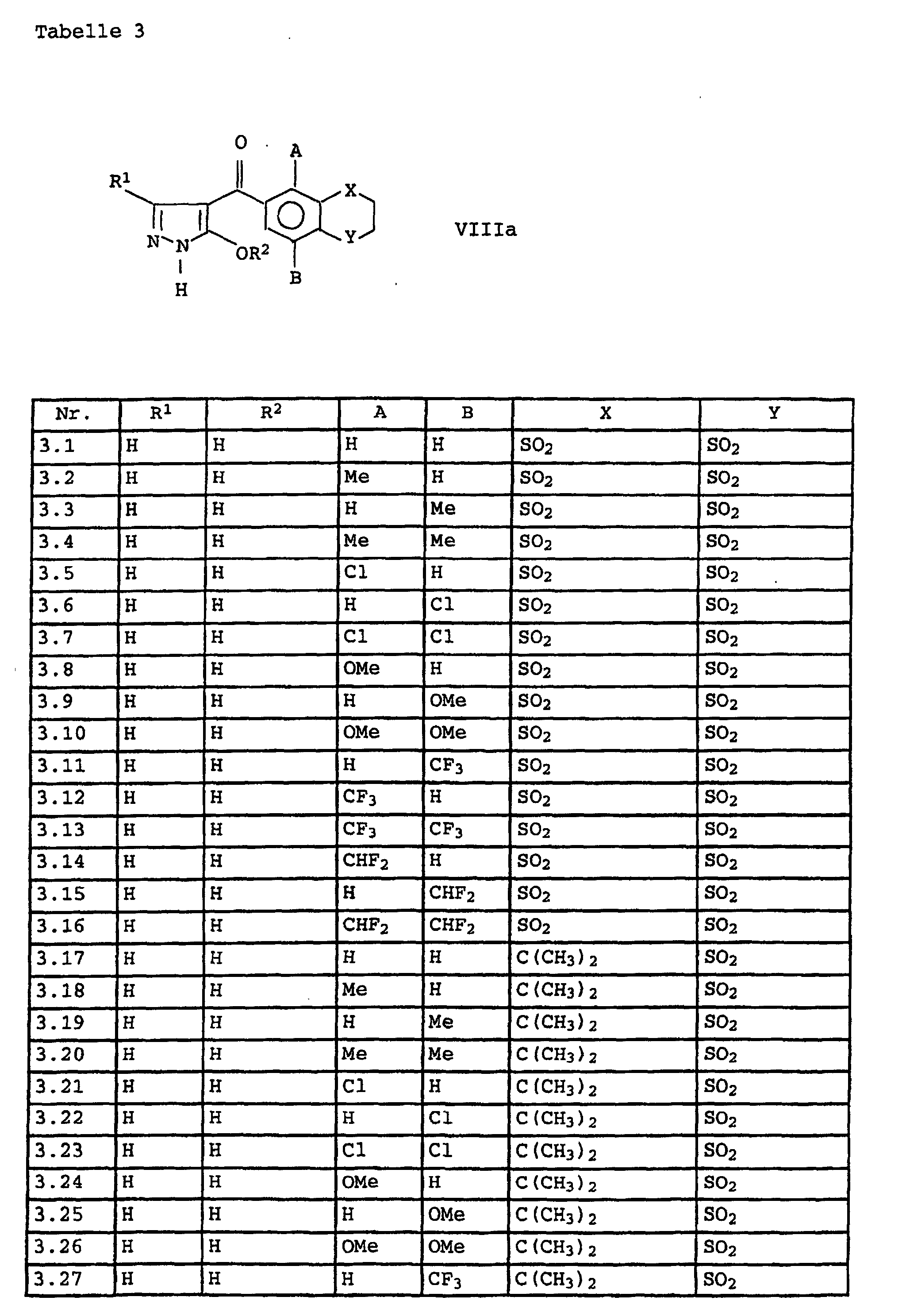

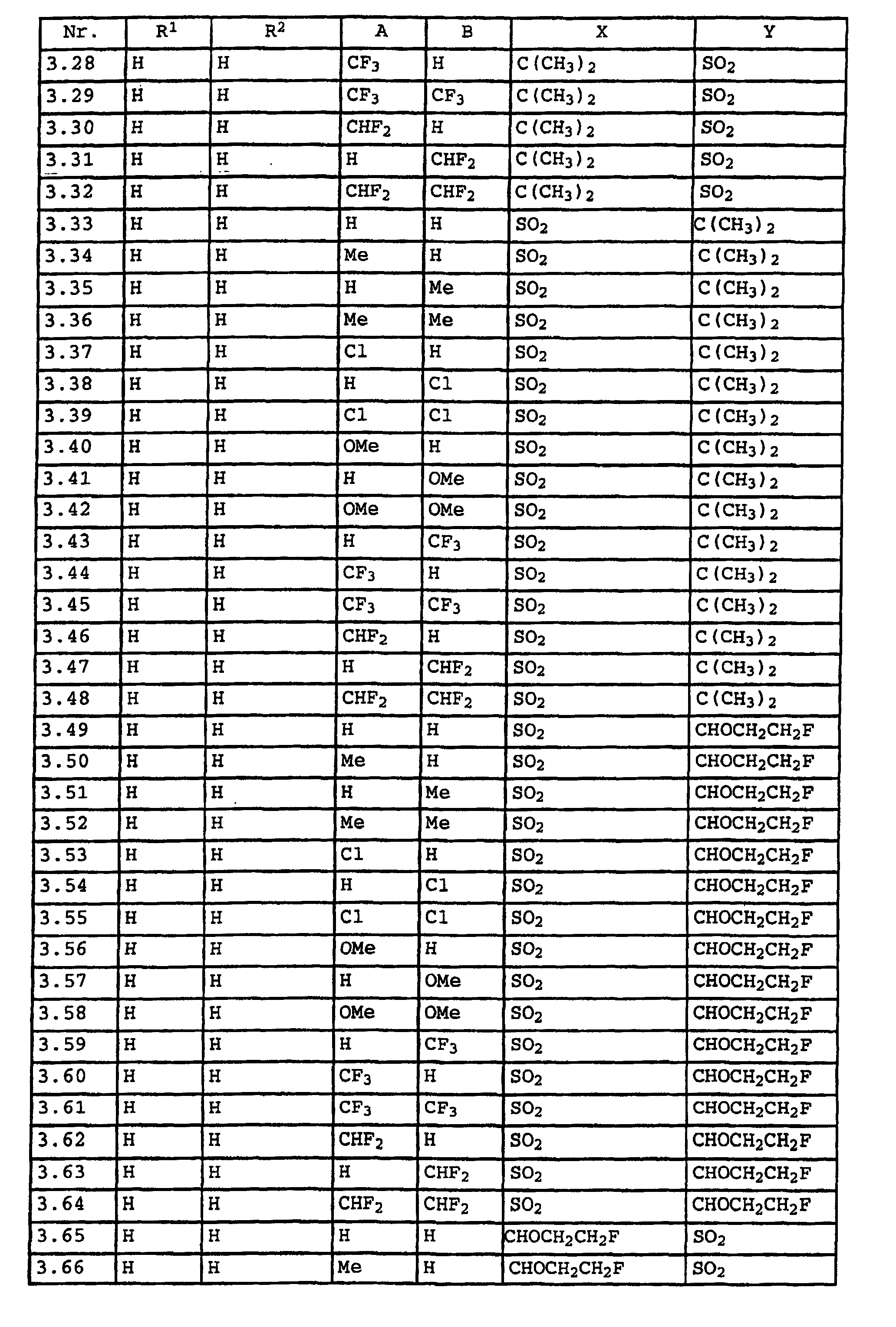

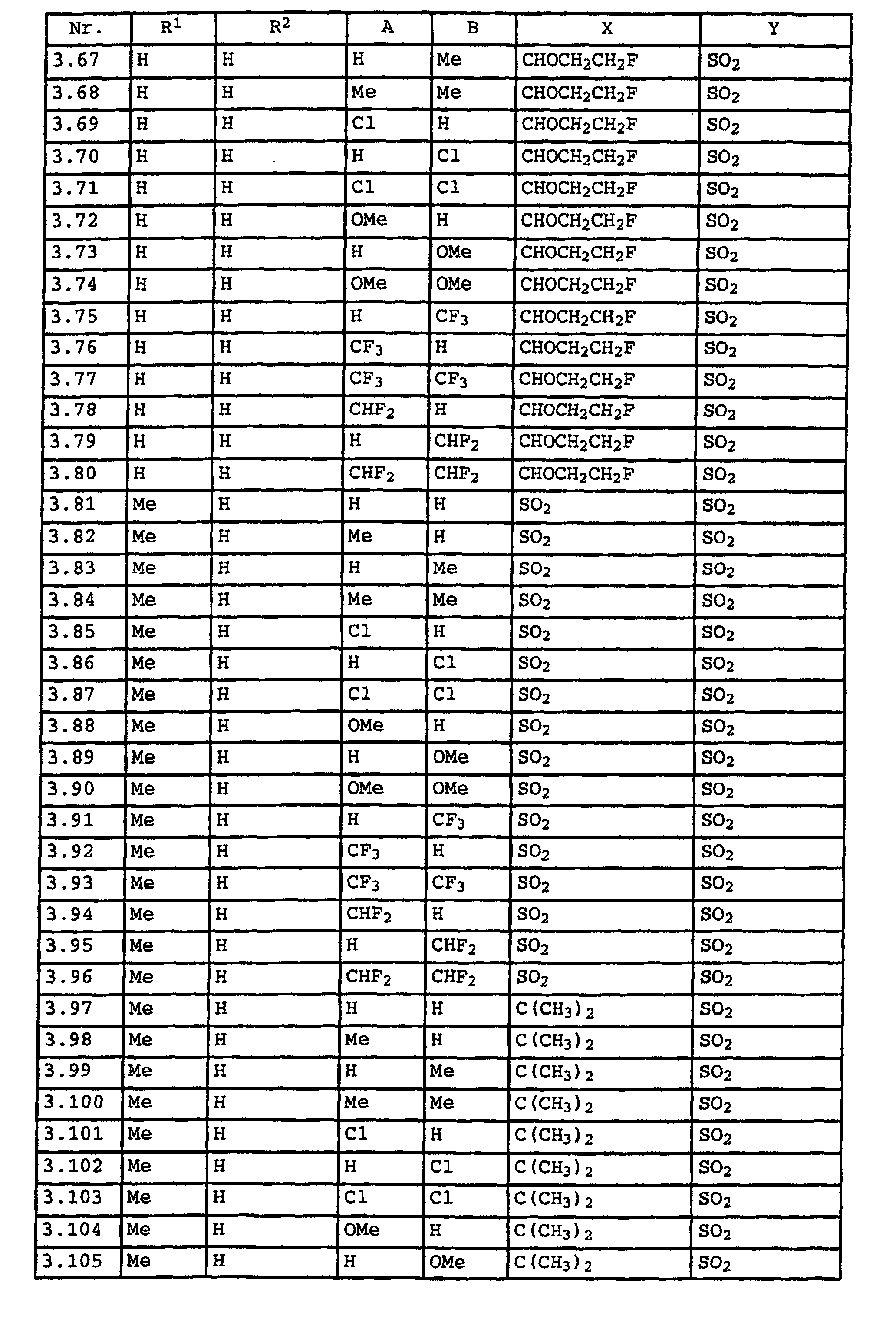

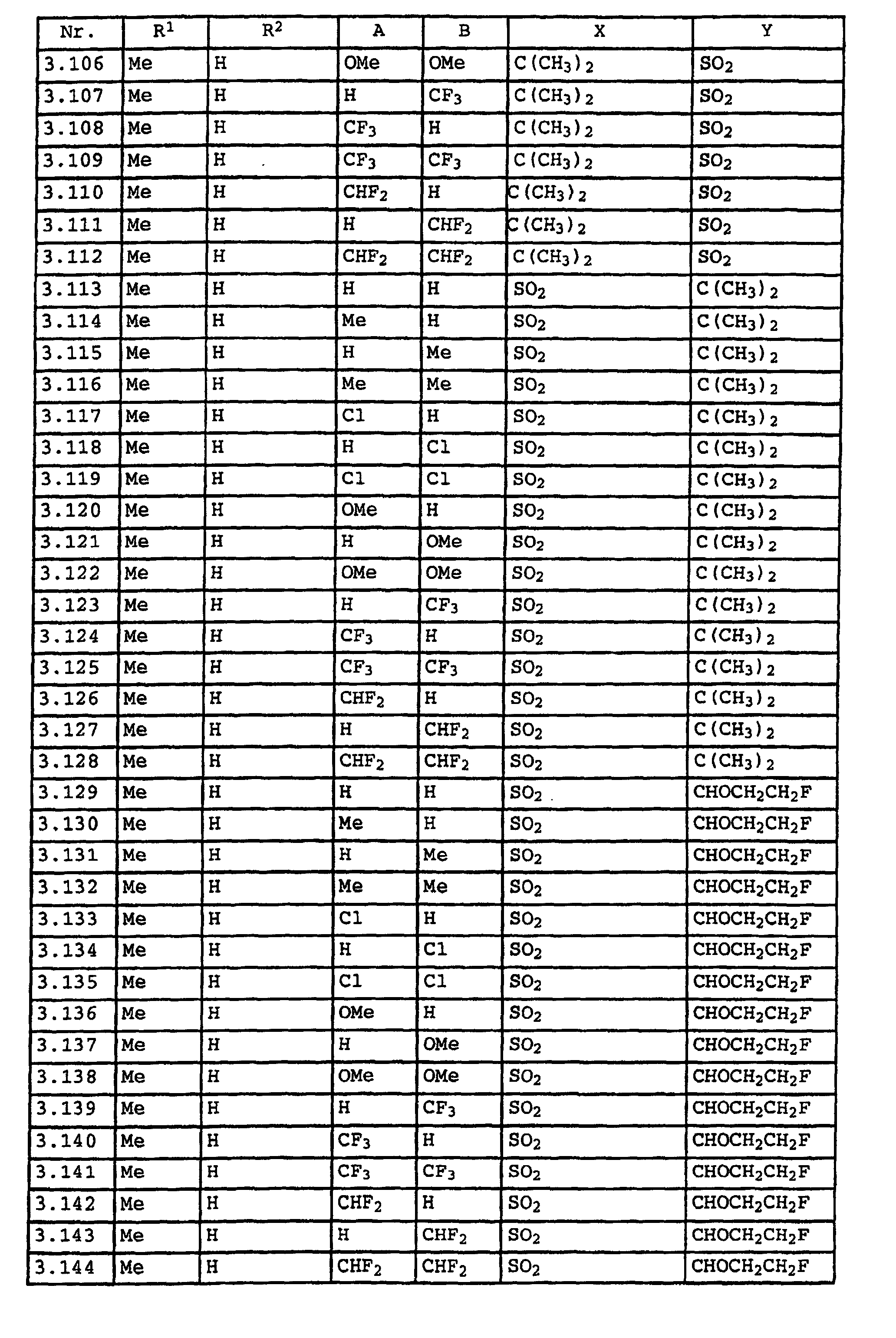

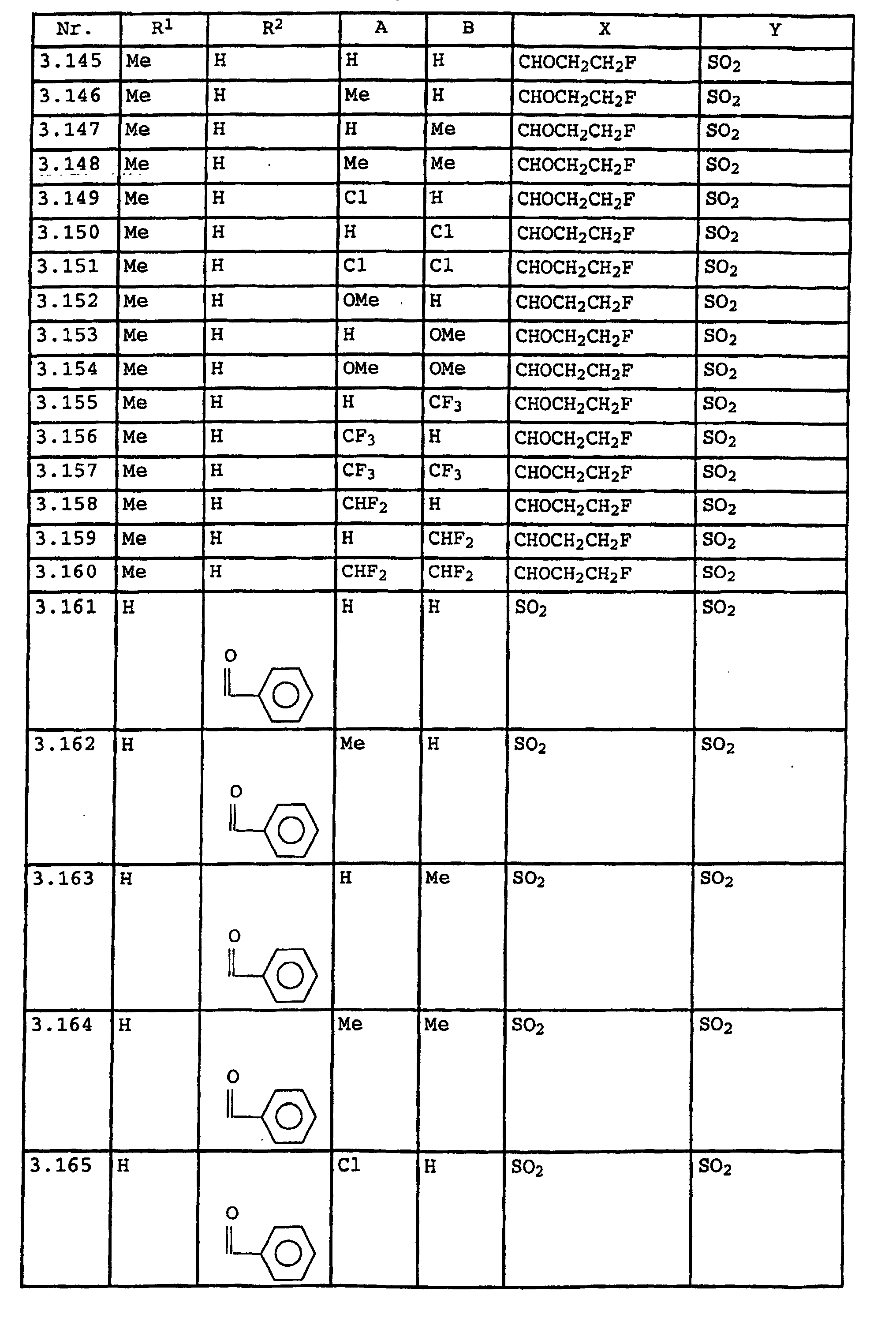

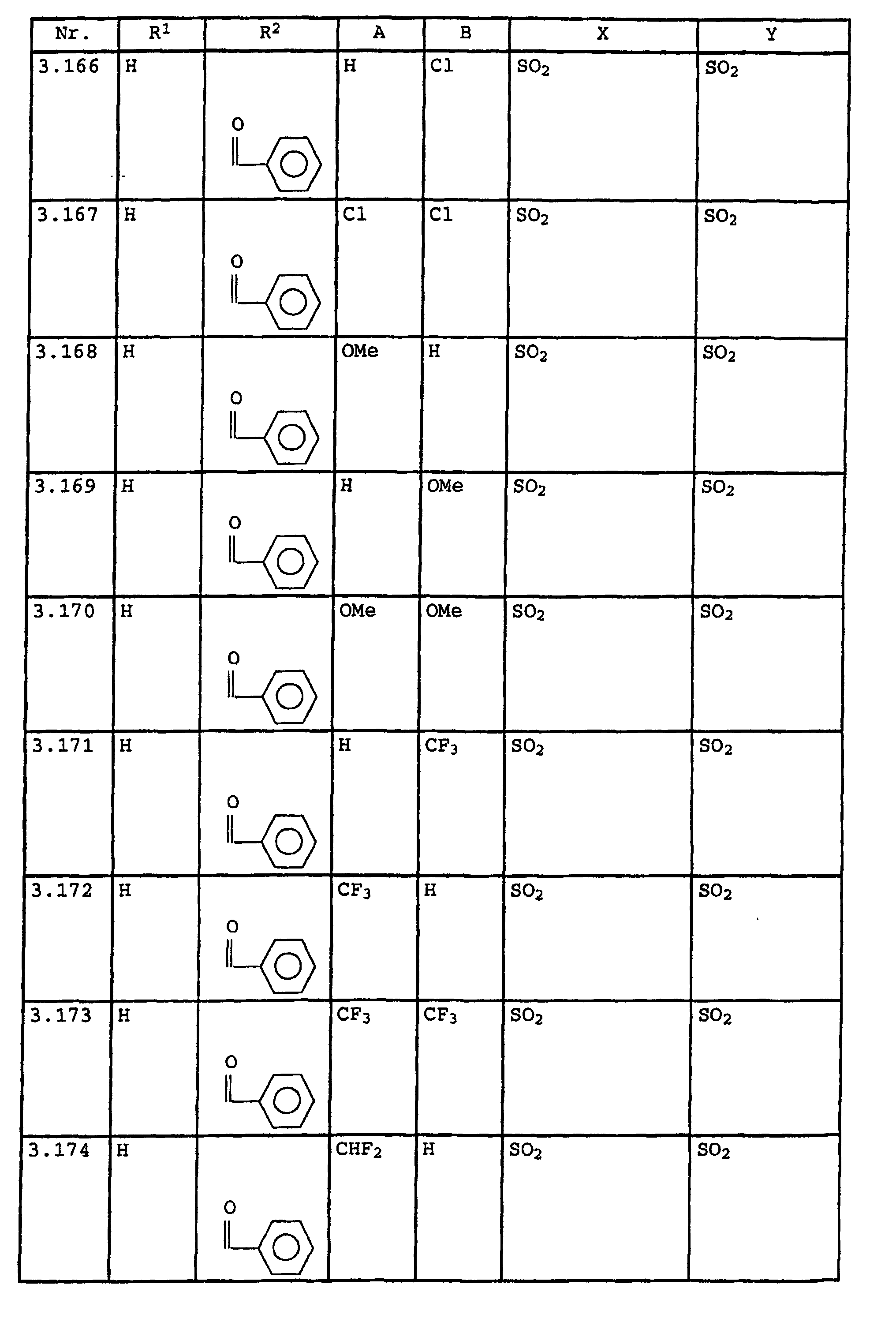

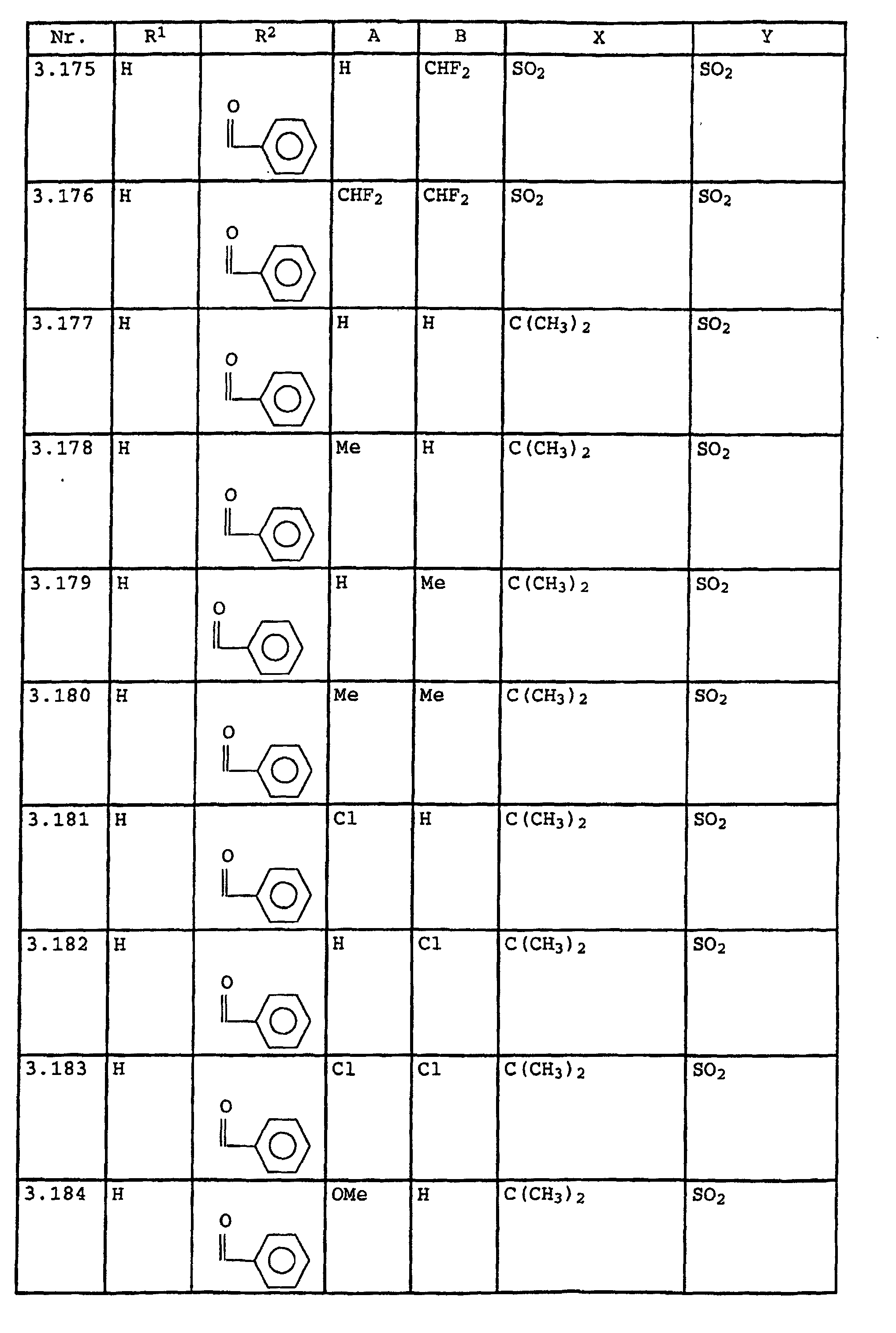

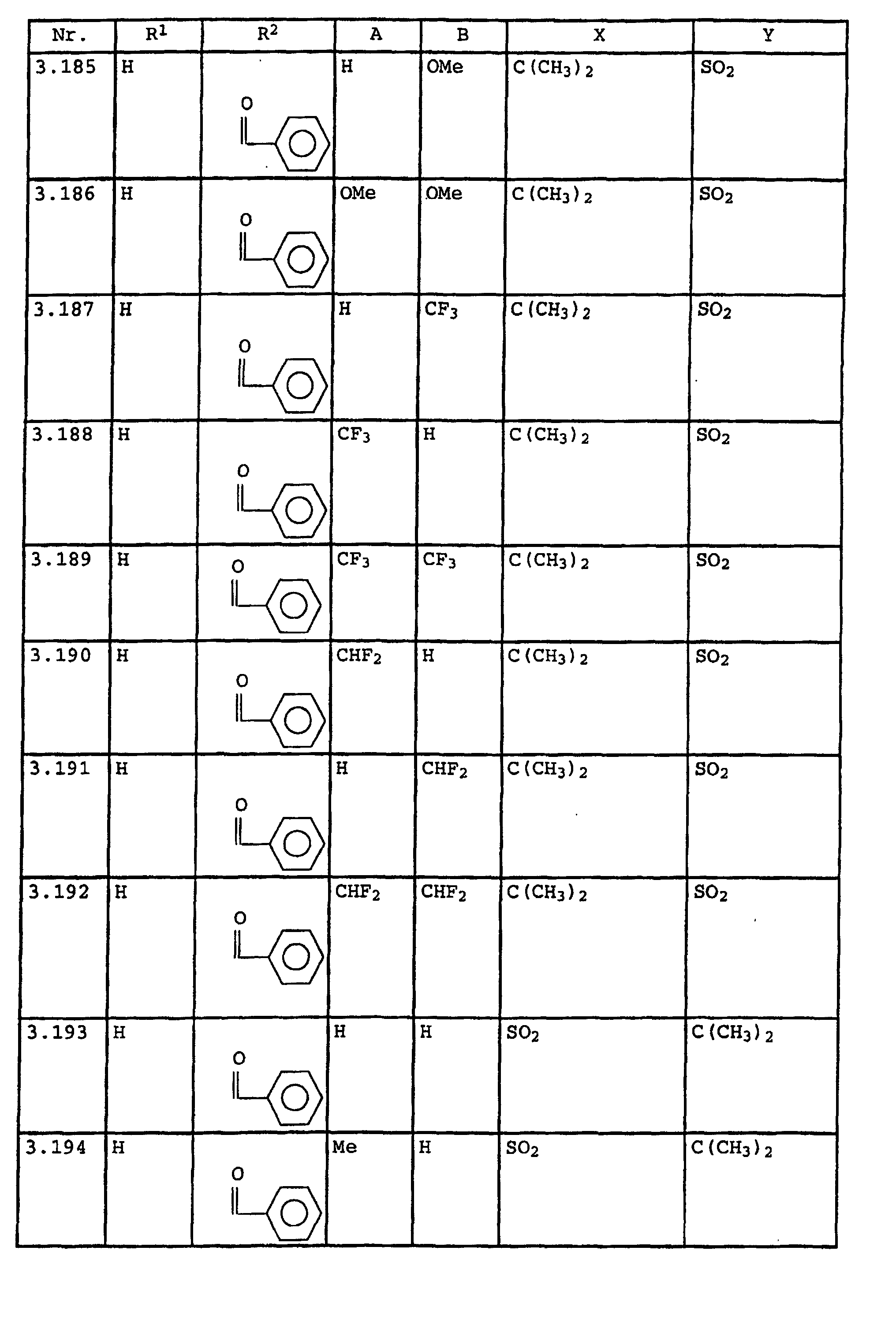

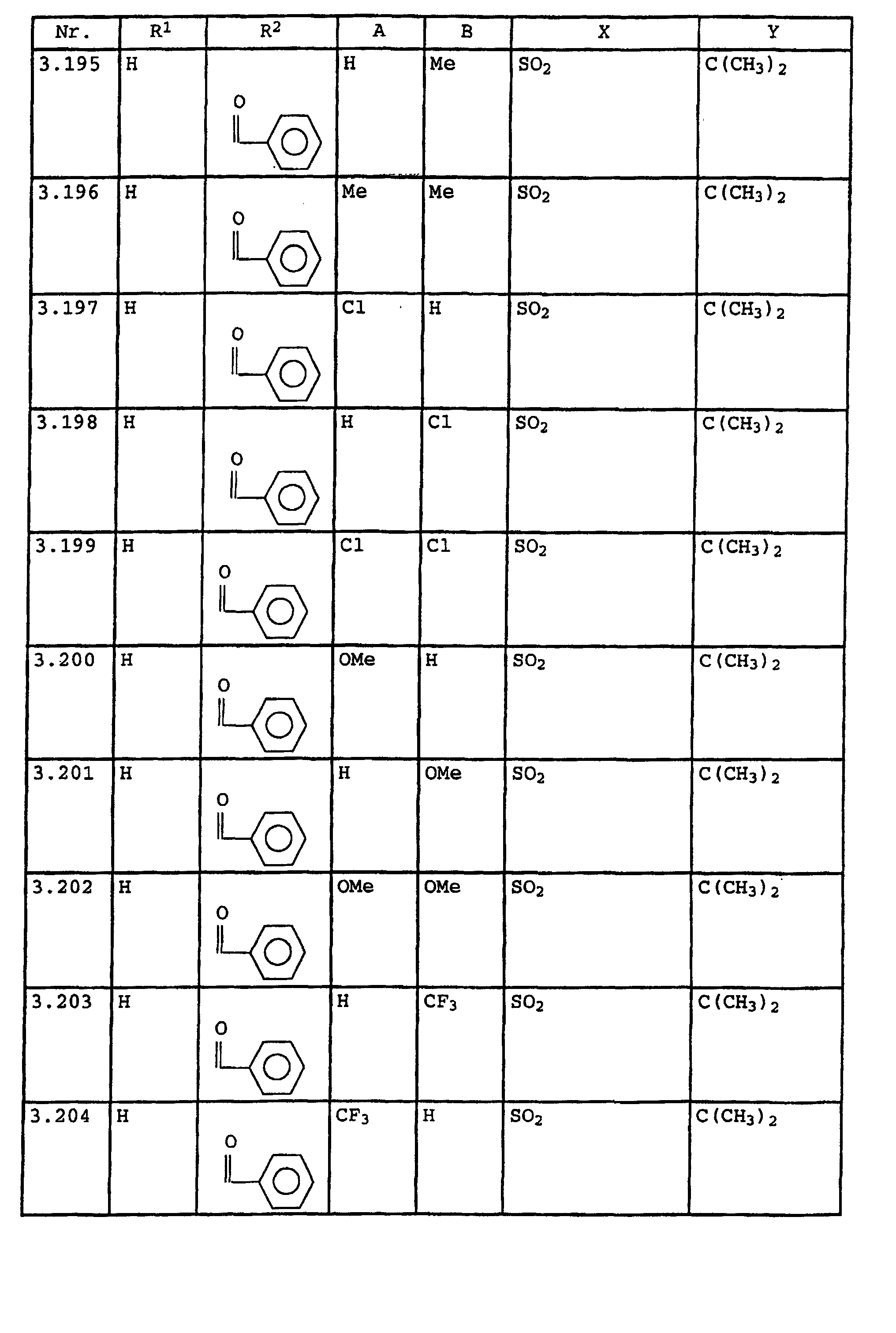

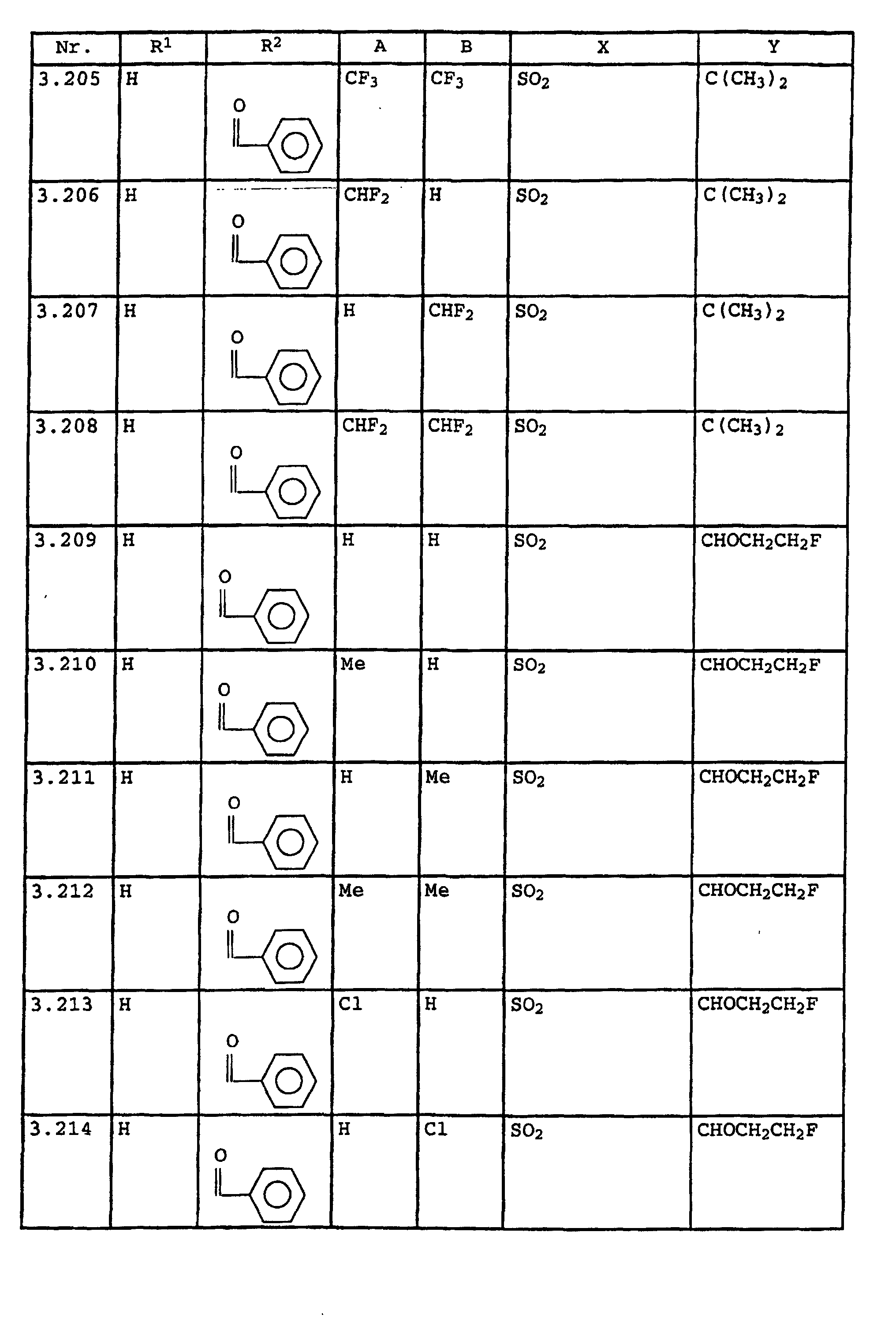

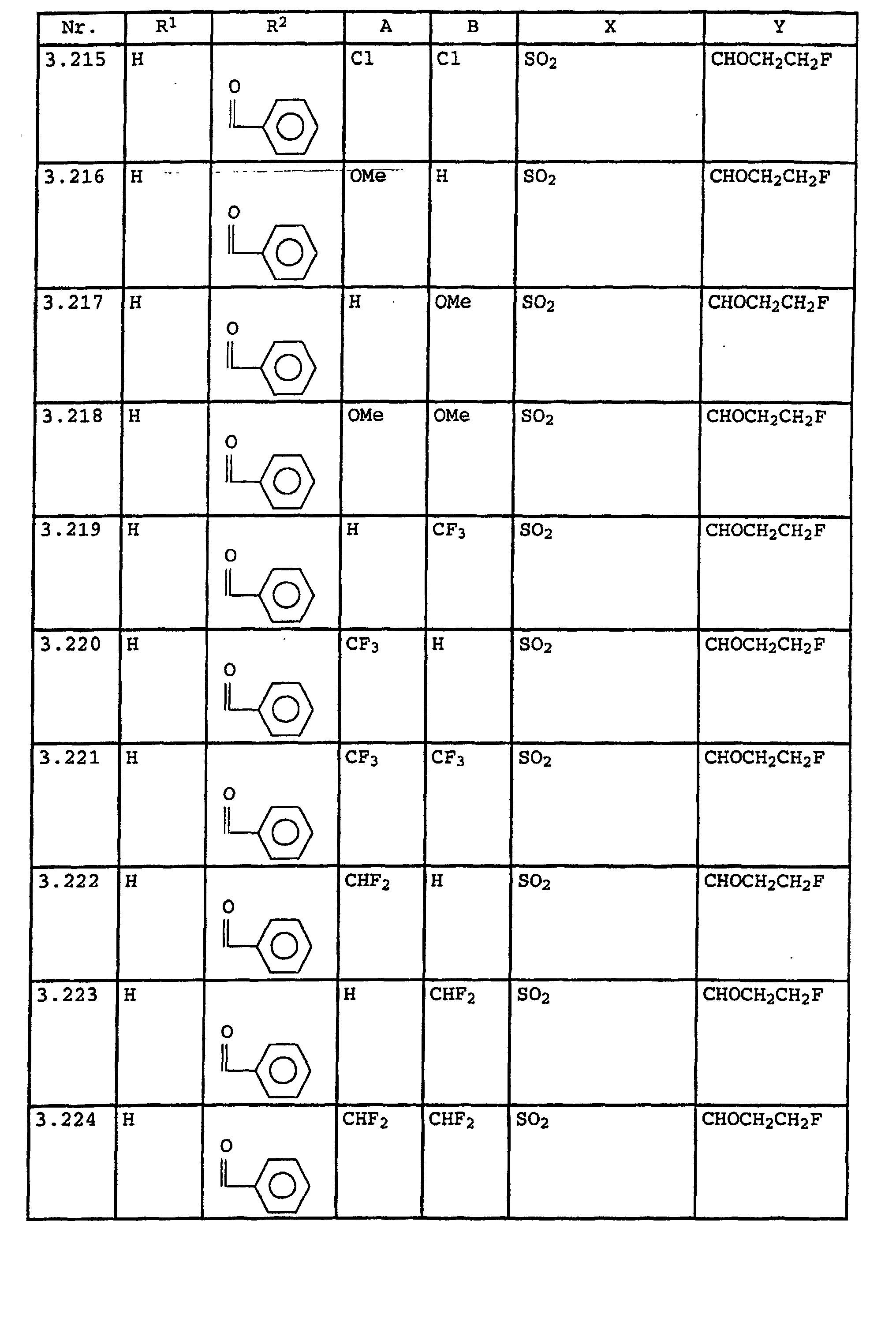

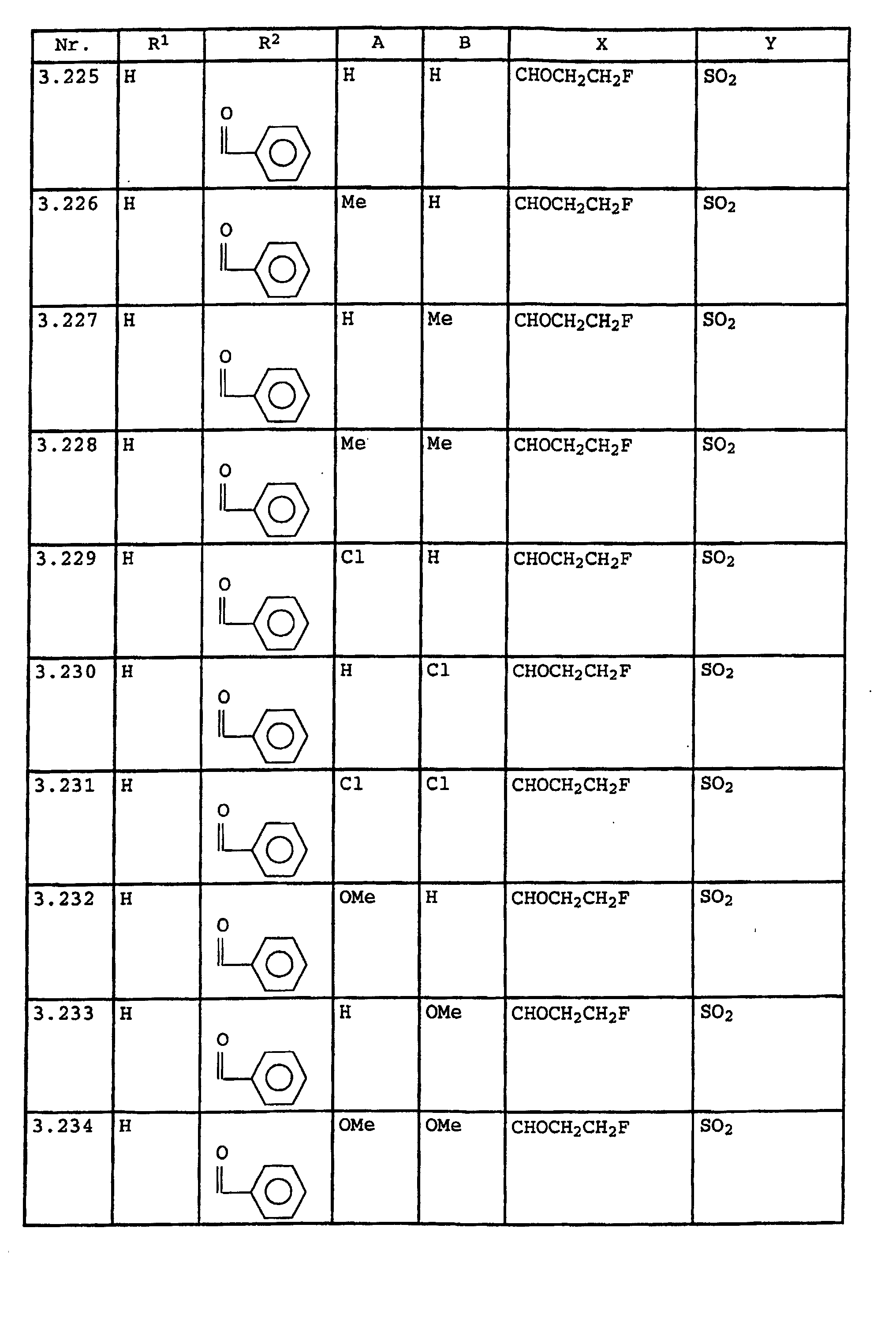

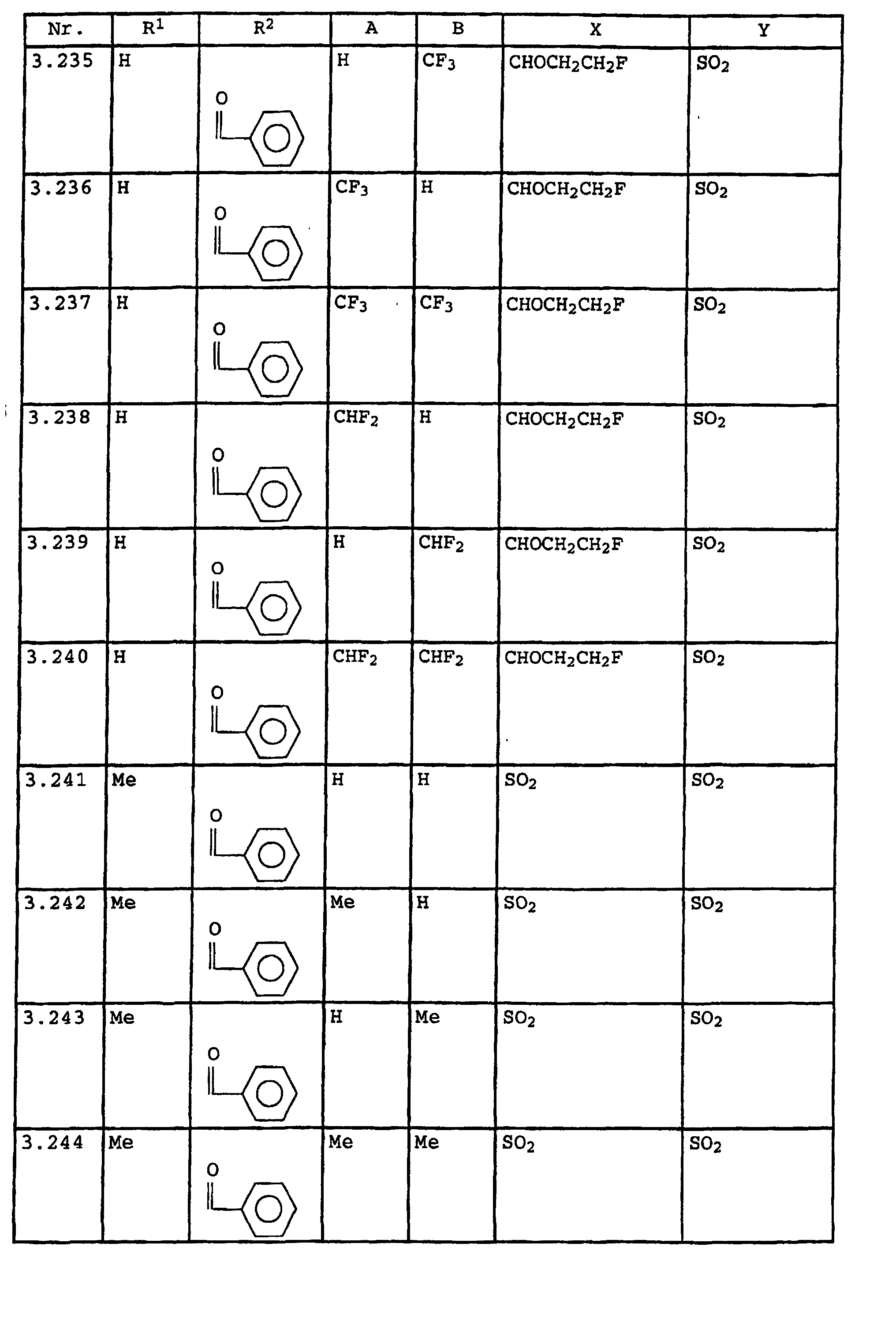

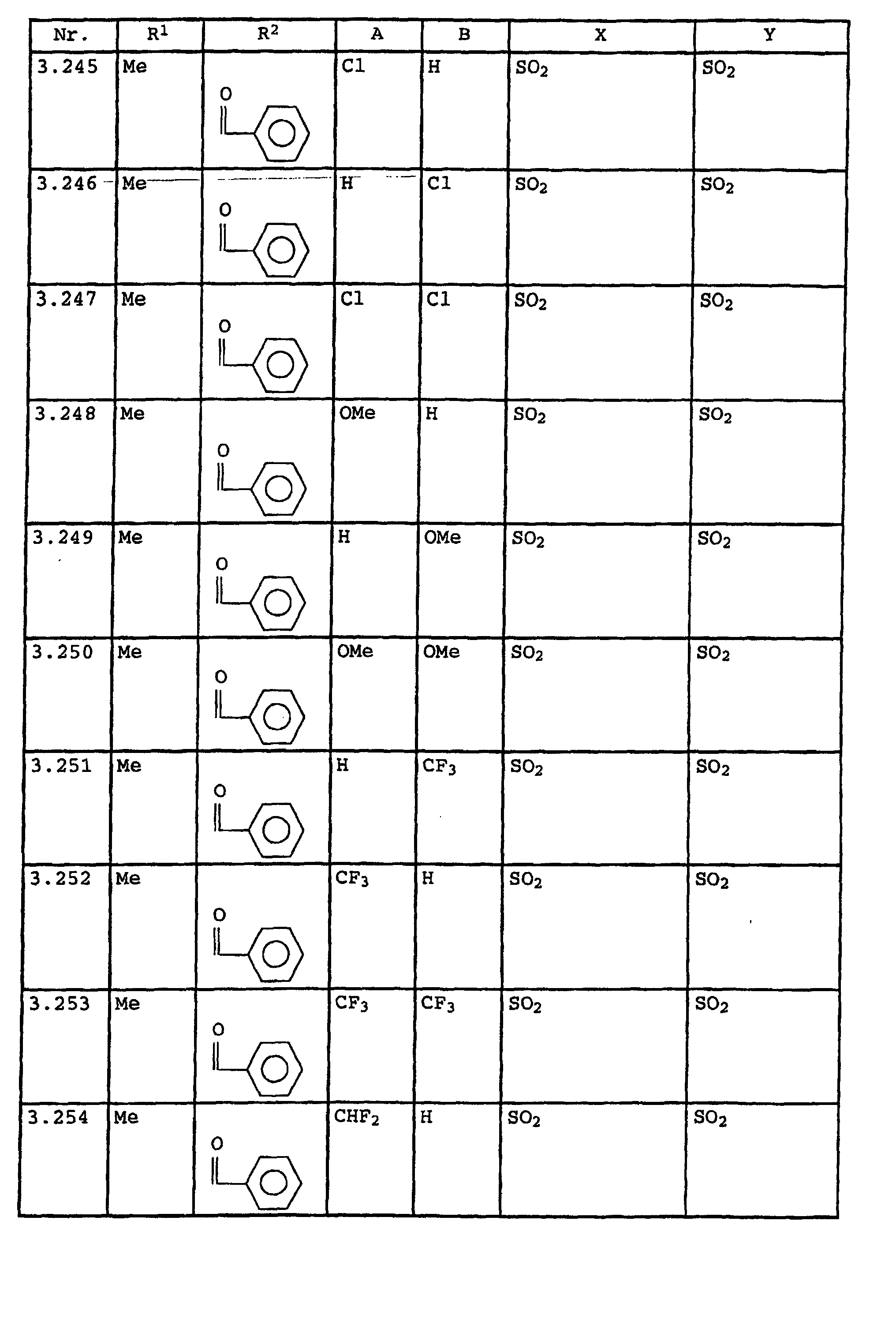

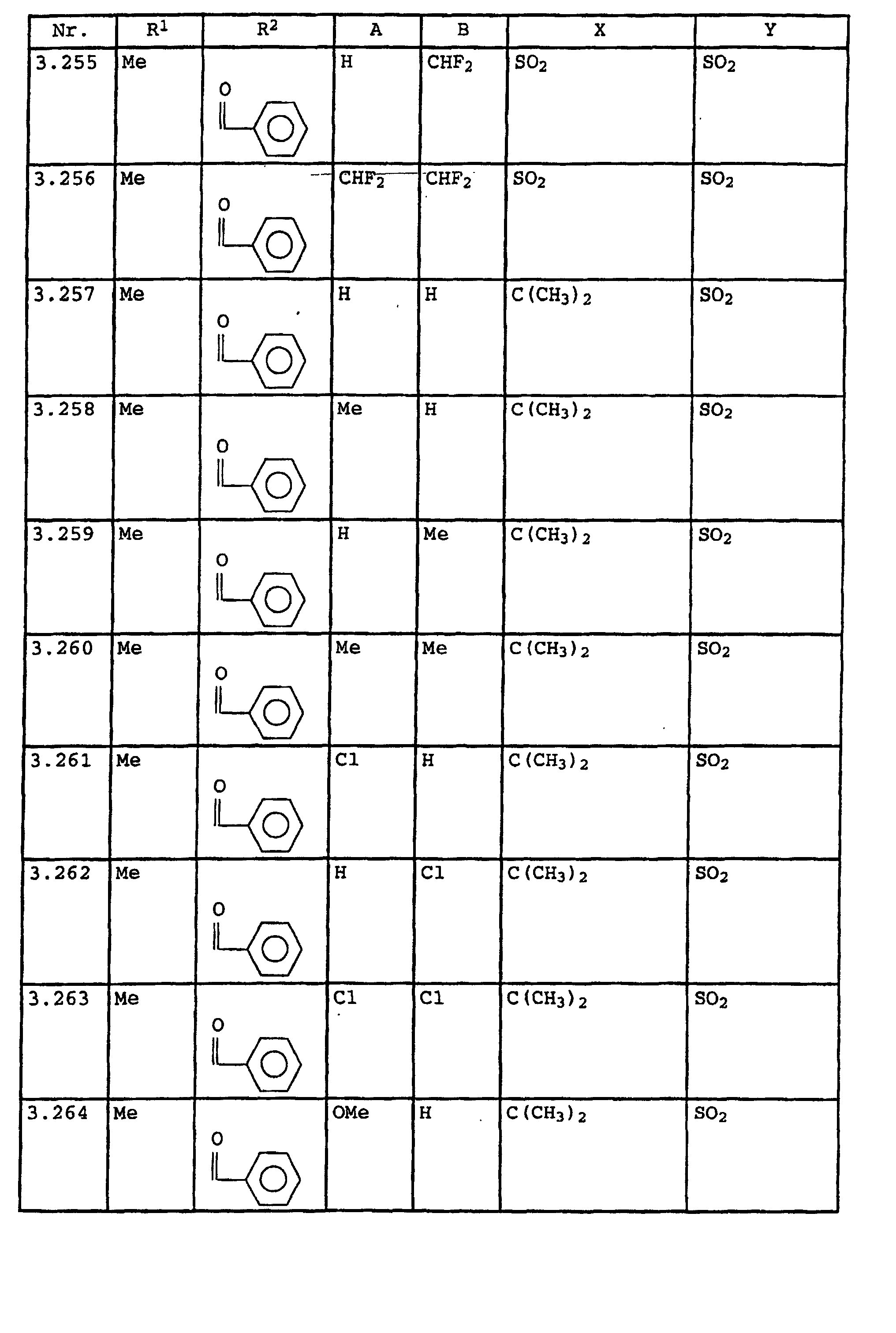

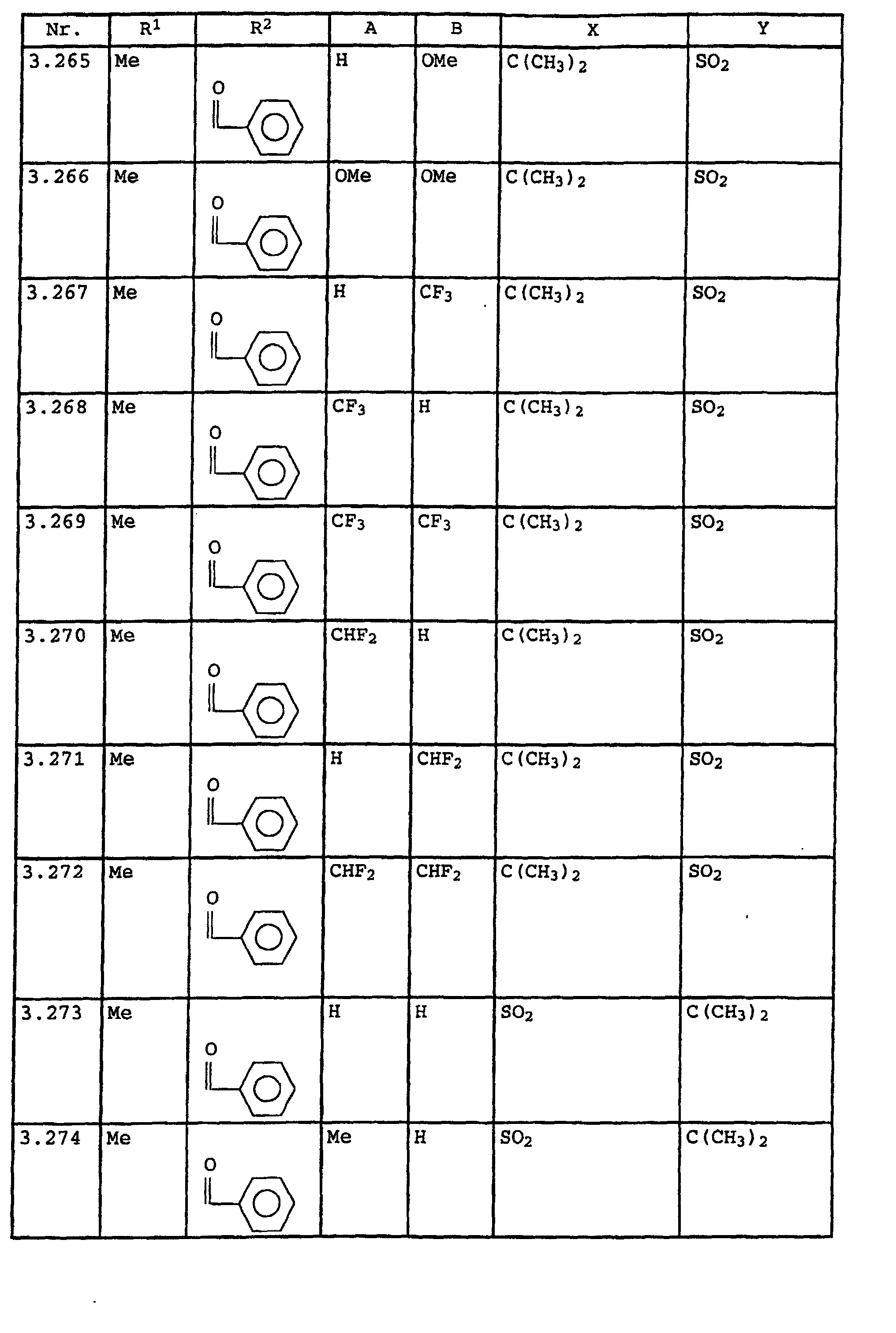

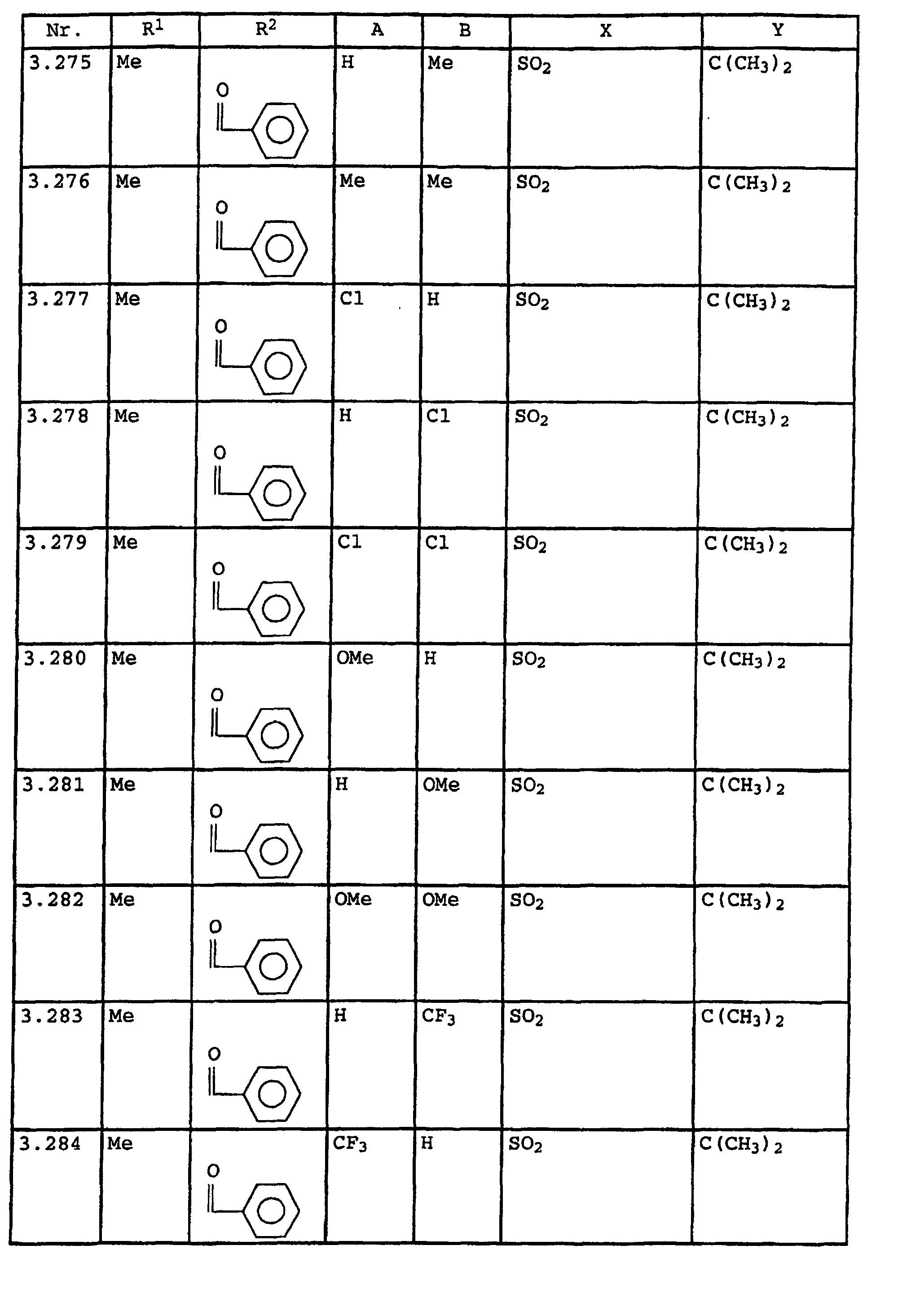

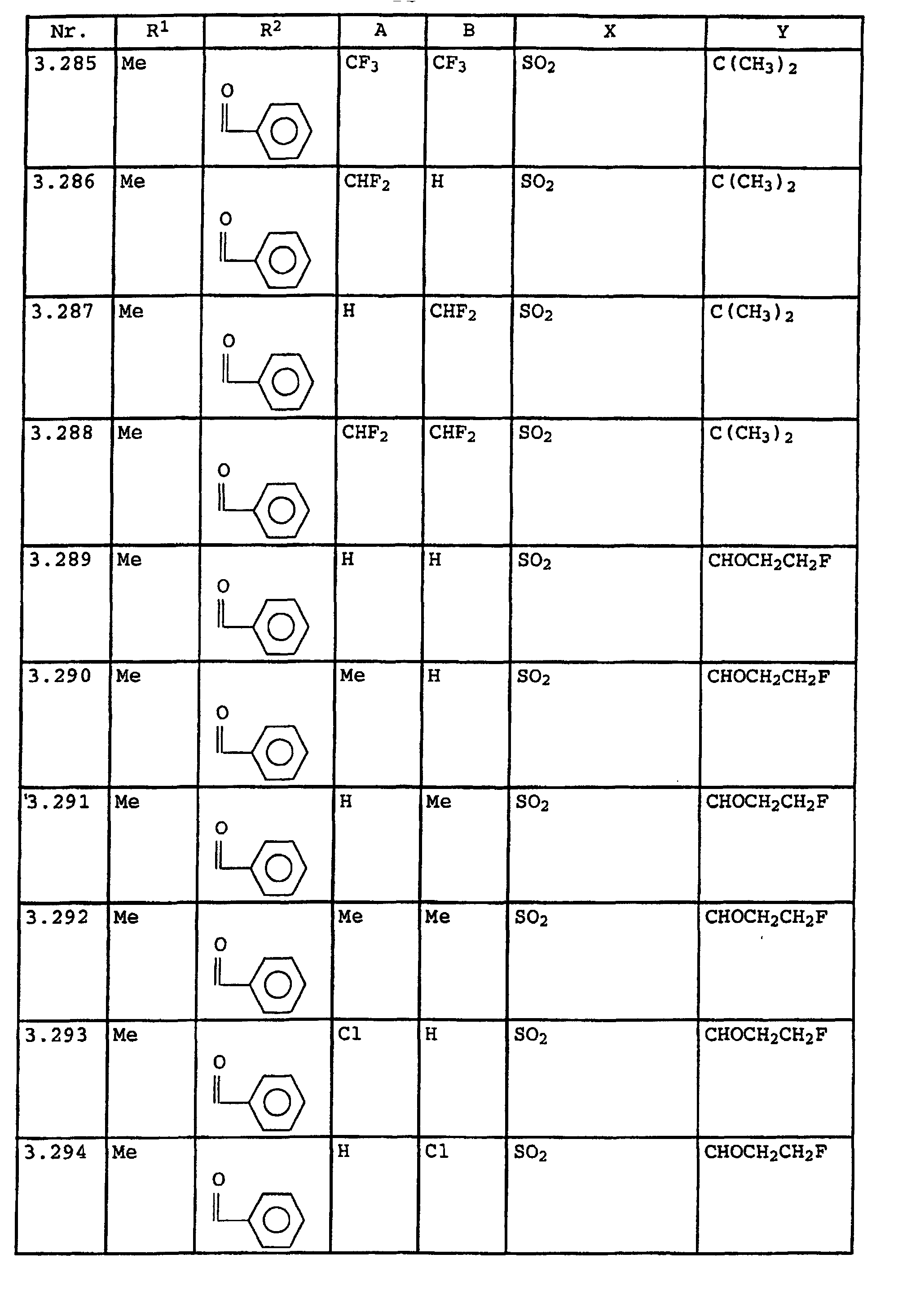

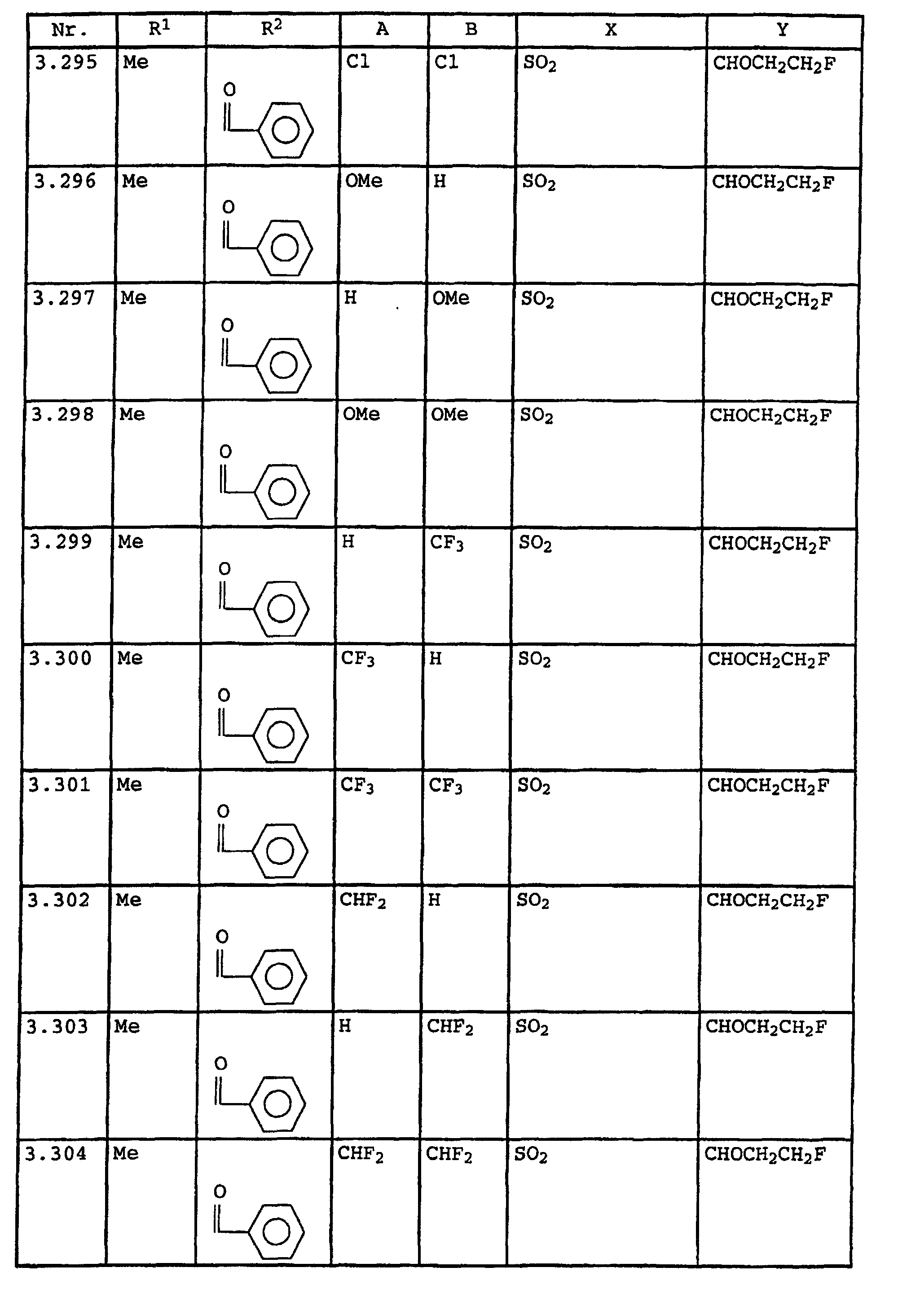

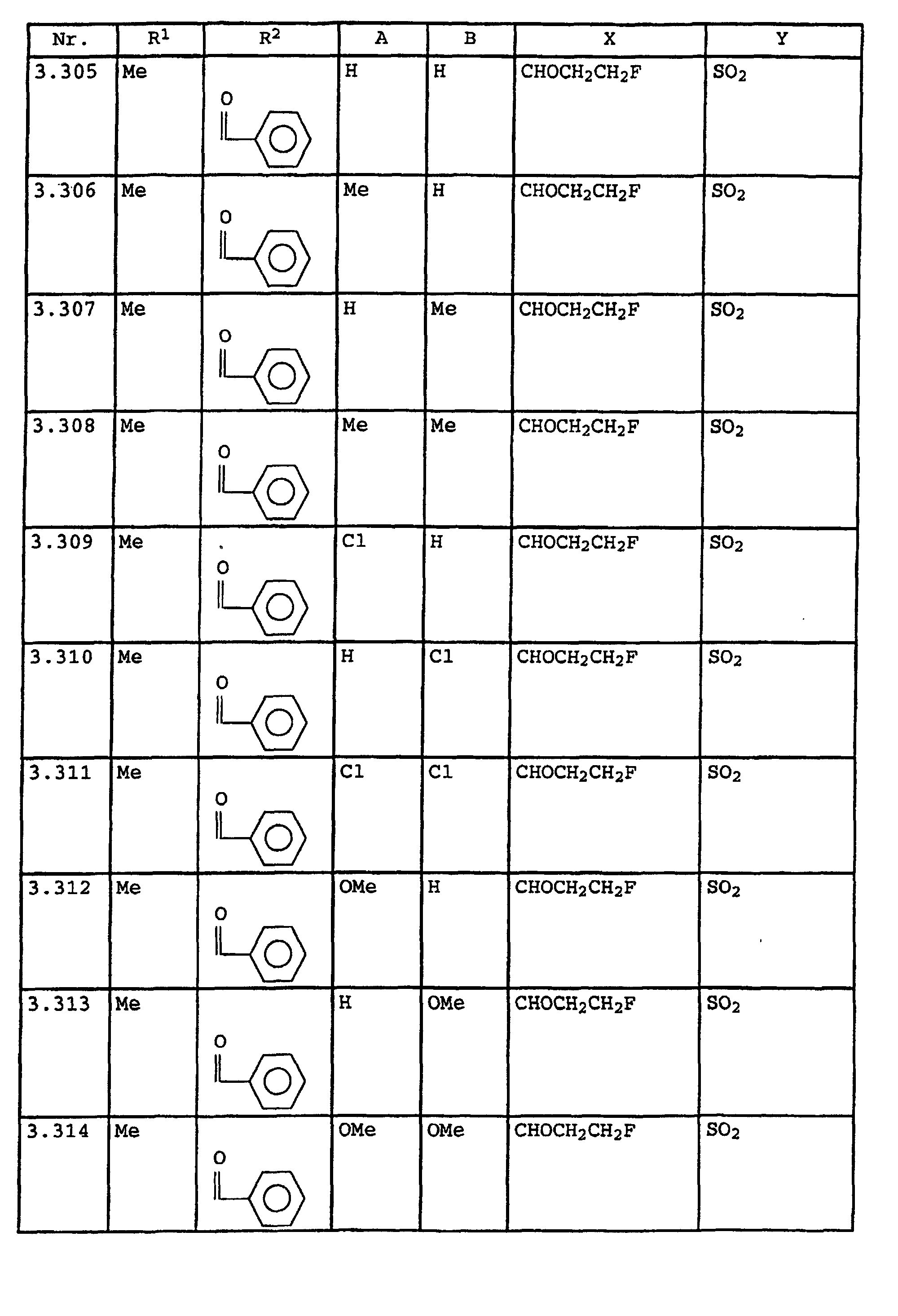

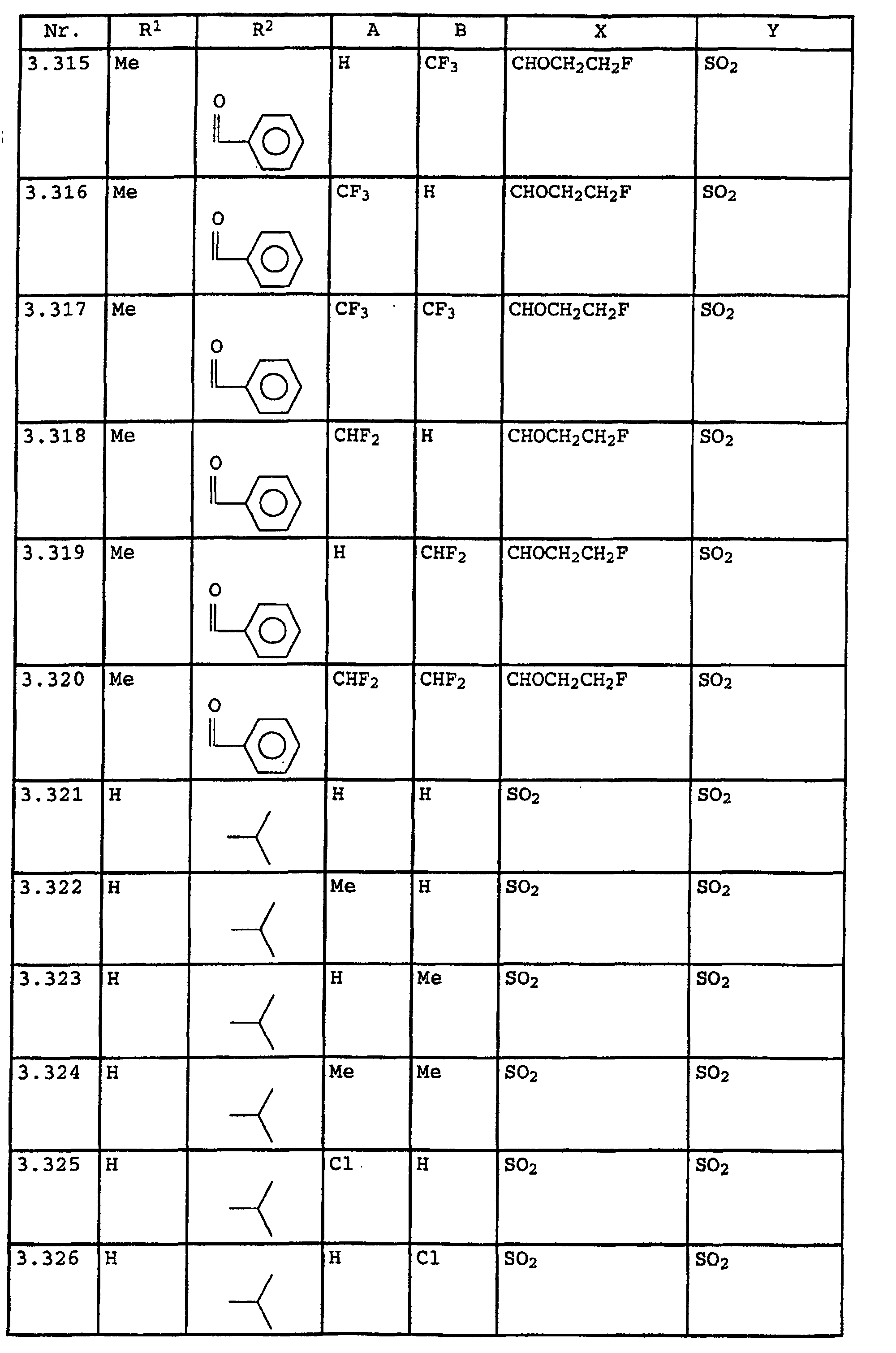

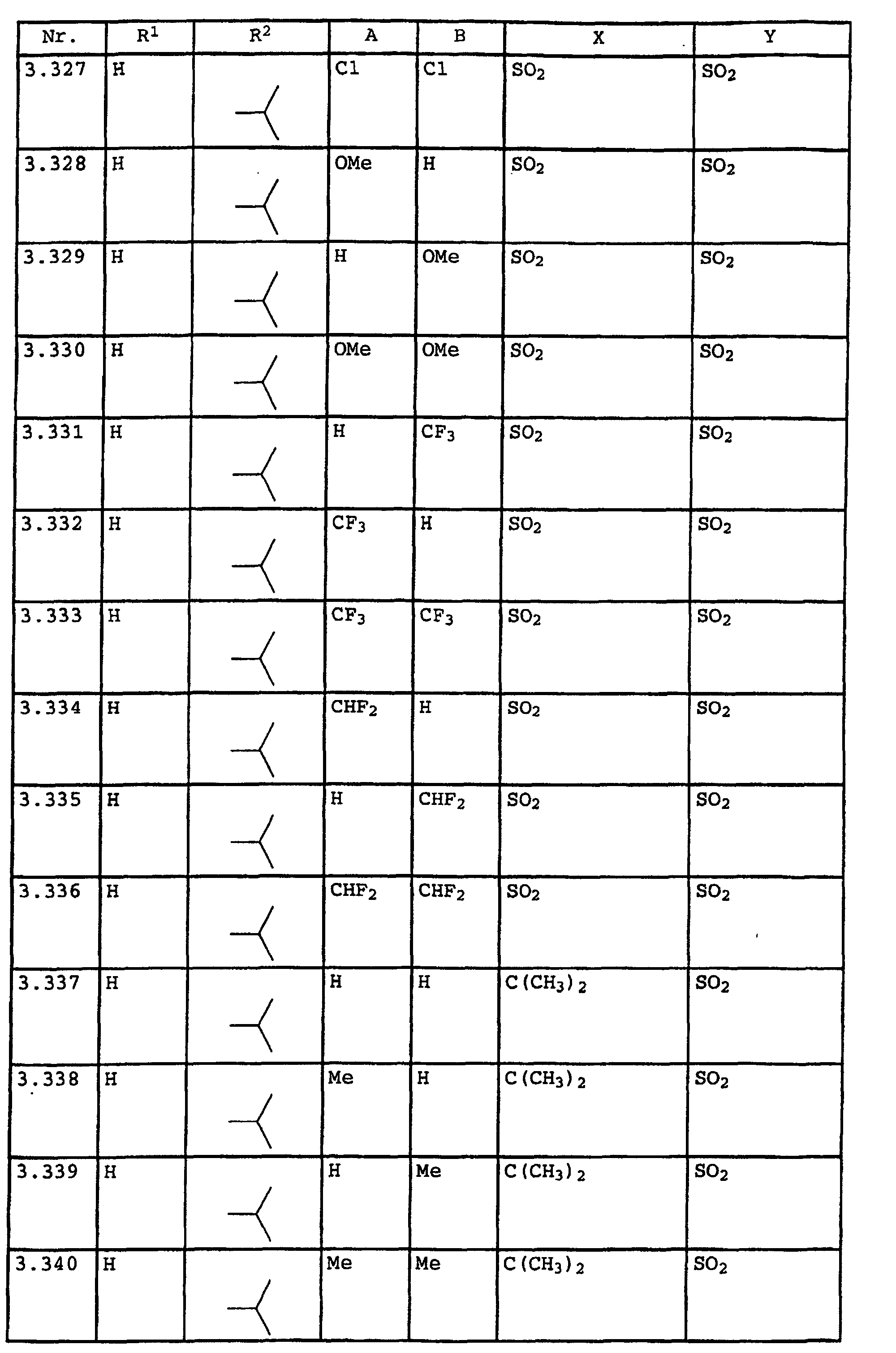

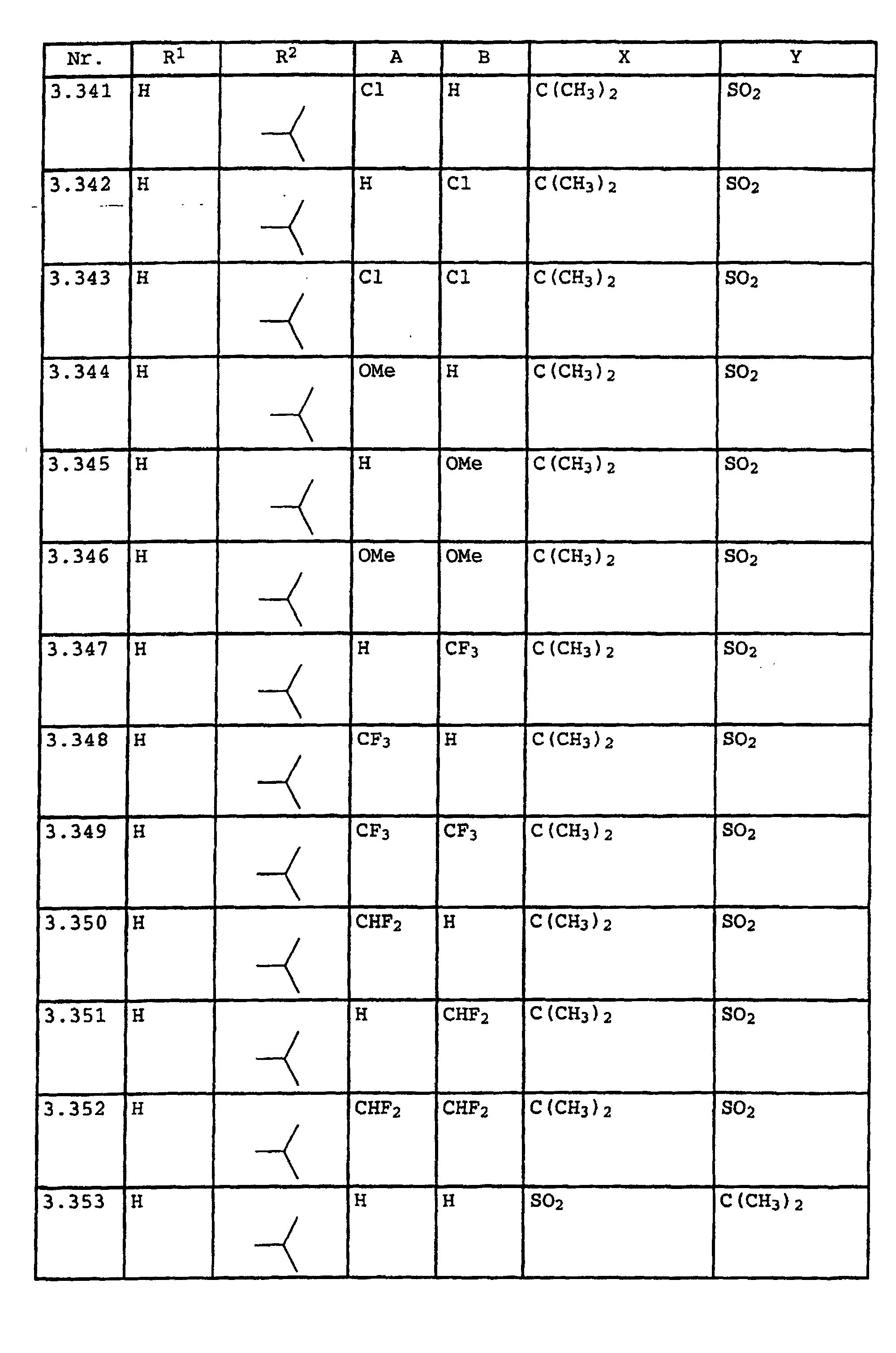

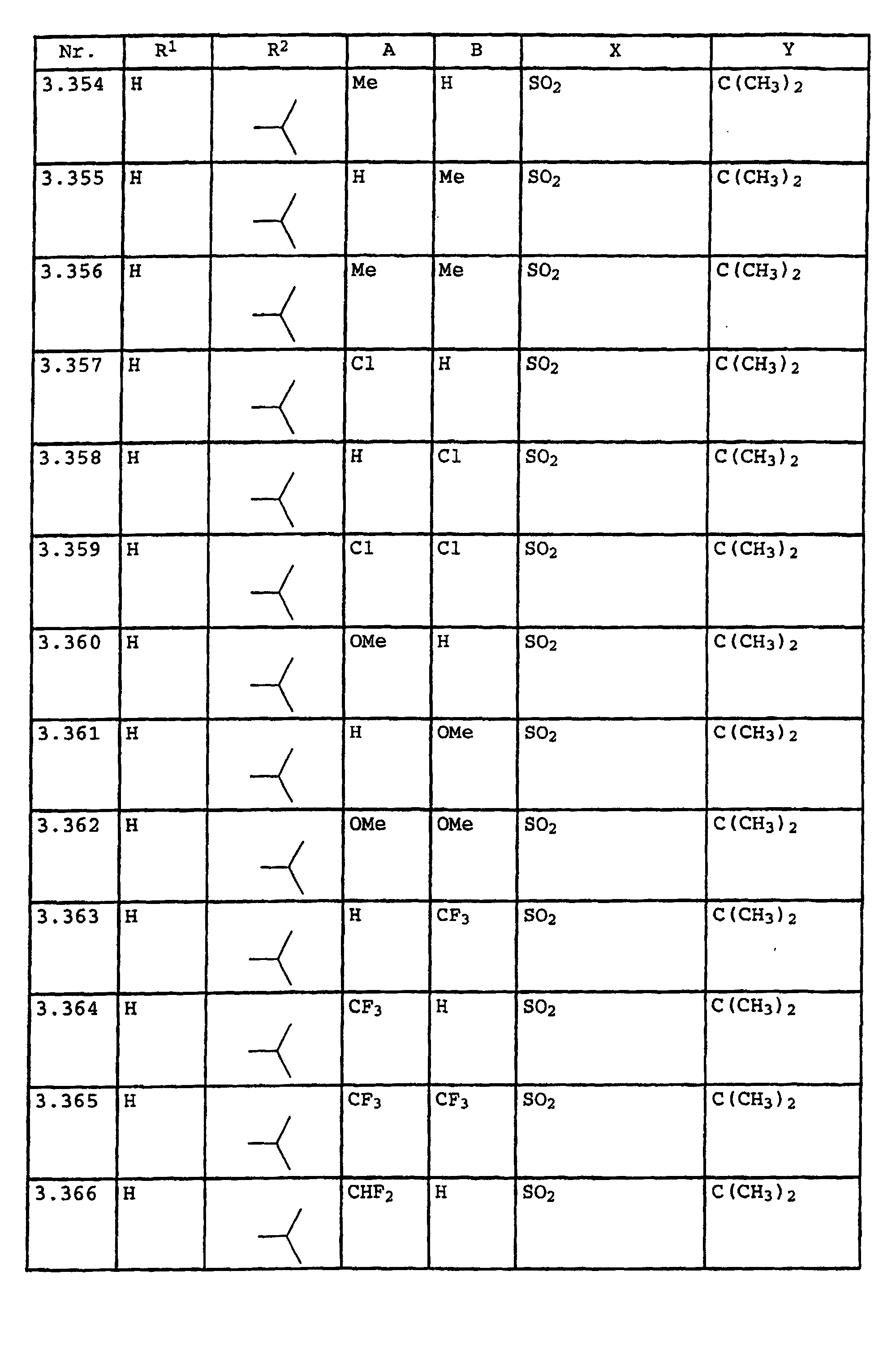

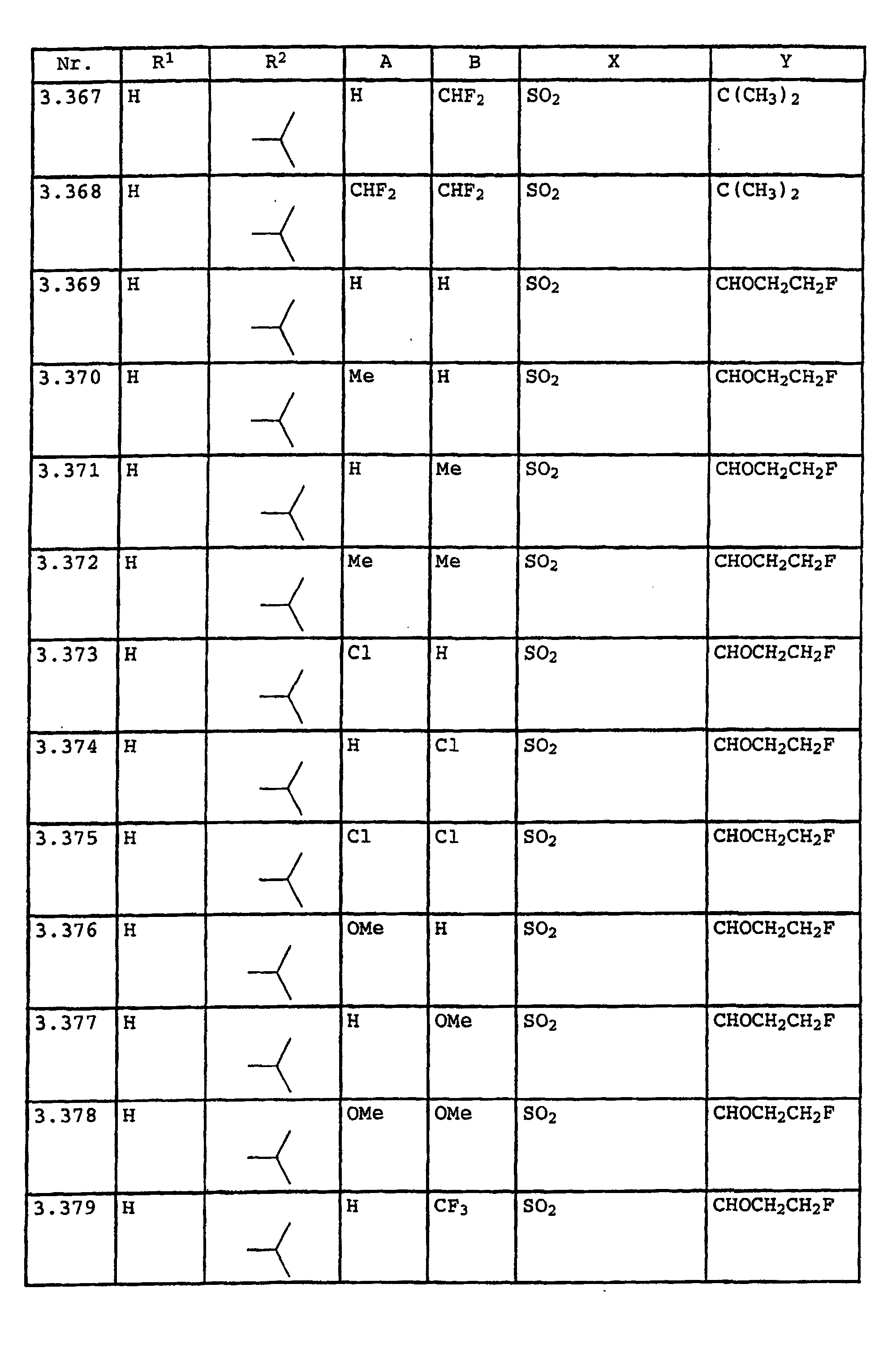

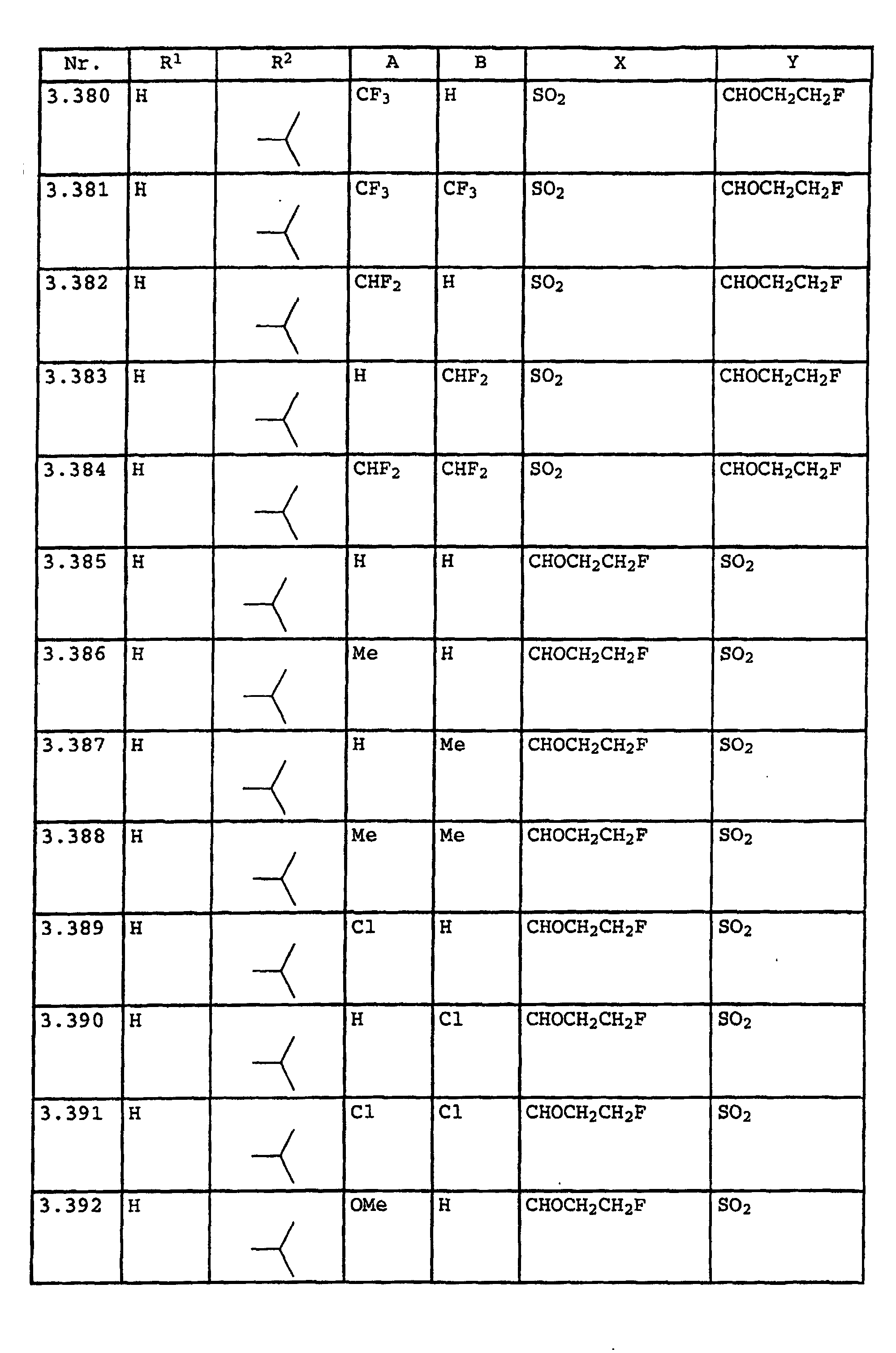

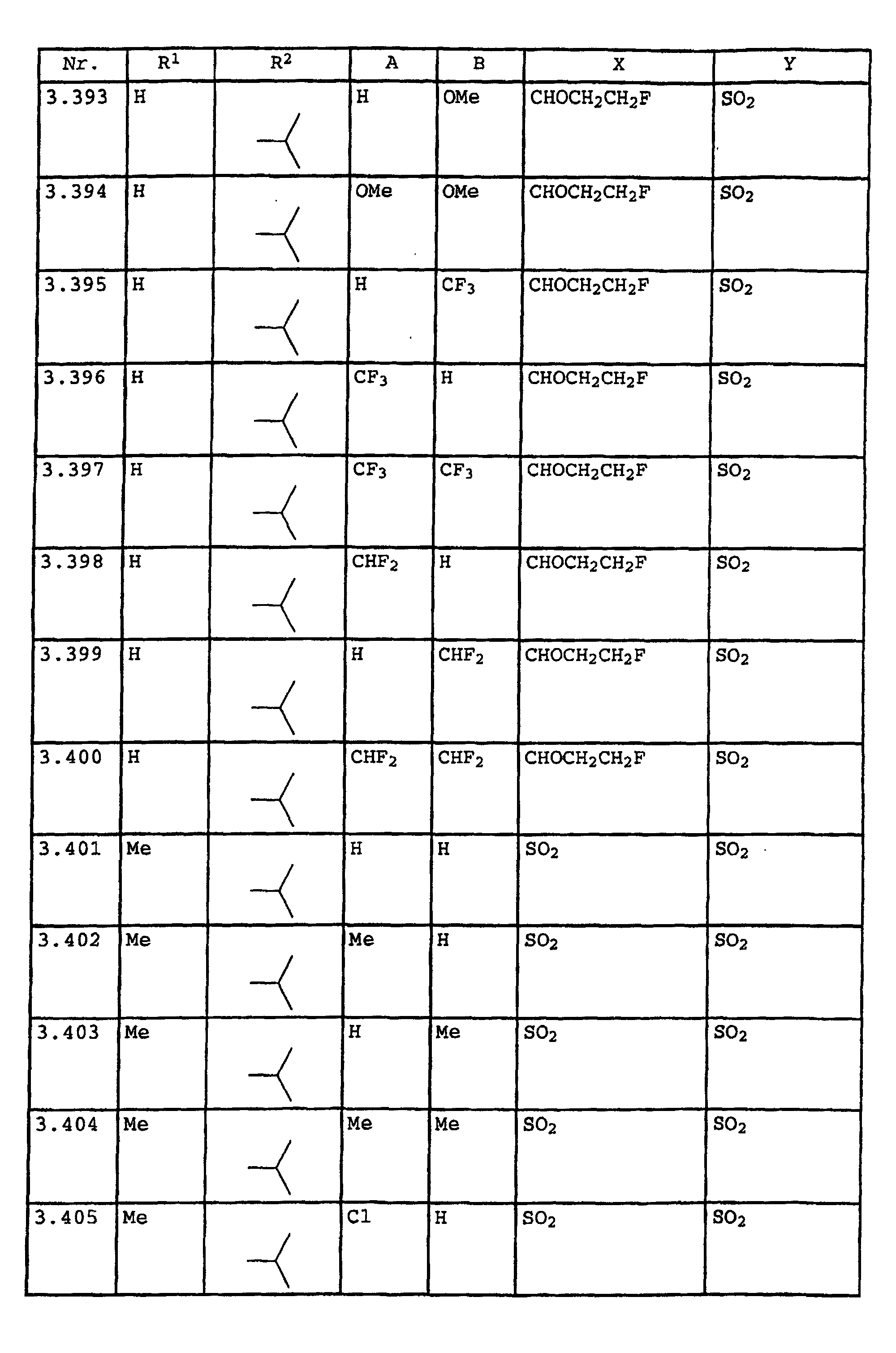

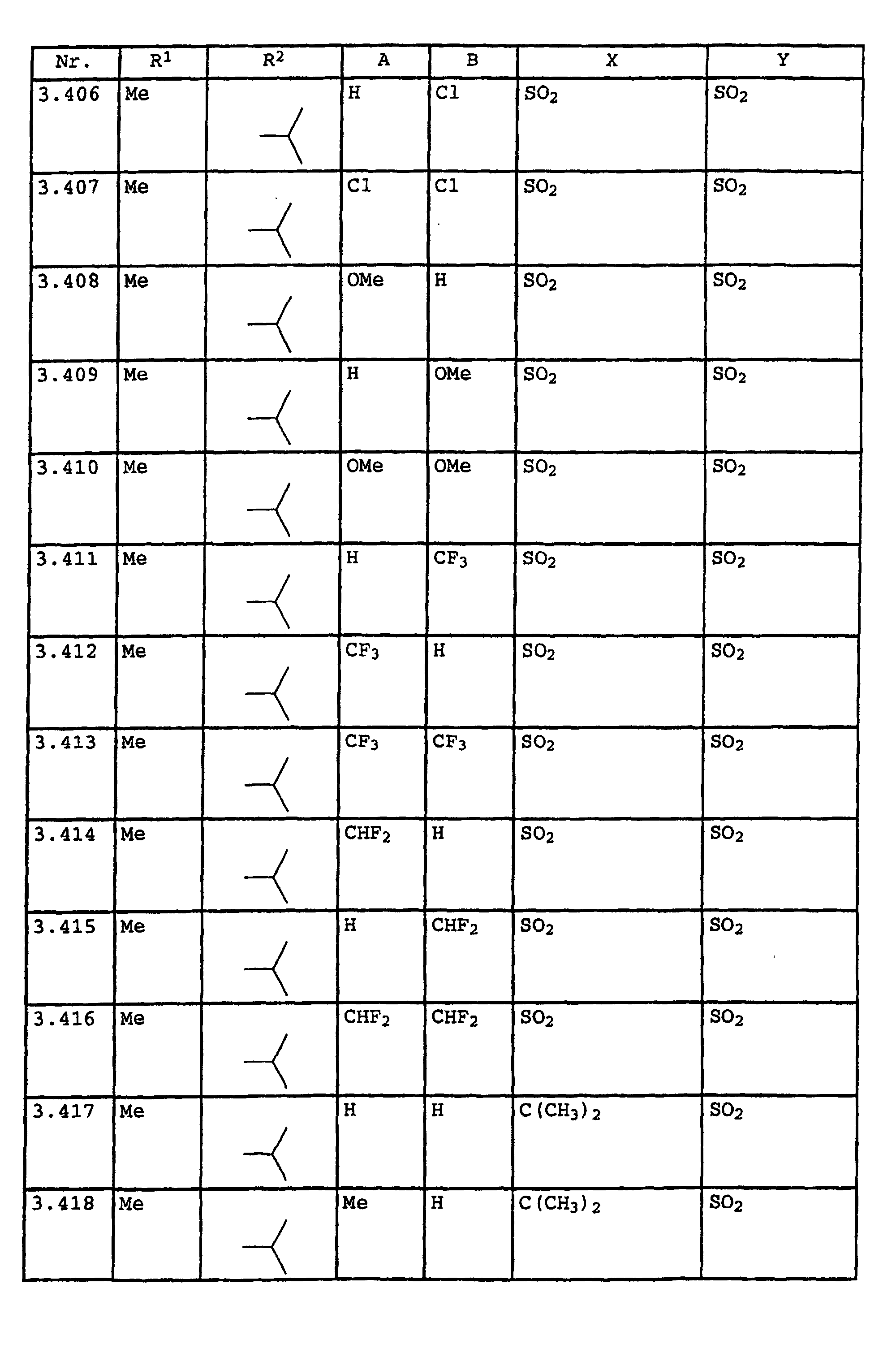

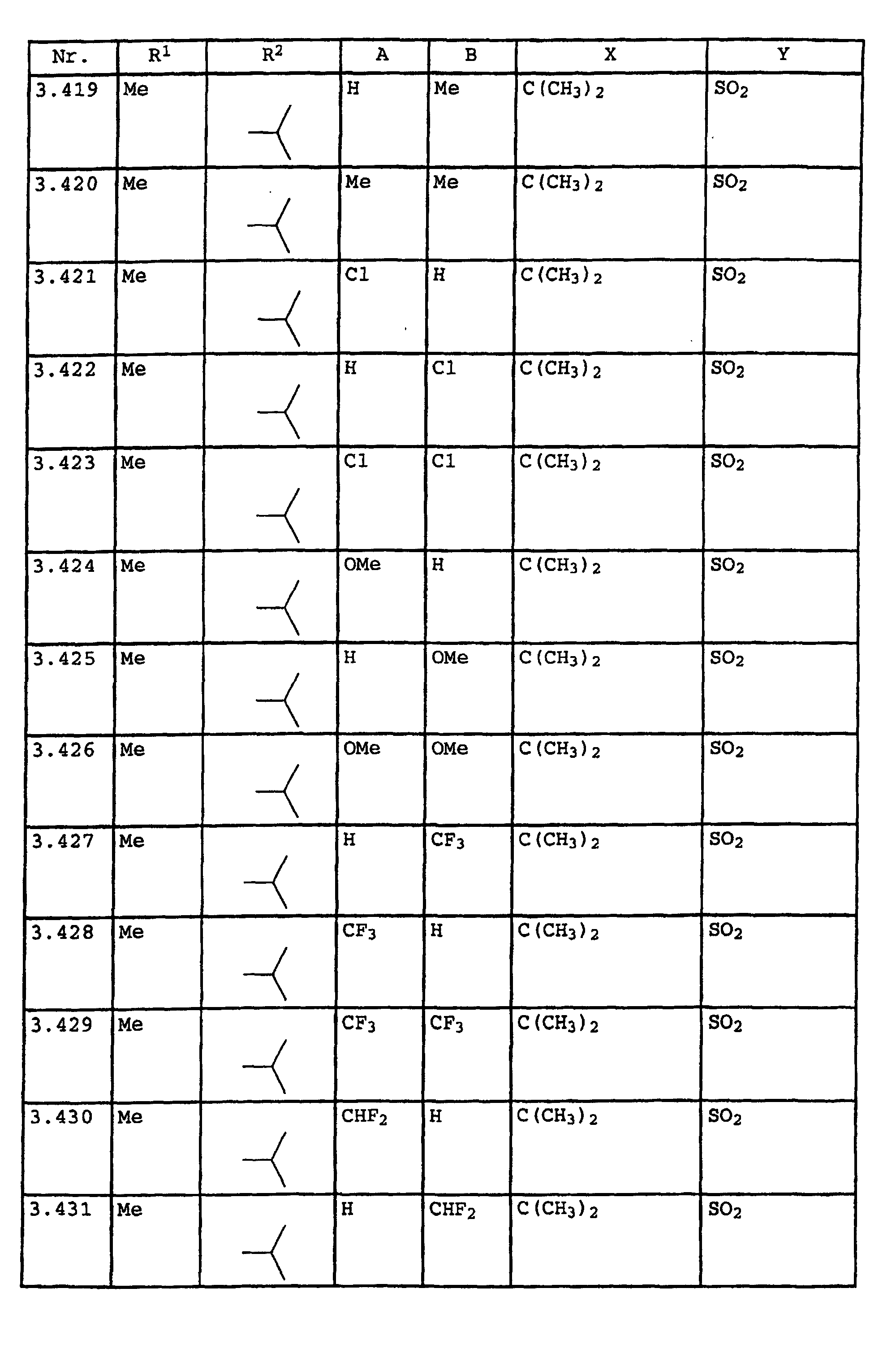

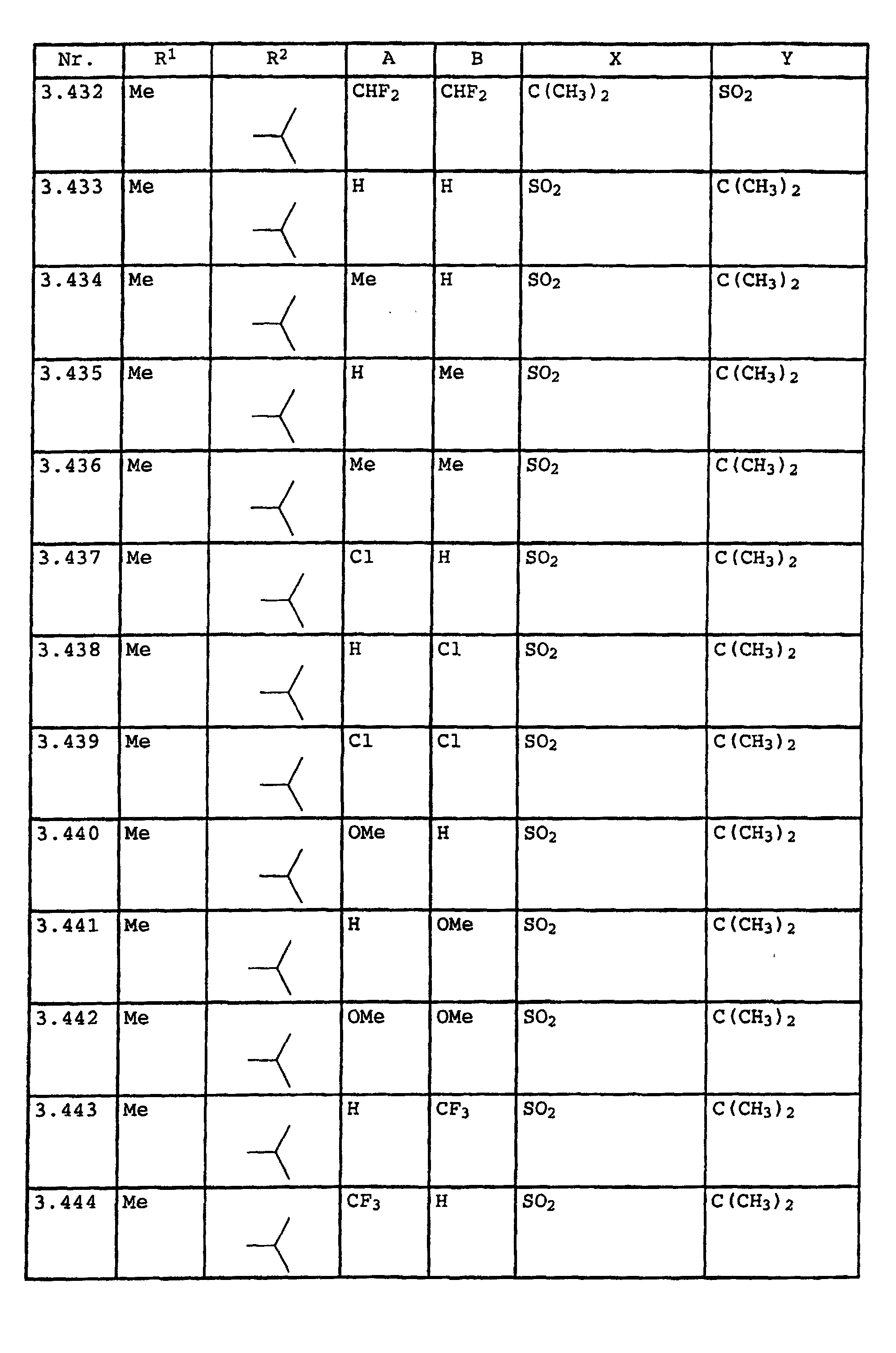

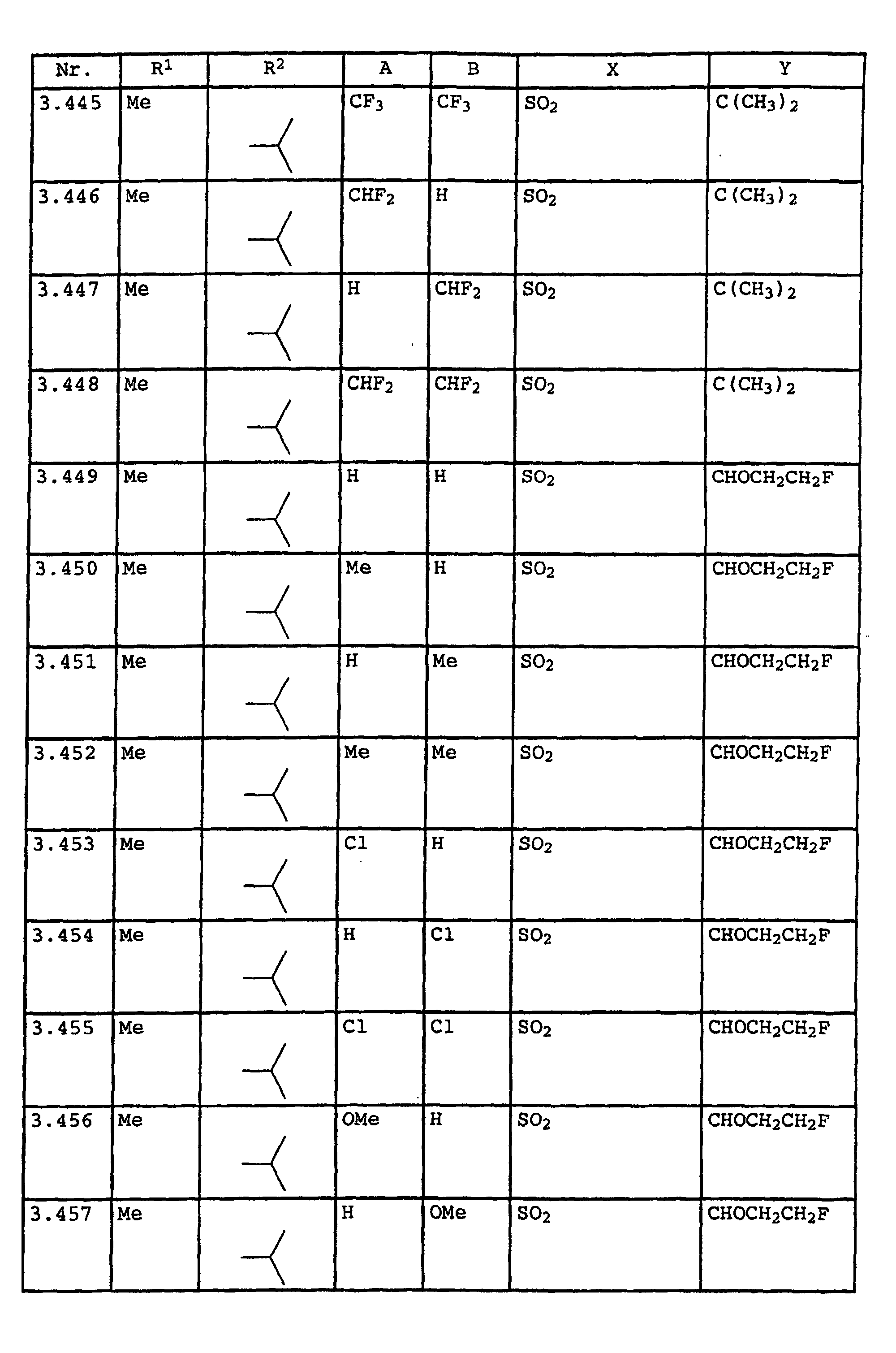

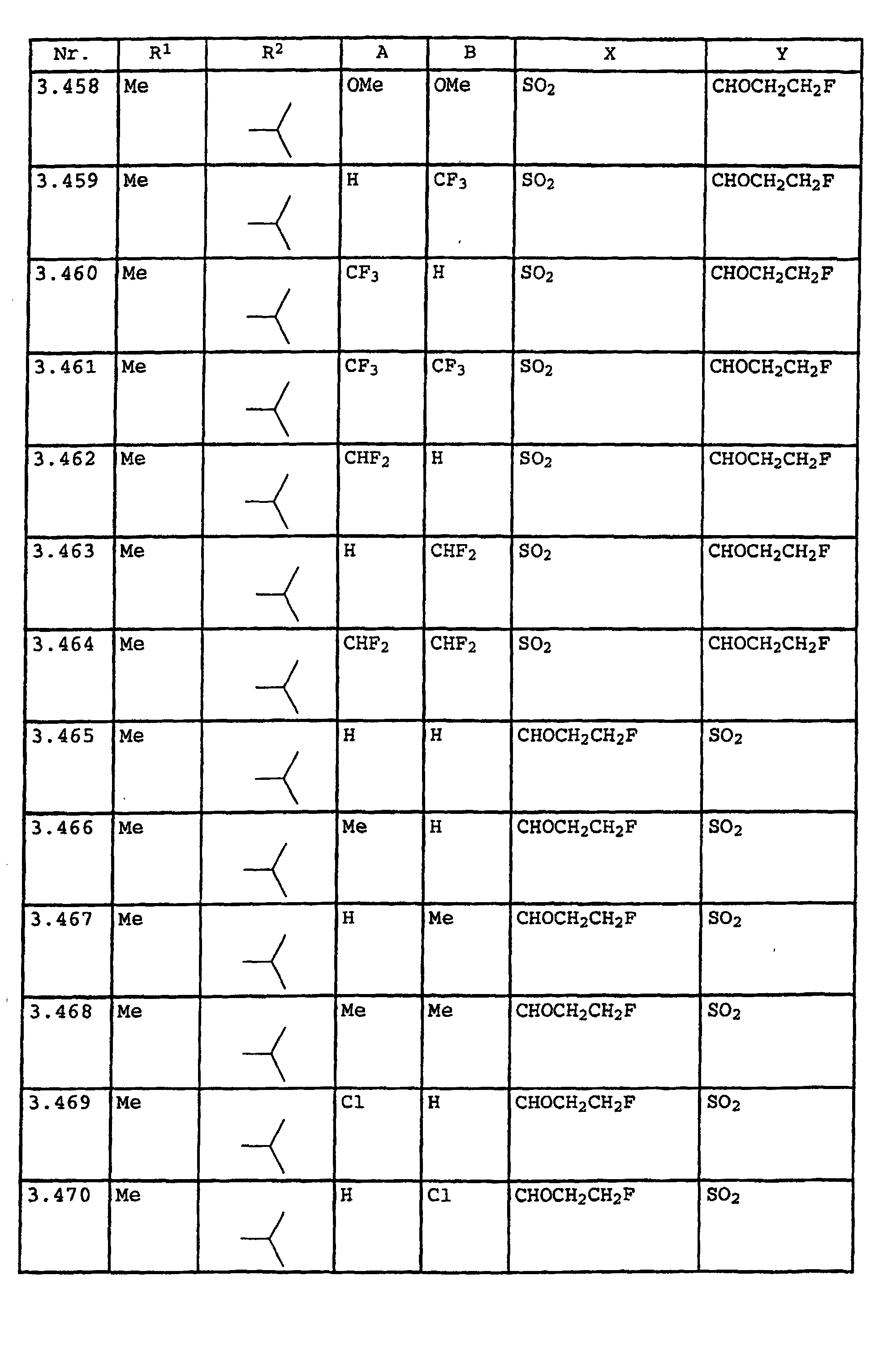

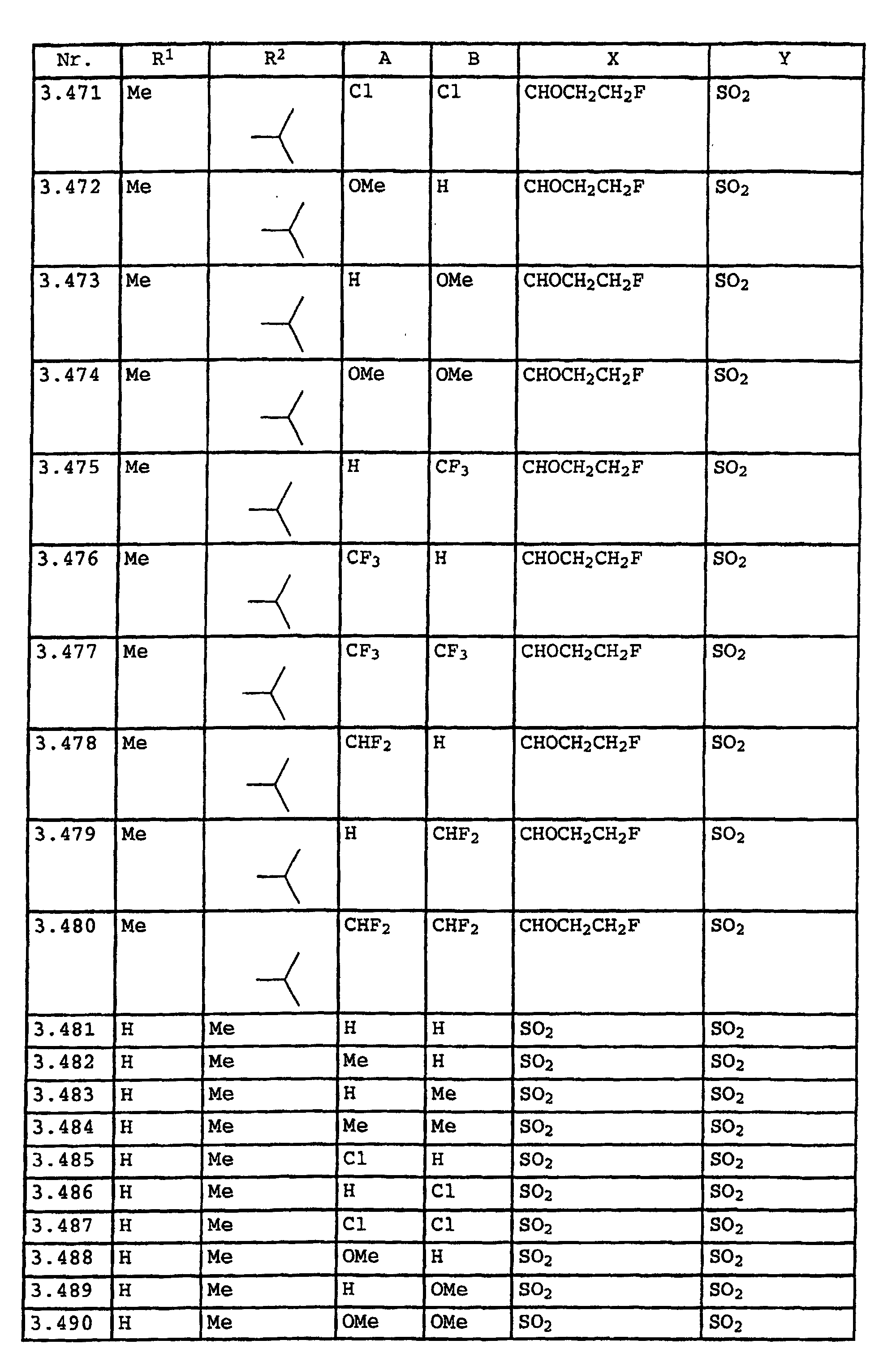

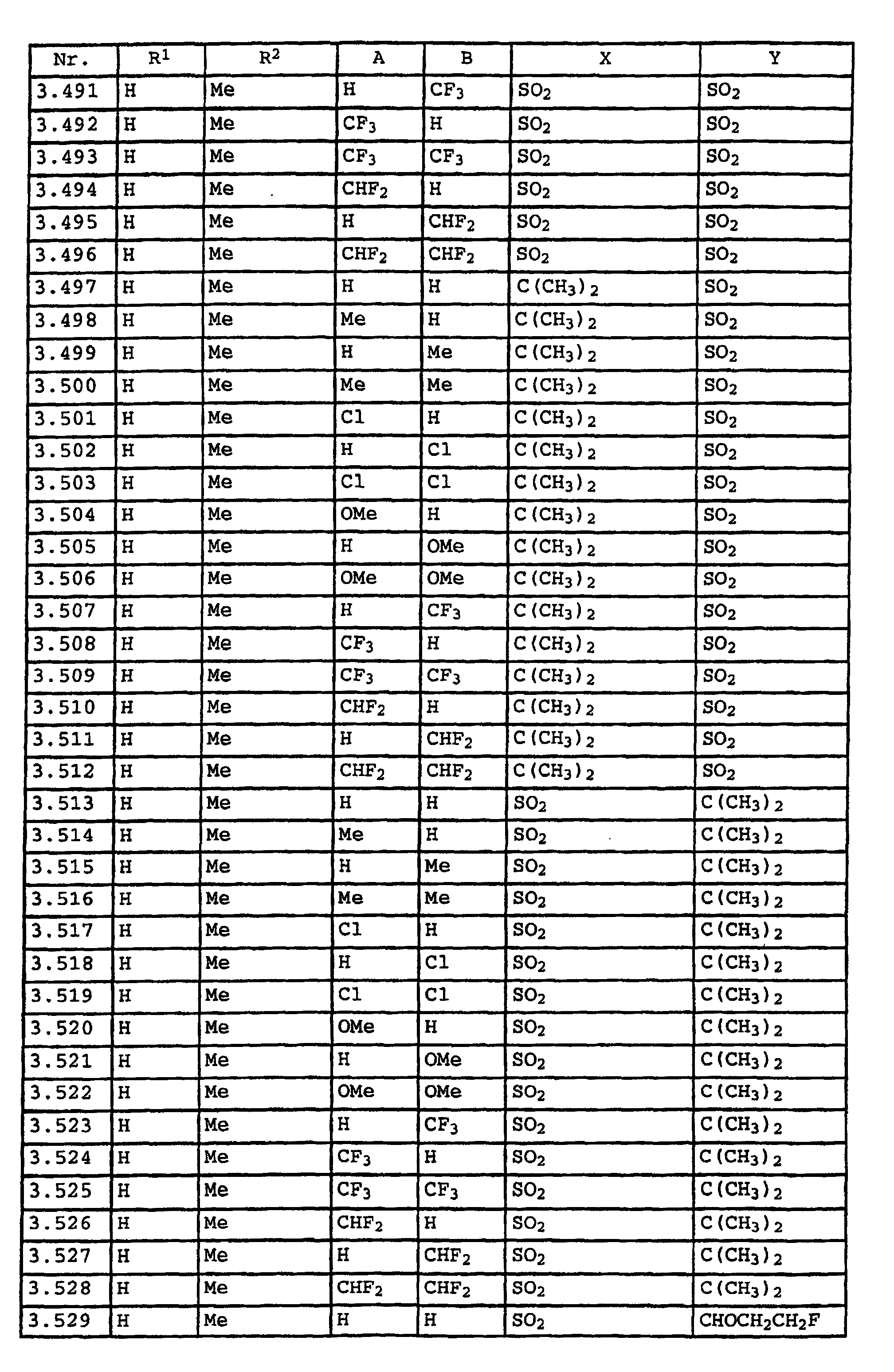

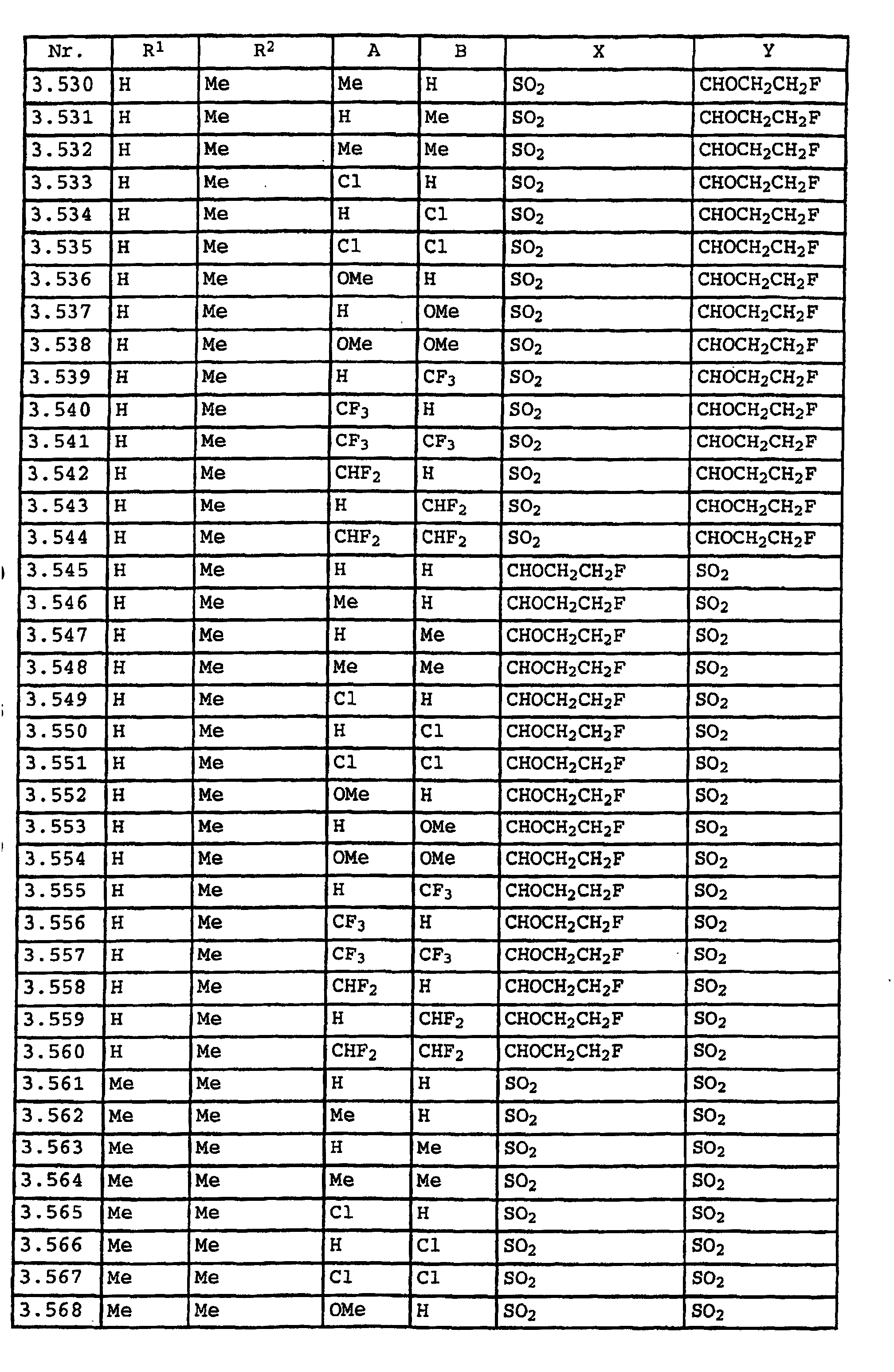

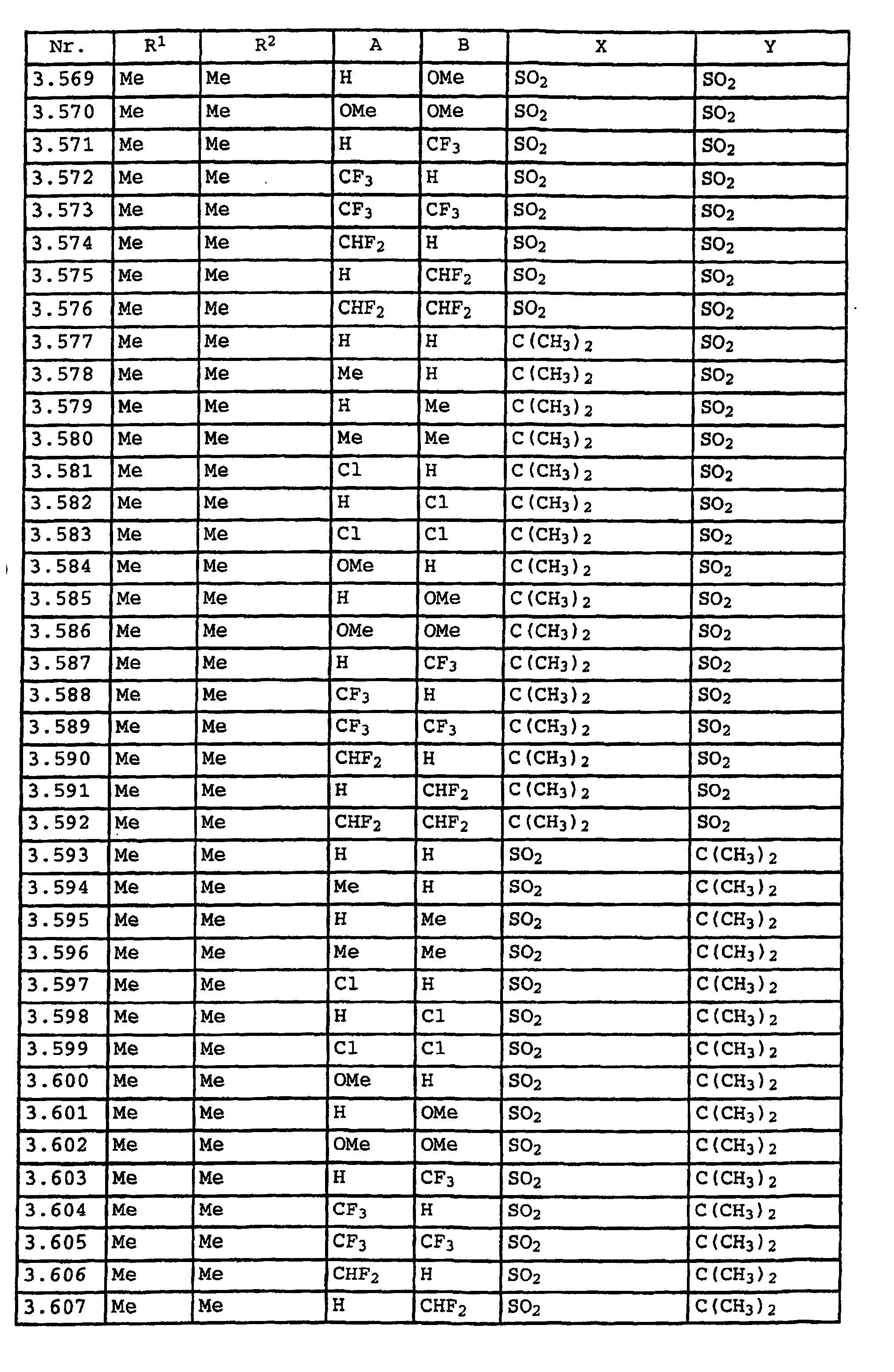

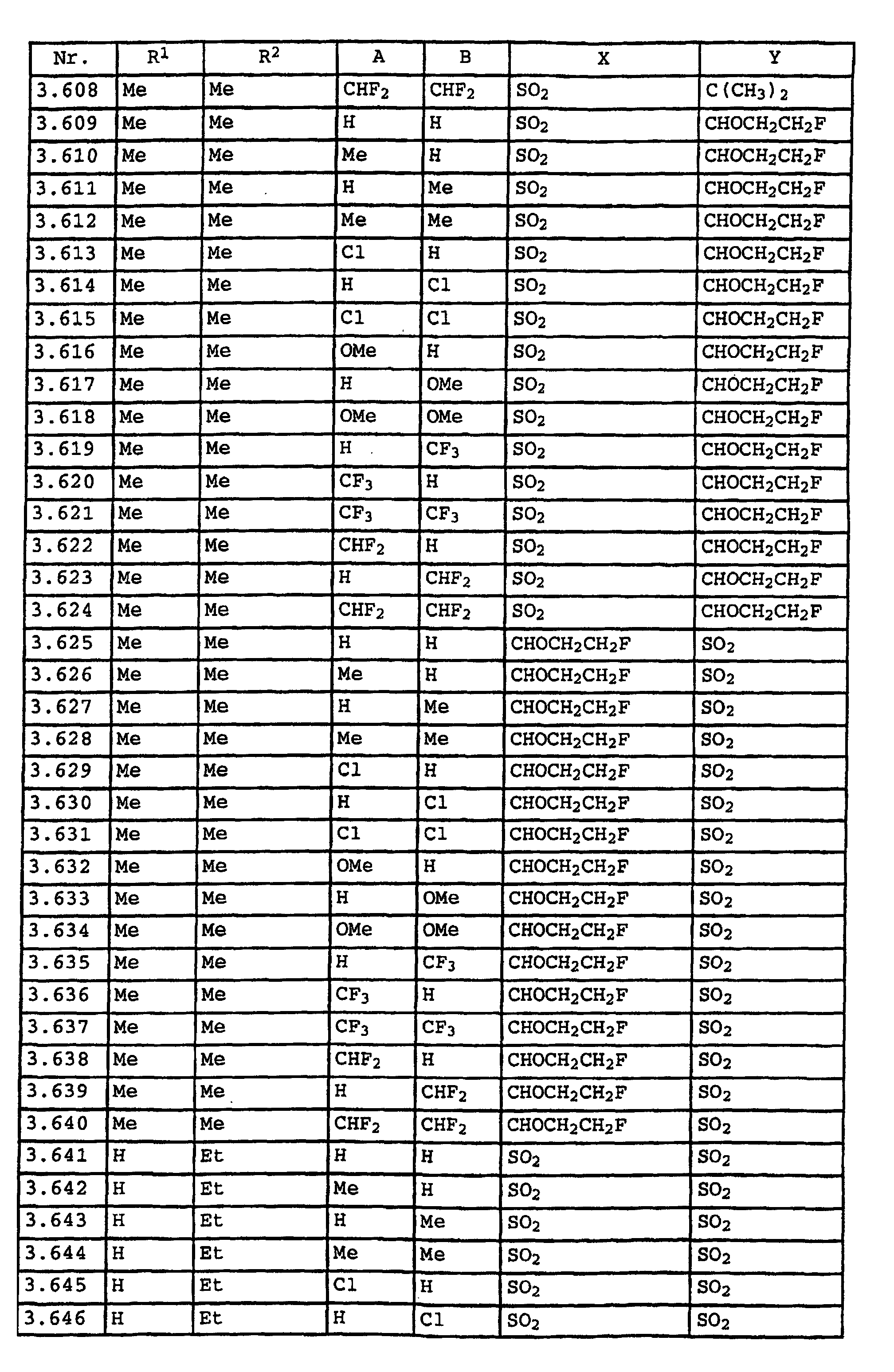

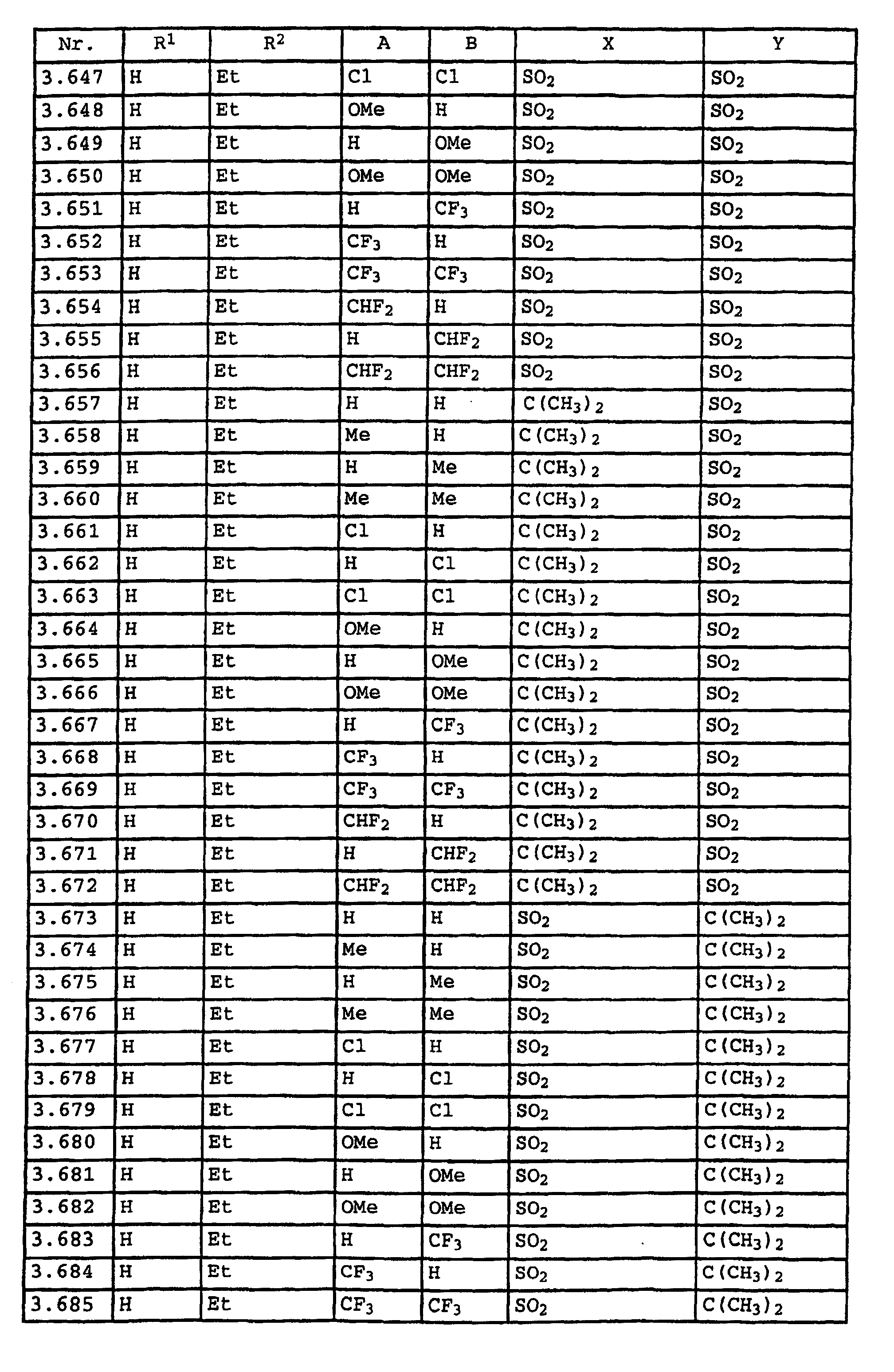

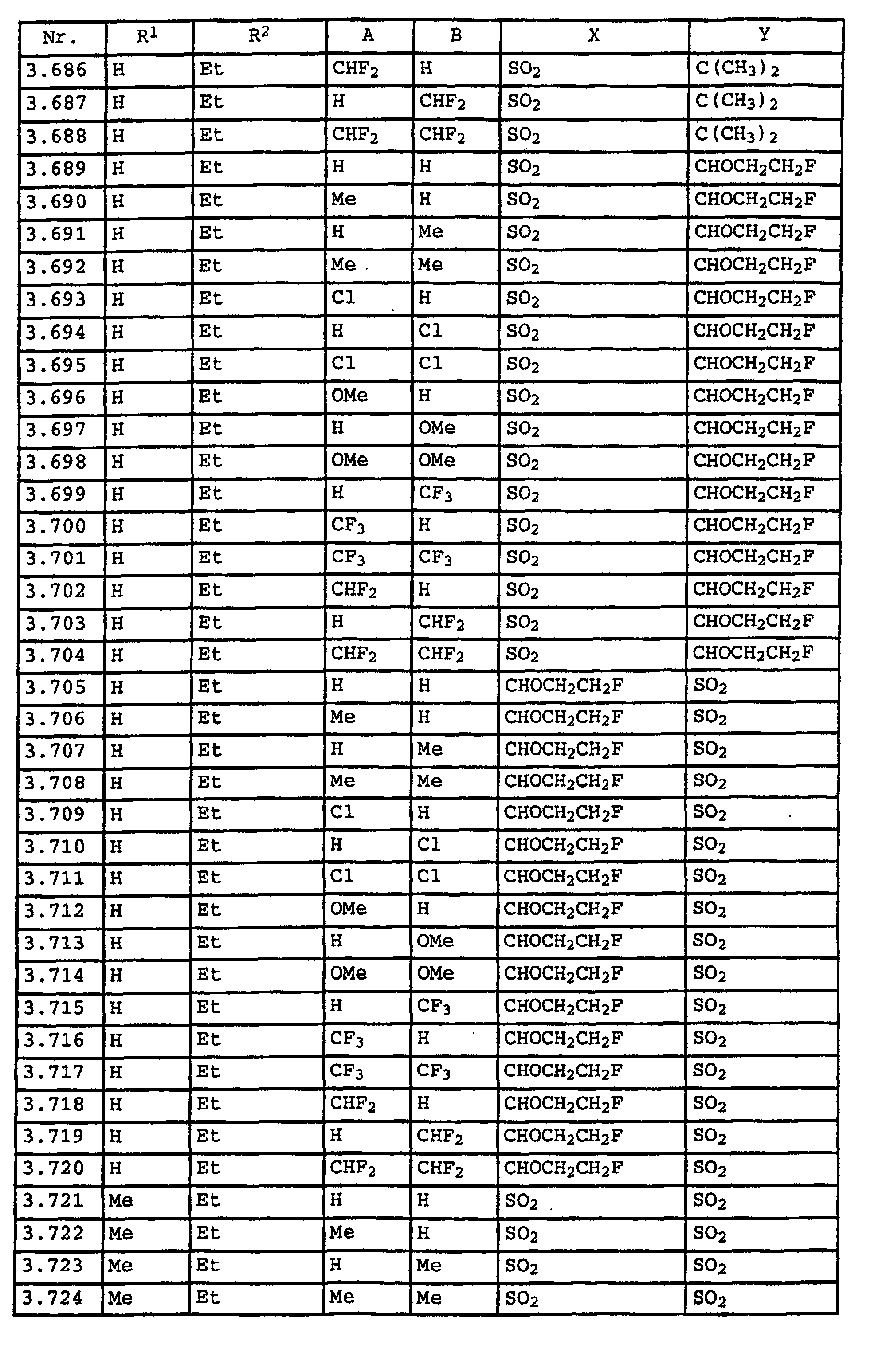

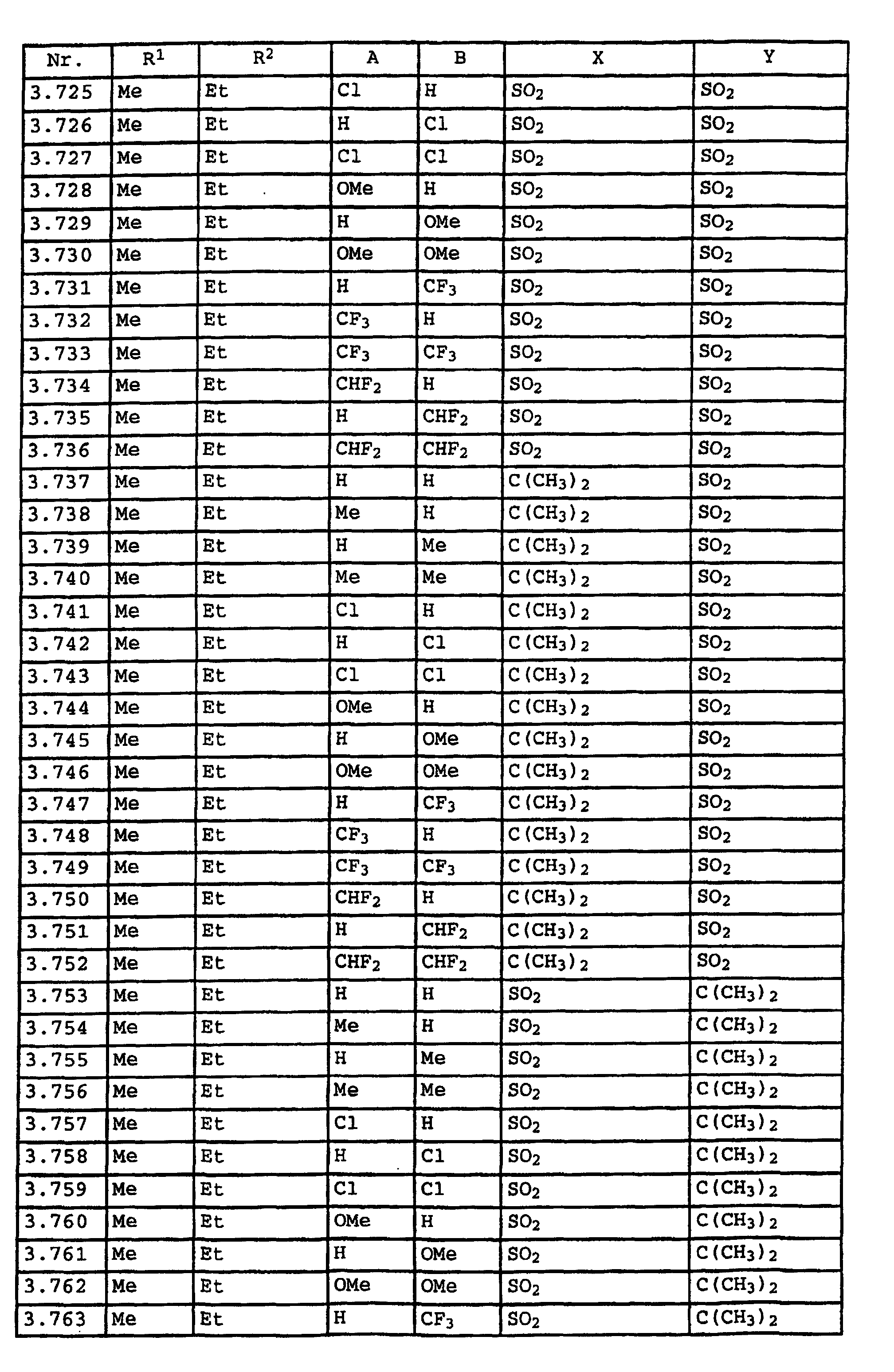

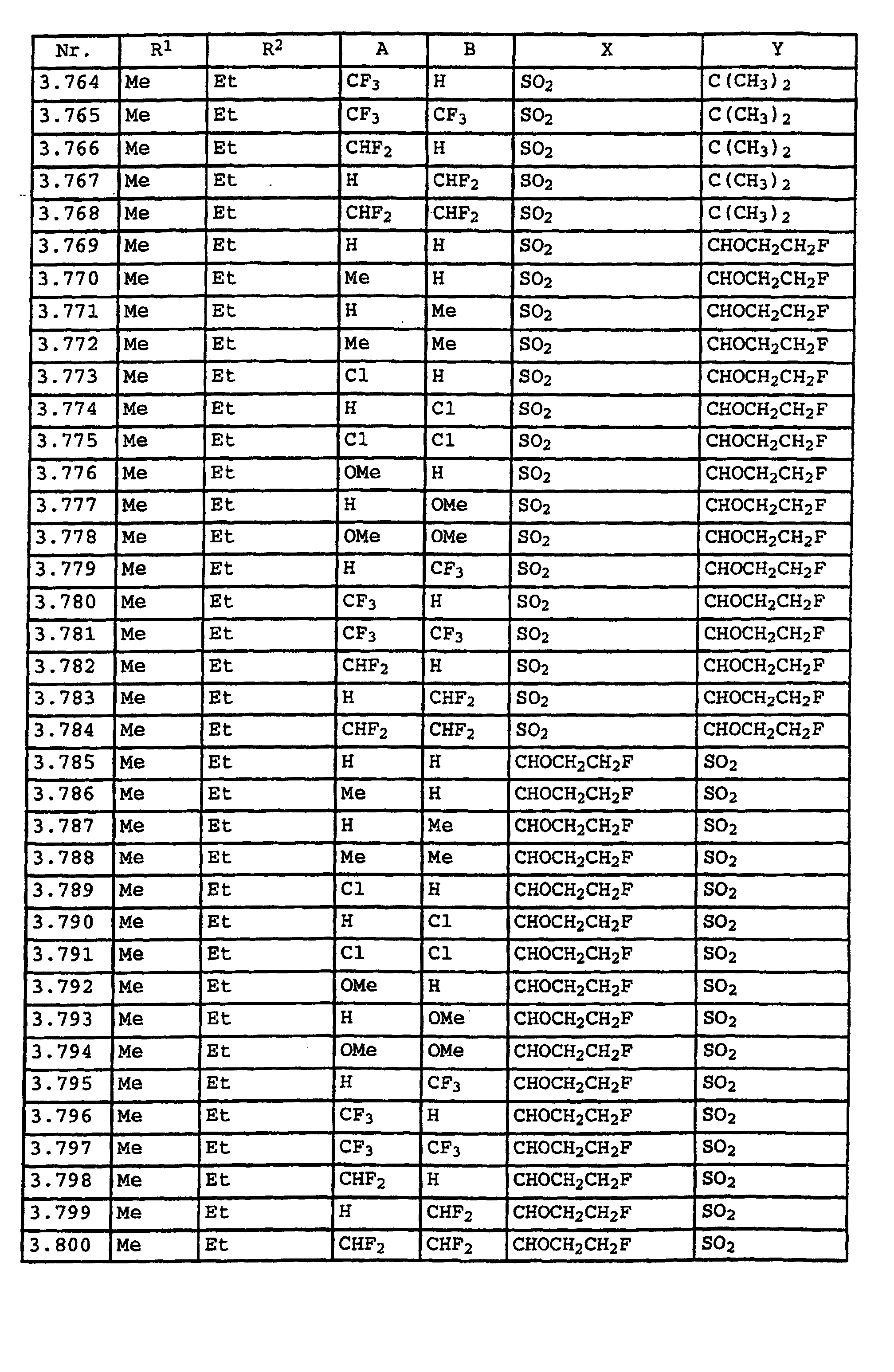

In den Tabellen 1 - 3 wurden für manche Reste Abkürzungen verwendet, die folgende Bedeutung haben:
Me = Methyl
Et = Ethyl

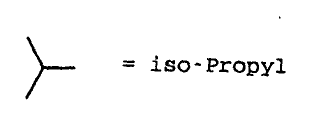

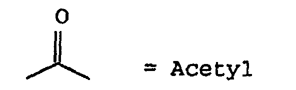

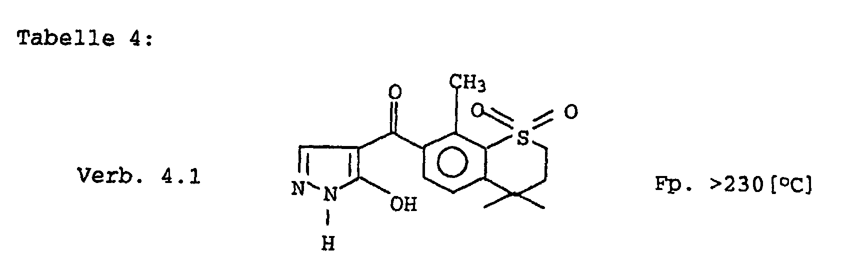

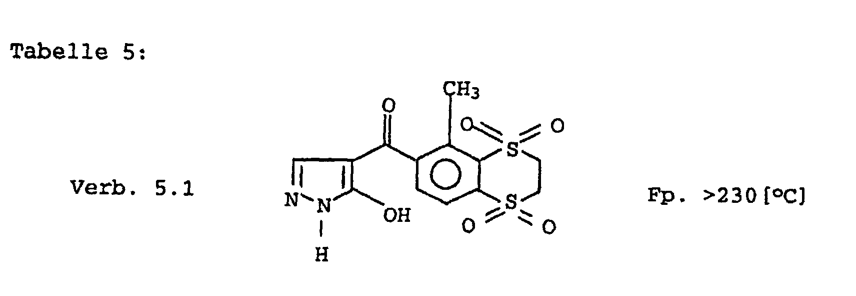

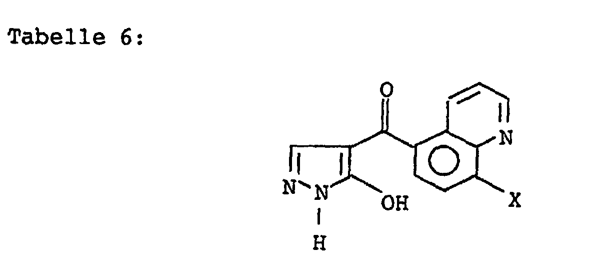

Verb. 6.1 X = Br
6.2 X = Cl
6.3 X = SO₂Me Fp. 138-140[°C]

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen I auch in Kulturen, die durch Züchtung einschließlich gentechnischer Methoden gegen die Wirkung von Herbiziden tolerant sind, verwandt werden.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten. Als inerte Zusatzstoffe kommen im Wesentlichen in Betracht: Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z. B. Amine wie N-Methylpyrrolidon oder Wasser. Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Pyrazoloylderivate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Sektrum) eingesetzt.
Die erfindungsgemäßen Verbindungen I können beispielsweise wie folgt formuliert werden:
- I: 20 Gewichtsteile der Verbindung Nr. 4.1 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew. % des Wirkstoffs enthält.
- II: 20 Gewichtsteile der Verbindung Nr. 5.1 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des Wirkstoffs enthält.
- III: 20 Gewichtsteile des Wirkstoffs Nr. 6.1 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des Wirkstoffs enthält.
- IV: 20 Gewichtsteile des Wirkstoffs Nr. 4.1 werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew. % des Wirkstoffs enthält.
- V: 3 Gewichtsteile des Wirkstoffs Nr. 5.1 werden mit 97 Gewichtsteilen feinteiligem Kaolin vermischt. Man erhält auf diese Weise ein Stäubemittel, das 3 Gew. % des Wirkstoffs enthält.
- VI: 20 Gewichtsteile des Wirkstoffs Nr. 6.1 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondesates und 68 Gewichtsteilen eines paraffinischen Mineralöls innig vermischt. Man erhält eine stabile ölige Dispersion.
- VII: 1 Gewichtsteil der Verbindung 4.1 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
- VIII: 1 Gewichtsteil der Verbindung 5.1 wird in einer Mischung gelöst, die aus 80 Gewichtsteilen Cyclohexanon und 20 Gewichtsteilen Wettol ® EM 31 (nicht ionischer Emulgator auf der Basis von ethoxyliertem Ricinusöl). Man erhält ein stabiles Emulsionskonzentrat.

Zur Verbreiterung des Wirkungsspektrums und zur Erzielung synergistischer Effekte können die Pyrazolylbenzoylderivate mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazinone, 2-Aroyl-1,3-cyclohexandione, Hetaryl-Aryl-Ketone, Benzylisoxazolidinone, Meta-CF3-phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen I allein oder in Kombination mit anderen herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha aktive Substanz (a. S.)

### Anwendungsbeispiele

Die herbizide Wirkung der Pyrazolylbenzoylderivate der Formel I ließ sich durch Gewächshausversuche zeigen:

Als Kulturgefäße dienten Plastiktöpfe mit lehmigem Sand mit etwa 3,0% Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde. Die Aufwandmenge für die Vorauflaufbehandlung betrug 0.250 und 0.125 kg/ha a. S.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt.

Die Aufwandmenge für die Nachauflaufbehandlung betrug 0.250 und 0.125 kg/ha a. S.

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| | | |
|---|---|---|
| Lateinischer Name | Deutscher Name | Englischer Name |
| Amaranthus retroflexus | Zurückgekrümmter Fuchsschwanz | redroot pigweed |
| Chenopodium album | Weißer Gänsefuß | lambsquarters (goosefoot) |
| Ecchinochloa crusgalli | Hühnerhirse | barnyardgras |
| Solanum nigrum | Schwarzer Nachtschatten | black nightshade |

Die Verbindungen 4.1, 5.1 und 6.1 zeigten eine ausgezeichnete herbizide Aktivität bei Nachauflaufanwendung im Gewächshaus bei Aufwandmengen von 0.250 und 0.125 kg/ha a.S.

## Patentansprüche

1. Verfahren zur Herstellung von Pyrazolylbenzoylderivaten der Formel I 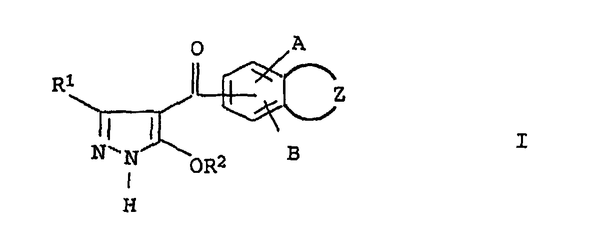 in der die Substituenten die folgende Bedeutung haben:
R¹ Wasserstoff, C₁-C₄-Alkyl oder C₁-C₄-Halogenalkyl;
R² Wasserstoff;
C₁-C₆-Alkyl, C₂-C₆-Alkenyl, C₂-C₆-Alkinyl, Benzyl, Benzoyl, C₁-C₄-Alkylcarbonyl, Hydroxycarbonyl-C₁-C₄-alkyl, C₁-C₄-Alkoxycarbonyl-C₁-C₄-alkyl, C₁-C₄-Alkylsulfonyl oder Phenylsulfonyl, wobei diese Gruppen durch C₁-C₄-Alkyl, C₁-C₄-Alkoxy, C₁-C₄-Alkylthio, Halogen, Hydroxy, Amino, Nitro oder Cyano substituiert sein können;
A,B unabhängig voneinander Wasserstoff;
C₁-C₆-Alkyl, C₂-C₆-Alkenyl, C₂-C₆-Alkinyl, C₁-C₄-Alkoxy, wobei diese Gruppen durch Halogen, Hydroxy, C₁-C₄-Alkoxy oder Cyano substituiert sein können;
Halogen, Hydroxy, Cyano, Nitro, eine Gruppe -(Y)ₙ-S(O)ₘR³ oder eine Gruppe -(Y)ₙ-CO-R^{4;}
Z ein 5- oder 6-gliedriger heterocyclischer, gesättigter oder ungesättigter ankondensierter Ring, enthaltend ein bis drei Heteroatome, ausgewählt aus der Gruppe Sauerstoff, Schwefel oder Stickstoff, der gegebenenfalls durch Halogen, Cyano, Nitro, eine Gruppe
-CO-R⁴, C₁-C₄-Alkyl, C₁-C₄-Halogenalkyl, C₃-C₈-Cycloalkyl, C₁-C₄-Alkoxy, C₁-C₄-Halogenalkoxy, C₁-C₄-Alkylthio, C₁-C₄-Halogenalkylthio, Di-C₁-C₄-Alkylamino, gegebenenfalls durch Halogen, Cyano, Nitro, C₁-C₄-Alkyl oder C₁-C₄-Halogenalkyl substituiertes Phenyl oder eine Oxogruppe, die gegebenenfalls auch in der tautomeren Form als Hydroxygruppe vorliegen kann, substituiert ist oder der mit einem ankondensierten, gegebenenfalls durch Halogen, Cyano, Nitro, C₁-C₄-Alkyl oder C₁-C₄-Halogenalkyl substituierten Phenylring, einem ankondensierten Carbocyclus oder
einem ankondensierten, gegebenenfalls durch Halogen, Cyano, Nitro, C₁-C₄-Alkyl, Di-C₁-C₄-Alkylamino, C₁-C₄-Alkoxy, C₁-C₄-Halogenalkoxy, oder C₁-C₄-Halogenalkyl substituierten zweiten Heterocyclus ein tricyclisches System bildet;
Y O, NR⁵;
n null oder eins;
m null, eins oder zwei;
R³ C₁-C₄-Alkyl, C₁-C₄-Halogenalkyl oder NR⁵R^{6;}
R⁴ C₁-C₄-Alkyl, C₁-C₄-Halogenalkyl, C₁-C₄-Alkoxy, oder NR⁵R^{6;}
R⁵ Wasserstoff oder C₁-C₄-Alkyl;
R⁶ C₁-C₄-Alkyl;
**dadurch gekennzeichnet, daß** man ein Pyrazolyl-benzoylderivat der Formel II 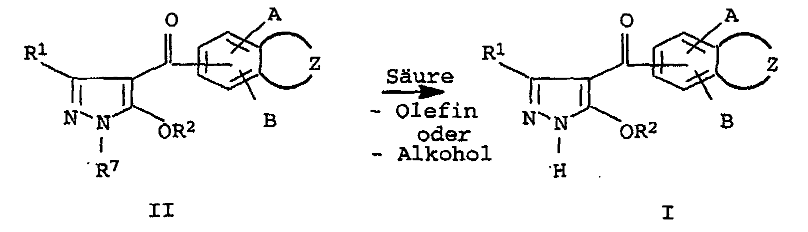 in der die Substituenten R¹, R², A, B und Z die oben angegebene Bedeutung haben und
R⁷ in α-Stellung verzweigtes C₃-C₁₂-Alkyl, C₃-C₁₂-Alkenyl, C₄-C₁₂-Alkinyl, die gegebenenfalls durch Halogen oder C₁-C₄-Alkoxy substituiert sind oder Benzyl, das gegebenenfalls durch Halogen, Cyano, Nitro, C₁-C₄-Halogenalkyl, C₁-C₄-Alkylsulfonyl oder C₁-C₄-Alkylcarbonyl substituiert ist, bedeutet,
mit einer anorganischen oder organischen Säure bei einem pH-Wert < 2 behandelt und unter Abspaltung eines Olefins oder eines Alkohols umsetzt.

2. Verfahren nach Anspruch 1, **dadurch gekennzeichnet, daß** man das Pyrazolylbenzoylderivat der Formel II mit R² = Wasserstoff durch Umsetzung der Pyrazole der Formel III 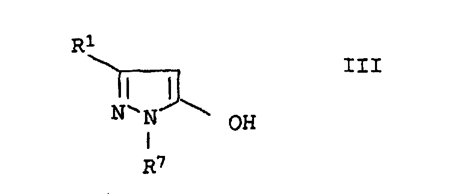 in der die Substituenten R¹ und R⁷ die in Anspruch 1 angegebene Bedeutung haben, mit einem Benzoylderivat der Formel IV 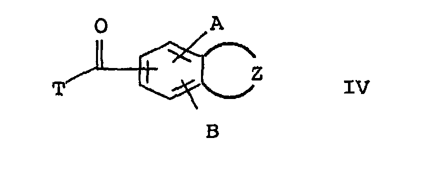 in der T = Halogen bedeutet und A, B und Z die in Anspruch 1 genannte Bedeutung haben, und anschließender Umlagerung des Acylierungsproduktes V 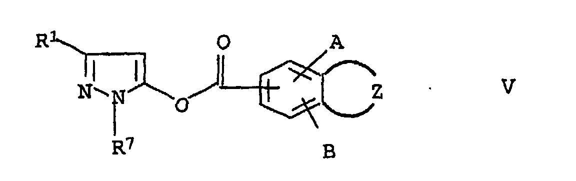 in Gegenwart eines Katalysators herstellt.

3. Verfahren nach Anspruch 1 oder 2, **dadurch gekennzeichnet, daß** der Substituent R⁷in α-Stellung verzweigtes C₃-C₆-Alkyl bedeutet.

4. Verfahren nach Anspruch 1 oder 2, **dadurch gekennzeichnet, daß** der Substituent R⁷ Benzyl, 4-Chlorbenzyl, 4-Cyanobenzyl, 4-Nitrobenzyl, 4-Trifluormethylbenzyl, 4-Methylsulfonylbenzyl oder 4-Acylbenzyl bedeutet.

5. Verfahren nach Anspruch 4, **dadurch gekennzeichnet, daß** der Substituent R⁷ Benzyl, 4-Chlorbenzyl oder 4-Nitrobenzyl bedeutet.

6. Verfahren nach Anspruch 1, **dadurch gekennzeichnet, daß** man als organische Säure Trifluormethansulfonsäure oder Trichloressigsäure verwendet.

7. Verfahren nach Anspruch 1, **dadurch gekennzeichnet, daß** man als anorganische Säure, Schwefelsäure, Salpetersäure, Salzsäure oder Bromwasserstoffsäure verwendet.

8. Verfahren nach Anspruch 1, **dadurch gekennzeichnet, daß** man die Umsetzung in einem Lösungsmittel durchführt.

9. Verfahren nach Anspruch 8, **dadurch gekennzeichnet, daß** man als Lösungsmittel Acetonitril, DMF, Dioxan, Tetrahydrofuran, Toluol oder Chlorbenzol verwendet.

10. Pyrazol-4-yl-benzoylderivate der Formel I 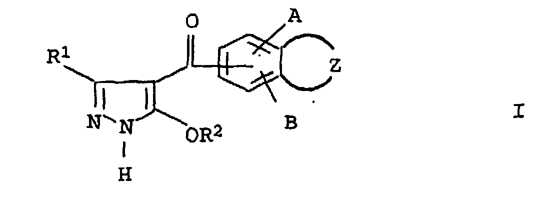 in der die Substituenten R¹, R², A, B und Z die in Anspruch 1 angegebene Bedeutung haben.

11. Pyrazol-4-yl-benzoylderivate der Formel I nach Anspruch 10, in der der Substituent Z ein 5-oder 6-gliedriger heterocyclischer, gesättigter oder ungesättigter ankondensierter Ring ist, so daß mit dem ankondensierten Phenylring die folgenden Bicyclischen Heterocyclen resultieren:
Benzofuranyl, Isobenzofuranyl, Benzothienyl, Isobenzothienyl, Thionaphthalenyl, Isothionaphthalenyl, Indolyl, Isoindolyl, Indazolyl, Indoleninyl, Isobenzazolyl, Pyranopyrrolyl, Isoindazolyl, Indoxazinyl, Benzoxazolyl, Benzothiazolyl, Benzothiazolyl-S,S-dioxid, Benzopyranyl, Chinolinyl, Isochinolinyl, Phthalazinyl, Chinoxalinyl, Chinazolinyl, Naphthyridinyl, Pyridopyridinyl, Pyranyl, Thiopyranyl, Chromanyl, Thiachromanyl, Chromenyl, Thiachromenyl, Benzoxazinyl und Benzisoxazinyl.

12. Pyrazol-4-yl-benzoylderivate der Formel VI gemäß Anspruch 10 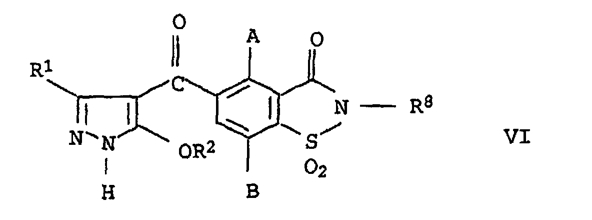 in der die Substituenten R¹, R², A und B die in Anspruch 10 angegebene Bedeutungen haben und R⁸ Wasserstoff, C₁-C₄-Alkyl, C₁-C₄-Alkoxy oder C₁-C₄-Halogenalkyl bedeutet.

13. Pyrazol-4-yl-benzoylderivate der Formel VII gemäß Anspruch 10, 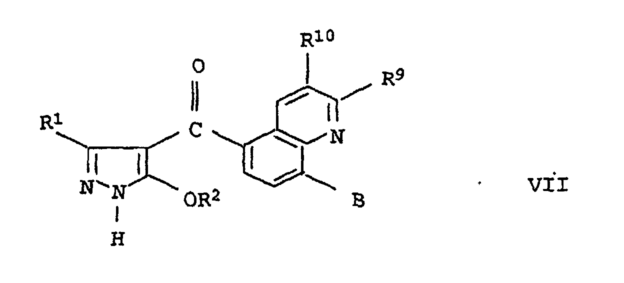 in der die Substituenten R¹, R² und B die in Anspruch 10 angegebene Bedeutung haben und R⁹, R¹⁰ unabhängig voneinander *Was*serstoff, C₁-C₄-Alkyl, C₁-C₄-Alkoxy oder C₁-C₄-Halogenalkyl bedeutet.

14. Pyrazol-4-yl-benzoylderivate der Formel VIII gemäß Anspruch 10 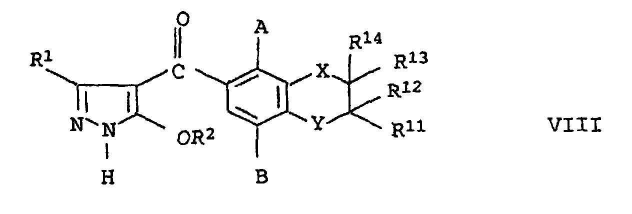 in der die Substituenten R¹, R² A und B die vorstehend genannte Bedeutung haben und
R¹¹, R¹², R¹³, R¹⁴ unabhängig voneinander Wasserstoff,
C₁-C₄-Alkyl, C₁-C₄-Alkoxy oder C₁-C₄ Halogenalkyl bedeutet;
R¹¹, R¹³ oder R¹¹, R¹⁴ oder R¹², R¹³ oder R¹², R¹⁴ können eine Bindung bilden;
X, Y unabhängig voneinander S(O)ₙ, C=O, CR¹⁵R¹⁶, NR¹⁷;
R¹⁵, R¹⁶ unabhängig voneinander Wasserstoff, C₁-C₄-Alkyl, C₁-C₄-Alkoxy, C₁-C₄-Halogenalkyl oder C₁-C₄-Halogenalkoxy;
R¹⁵, R¹⁶ können zusammen einen 5-6gliedrigen Carbocyclus bilden, der 2 Sauerstoffatome oder Schwefelatome enthalten kann;
R¹⁷ Wasserstoff oder C₁-C₄-Alkyl;
n 0, 1 oder 2
bedeutet.

15. Pyrazol-4-yl-benzoylderivate der Formel I nach einem der Ansprüche 10 bis 14, in der die Reste R¹ und R² unahängig voneinander für Wasserstoff oder C₁-C₄-Alkyl, A und B unabhängig voneinander für Wasserstoff, C₁-C₄-Alkyl, C₁-C₄-Alkoxy, C₁-C₄-Alkylthio, C₁-C₄-Alkylsulfonyl, C₁-C₄-Halogenalkyl, Halogen, Hydroxy, Cyano oder Nitro stehen.

16. Pyrazol-4-yl-benzoylderivate der Formel I nach Anspruch 14, in der die Reste A und B unabhängig voneinander für Wasserstoff, Methyl, Methoxy, Methylthio, Methylsulfonyl, Trifluormethyl, Chlor, Cyano oder Nitro stehen.

17. Pyrazol-4-yl-benzoylderivate der Formel I nach Anspruch 14, in der die Reste R¹, R² für Wasserstoff und A und B unabhängig voneinander für Wasserstoff, Methyl, Methoxy, Methylthio, Methylsulfonyl oder Chlor stehen.

18. Pyrazol-4-yl-benzoylderivate der Formel I nach Anspruch 14, in der die Reste R¹, R² für Wasserstoff, A für Methyl oder Chlor und B für Methylthio oder Methylsulfonyl stehen.

19. Herbizides Mittel, enthaltend mindestens ein Pyrazol-4-ylbenzoylderivat der Formel I gemäß Anspruch 10 und inerte Zusatzstoffe.

20. Verfahren zur Bekämpfung unerwünschten Pflanzenwuchses, **dadurch gekennzeichnet, daß** man eine herbizid wirksame Menge eines Pyrazol-4-yl-benzoylderivates der Formel I gemäß Anspruch 10 auf die Pflanzen oder deren Lebensraum einwirken läßt.

## Claims

1. A process for preparing pyrazolylbenzoyl derivatives of the formula I 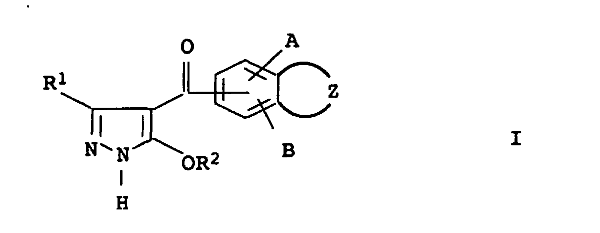 where:
R¹ is hydrogen, C₁-C₄-alkyl or C₁-C₄-haloalkyl;
R² is hydrogen;
C₁-C₆-alkyl, C₂-C₆-alkenyl, C₂-C₆-alkynyl, benzyl, benzoyl, C₁-C₄-alkylcarbonyl, hydroxycarbonyl-C₁-C₄-alkyl, C₁-C₄-alkoxycarbonyl-C₁-C₄-alkyl,
C₁-C₄-alkylsulfonyl or phenylsulfonyl, where these groups can be substituted by C₁-C₄-alkyl,
C₁-C₄-alkoxy, C₁-C₄-alkylthio, halogen, hydroxyl, amino, nitro or cyano;
A,B independently of one another are hydrogen;
C₁-C₆-alkyl, C₂-C₆-alkenyl, C₂-C₆-alkynyl,
C₁-C₄-alkoxy, where these groups can be substituted by halogen, hydroxyl, C₁-C₄-alkoxy or cyano;
halogen, hydroxyl, cyano, nitro, a group -(Y)ₙ-S(O)ₘR³ or a group -(Y)ₙ-CO-R⁴;
Z is a 5- or 6-membered heterocyclic, saturated or unsaturated fused-on ring containing one to three heteroatoms selected from the group consisting of oxygen, sulfur and nitrogen which is unsubstituted or substituted by halogen, cyano, nitro, a group -CO-R⁴, C₁-C₄-alkyl, C₁-C₄-haloalkyl, C₃-C₈-cycloalkyl, C₁-C₄-alkoxy, C₁-C₄-haloalkoxy, C₁-C₄-alkylthio, C₁-C₄-haloalkylthio, di-C₁-C₄-alkylamino, unsubstituted or halogen-, cyano-, nitro-, C₁-C₄-alkyl- or C₁-C₄-haloalkyl-substituted phenyl or an oxo group which may also be present in the tautomeric form as a hydroxyl group, or which together with a fused-on, unsubstituted or halogen-, cyano-, nitro-, C₁-C₄-alkyl or C₁-C₄-haloalkyl-substituted phenyl ring, a fused-on carbocycle or a fused-on, unsubstituted or halogen-, cyano-, nitro-, C₁-C₄-alkyl-, di-C₁-C₄-alkylamino-, C₁-C₄-alkoxy-, C₁-C₄-haloalkoxy- or C₁-C₄-haloalkyl-substituted second heterocycle forms a tricyclic system;
Y is O, NR⁵;
n is zero or one;
m is zero, one or two;
R³ is C₁-C₄-alkyl, C₁-C₄-haloalkyl or NR⁵R⁶;
R⁴ is C₁-C₄-alkyl, C₁-C₄-haloalkyl, C₁-C₄-alkoxy or NR⁵R⁶;
R⁵ is hydrogen or C₁-C₄-alkyl;
R⁶ is C₁-C₄-alkyl;
which comprises treating a pyrazolylbenzoyl derivative of the formula II 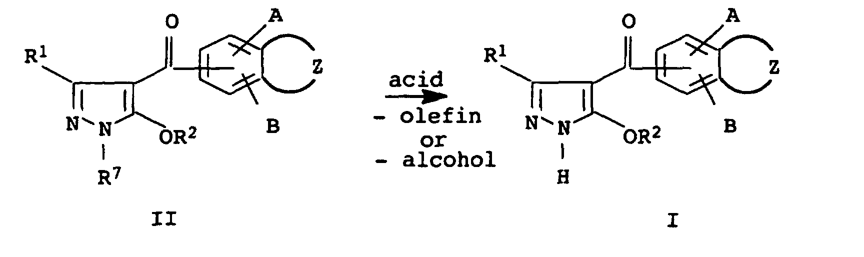 where the substituents R¹, R², A, B and Z are as defined above and
R⁷ is C₃-C₁₂-alkyl, C₃-C₁₂-alkenyl, C₄-C₁₂-alkynyl, branched in the α position, which are unsubstituted or substituted by halogen or C₁-C₄-alkoxy, or is benzyl which is unsubstituted or substituted by halogen, cyano, nitro, C₁-C₄-haloalkyl, C₁-C₄-alkylsulfonyl or C₁-C₄-alkylcarbonyl
with an inorganic or organic acid at a pH < 2 and reacting it with elimination of an olefin or an alcohol.

2. A process as claimed in claim 1, wherein the pyrazolylbenzoyl derivative of the formula II where R² = hydrogen is prepared by reacting the pyrazole of the formula III 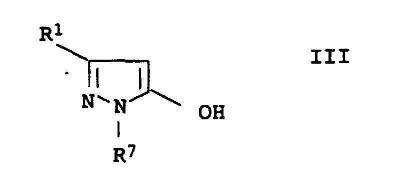 in which the substituents R¹ and R⁷ are as defined in claim 1 with a benzoyl derivative of the formula IV 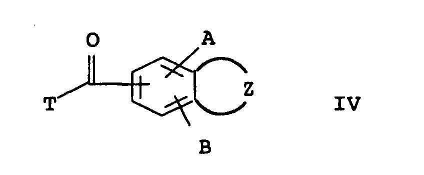 in which T = halogen and A, B and Z are as defined in claim 1 and the subsequent rearrangement of the acylation product V 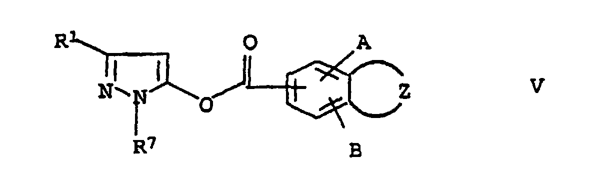 in the presence of a catalyst.

3. A process as claimed in claim 1 or 2, wherein the substituent R⁷ is C₃-C₆-alkyl which is branched in the α position.

4. A process as claimed in claim 1 or 2, wherein the substituent R⁷ is benzyl, 4-chlorobenzyl, 4-cyanobenzyl, 4-nitrobenzyl, 4-trifluoromethylbenzyl, 4-methylsulfonylbenzyl or 4-acylbenzyl.

5. A process as claimed in claim 4, wherein the substituent R⁷ is benzyl, 4-chlorobenzyl or 4-nitrobenzyl.

6. A process as claimed in claim 1, wherein the organic acid used is trifluoromethanesulfonic acid or trichloroacetic acid.

7. A process as claimed in claim 1, wherein the inorganic acid used is sulfuric acid, nitric acid, hydrochloric acid or hydrobromic acid.

8. A process as claimed in claim 1, wherein the reaction is carried out in a solvent.

9. A process as claimed in claim 8, wherein the solvent used is acetonitrile, DMF, dioxane, tetrahydrofuran, toluene or chlorobenzene.

10. A pyrazol-4-ylbenzoyl derivative of the formula I as claimed in claim 1 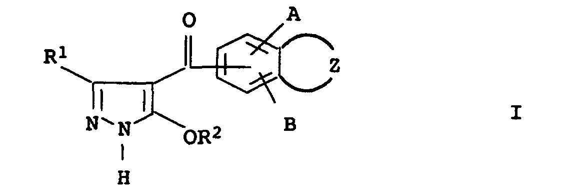 where R¹, R², A, B and Z have the meaning given in claim 1.

11. A pyrazol-4-ylbenzoyl derivative of the formula I as claimed in claim 10 in which the substituent Z is a 5- or 6-membered heterocyclic, saturated or unsaturated fused-on ring, so that, with the fused-on phenyl ring, the following bicyclic heterocycles result:
benzofuranyl, isobenzofuranyl, benzothienyl, isobenzothienyl, thionaphthalenyl, isothionaphthalenyl, indolyl, isoindolyl, indazolyl, indoleninyl, isobenzazolyl, pyranopyrrolyl, isoindazolyl, indoxazinyl, benzoxazolyl, benzothiazolyl, benzothiazolyl S,S-dioxide, benzopyranyl, quinolinyl, isoquinolinyl, phthalazinyl, quinoxalinyl, quinazolinyl, naphthyridinyl, pyridopyridinyl, pyranyl, thiopyranyl, chromanyl, thiachromanyl, chromenyl, thiachromenyl, benzoxazinyl and benzisoxazinyl.

12. A pyrazol-4-ylbenzoyl derivative of the formula VI as claimed in claim 10 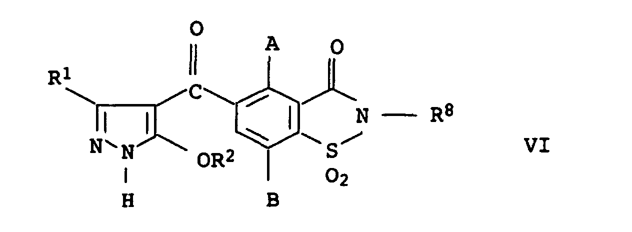 in which the substituents R¹, R², A and B are as defined in claim 10 and R⁸ is hydrogen, C₁-C₄-alkyl, C₁-C₄-alkoxy or C₁-C₄-haloalkyl.

13. A pyrazol-4-ylbenzoyl derivative of the formula VII as claimed in claim 10, 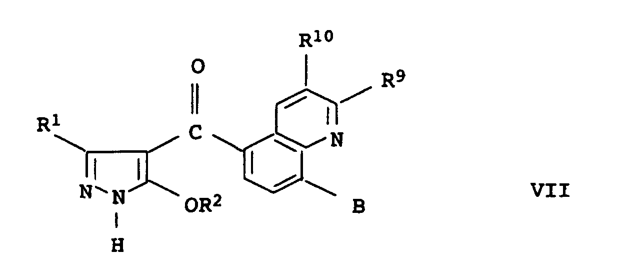 in which the substituents R¹, R² and B are as defined in claim 10 and R⁹, R¹⁰ independently of one another are hydrogen, C₁-C₄-alkyl, C₁-C₄-alkoxy or C₁-C₄-haloalkyl.

14. A pyrazol-4-ylbenzoyl derivative of the formula VIII as claimed in claim 10 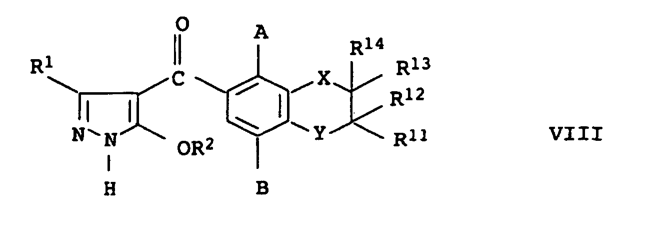 in which the substituents R¹, R², A and B are as defined above and
R¹¹, R¹², R¹³, R¹⁴ independently of one another are hydrogen, C₁-C₄-alkyl, C₁-C₄-alkoxy or C₁-C₄-haloalkyl;
R¹¹, R¹³ or R¹¹, R¹⁴ or R¹², R¹³ or R¹², R¹⁴ can form a bond;
X, Y independently of one another are S(O)ₙ, C=O, CR¹⁵R¹⁶, NR¹⁷;
R¹⁵, R¹⁶ independently of one another are hydrogen,
C₁-C₄-alkyl, C₁-C₄-alkoxy, C₁-C₄-haloalkyl or C₁-C₄-haloalkoxy;
R¹⁵, R¹⁶ together can form a 5 to 6-membered carbocycle which can contain 2 oxygen atoms or sulfur atoms;
_{R}17 is hydrogen or C₁-C₄-alkyl;
n is 0, 1 or 2.

15. A pyrazol-4-ylbenzoyl derivative of the formula I as claimed in any of claims 10 to 14, in which the radicals R¹ and R² independently of one another are hydrogen or C₁-C₄-alkyl, A and B independently of one another are hydrogen, C₁-C₄-alkyl, C₁-C₄-alkoxy, C₁-C₄-alkylthio, C₁-C₄-alkylsulfonyl, C₁-C₄-haloalkyl, halogen, hydroxyl, cyano or nitro.

16. A pyrazol-4-ylbenzoyl derivative of the formula I as claimed in claim 14, in which the radicals A and B independently of one another are hydrogen, methyl, methoxy, methylthio, methylsulfonyl, trifluoromethyl, chlorine, cyano or nitro.

17. A pyrazol-4-ylbenzoyl derivative of the formula I as claimed in claim 14, in which the radicals R¹, R² are hydrogen and A and B independently of one another are hydrogen, methyl, methoxy, methylthio, methylsulfonyl or chlorine.

18. A pyrazol-4-ylbenzoyl derivative of the formula I as claimed in claim 14, in which the radicals R¹, R² are hydrogen, A is methyl or chlorine and B is methylthio or methylsulfonyl.

19. A herbicidal composition comprising at least one pyrazol-4-ylbenzoyl derivative of the formula I as claimed in claim 10 and inert additives.

20. A method for controlling undesirable vegetation, which comprises allowing a herbicidally effective amount of a pyrazol-4-ylbenzoyl derivative of the formula I as claimed in claim 10 to act on the plants or their habitat.

## Revendications

1. Procédé de préparation de dérivés pyrazolylbenzoyle de formule I 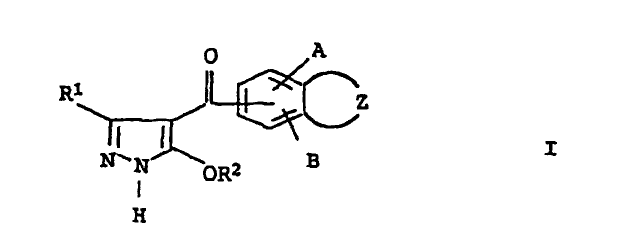 dans laquelle les substituants prennent la signification suivante :
R¹ représente un atome d'hydrogène, un groupe alkyle en C₁ à C₄, ou halogénoalkyle en C₁ à C₄;
R² représente un atome d'hydrogène;
un groupe alkyle en C₁ à C₆, alcényle en C₂ à C₆, alcynyle en C₂ à C₆, benzyle, benzoyle, (alkyle en C₁ à C₄)-carbonyle, hydroxycarbonyl-(alkyle en C₁ à C₄), (alcoxy en C₁ à C₄)-carbonyl-(alkyle en C₁ à C₄), alkylsulfonyle en C₁ à C₄, ou phénylsulfonyle, ces groupes pouvant être substitués par un groupe alkyle en C₁ à C₄, alcoxy en C₁ à C₄, alkylthio en C₁ à C₄, un atome d'halogène, un groupe hydroxy, amino, nitro, ou cyano;
A, B représentent indépendamment l'un de l'autre un atome d'hydrogène;
un groupe alkyle en C₁ à C₆, alcényle en C₂ à C₆, alcynyle en C₂ à C₆, alcoxy en C₁ à C₄, ces groupes pouvant être substitués par un atome d'halogène, un groupe hydroxy, alcoxy en C₁ à C₄, ou cyano;
un atome d'halogène, un groupe hydroxy, cyano, nitro, un groupe -(Y)ₙ-S(O)ₘR³ ou un groupe -(Y)ₙ-CO-R⁴;
z représente un cycle hétérocyclique à 5 ou 6 maillons condensés, saturé ou insaturé, contenant 1 à 3 hétéroatomes, choisis dans le groupe formé par l'atome d'oxygène, de soufre, ou d'azote, qui est substitué éventuellement par un atome d'halogène, un groupe cyano, nitro, un groupe -CO-R⁴, alkyle en C₁ à C₄, halogénoalkyle en C₁ à C₄, cycloalkyle en C₃ à C₈, alcoxy en C₁ à C₄, halogénoalcoxy en C₁ à C₄, alkylthio en C₁ à C₄, halogénoalkylthio en C₁ à C₄, di-(alkyle en C₁ à C₄)amino, éventuellement par un groupe phényle substitué par un atome d'halogène, un groupe cyano, nitro, alkyle en C₁ à C₄, ou halogénoalkyle en C₁ à C₄, ou un groupe oxo, qui peut exister également sous la forme tautomère en tant que groupe hydroxy, ou qui forme un système tricyclique avec un noyau phénylique condensé éventuellement substitué par un atome d'halogène, un groupe cyano, nitro, alkyle en C₁ à C₄, ou halogénoalkyle en C₁ à C₄, avec un carbocycle condensé, ou un deuxième hétérocycle condensé, éventuellement substitué par un atome d'halogène, un groupe cyano, nitro, alkyle en C₁ à C₄, di-(alkyle en C₁ à C₄)amino, alcoxy en C₁ à C₄, halogénoalcoxy en C₁ à C₄, ou halogénoalkyle en C₁ à C₄;
Y représente O, NR⁵;
n vaut zéro ou un;
m vaut zéro, un ou deux;
R³ représente un groupe alkyle en C₁ à C₄, halogénoalkyle en C₁ à C₄, ou NR⁵R⁶;
R⁴ représente un groupe alkyle en C₁ à C₄, halogénoalkyle en C₁ à C₄, alcoxy en C₁ à C₄, ou NR⁵R⁶;
R⁵ représente un atome d'hydrogène ou un groupe alkyle en C₁ à C₄;
R⁶ représente un groupe alkyle en C₁ à C₄;
**caractérisé en ce que** l'on traite un dérivé pyrazolyl-benzoyle de formule II 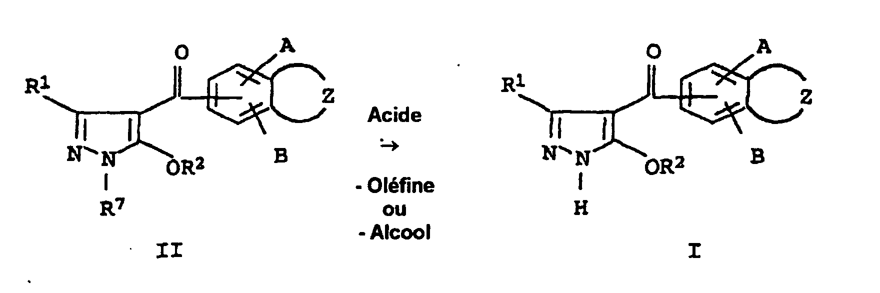 dans laquelle les substituants R¹, R², A, B, et Z prennent la signification indiquée ci-dessus et
R⁷ dans la position α représente un groupe alkyle en C₃ à C₁₂, alcényle en C₃ à C₁₂, alcynyle en C₄ à C₁₂, ramifiés qui sont substitués éventuellement par un atome d'halogène ou un groupe alcoxy en C₁ à C₄, ou un groupe benzyle qui est substitué éventuellement par un atome d'halogène, un groupe cyano, nitro, halogénoalkyle en C₁ à C₄, alkylsulfonyle en C₁ à C₄, ou (alkyle en C₁ à C₄)-carbonyle,
avec un acide inorganique ou organique à une valeur de pH < 2 et que l'on met à réagir avec clivage d'une oléfine ou d'un alcool.

2. Procédé selon la revendication 1, **caractérisé en ce que**, l'on prépare le dérivé pyrazolylbenzoyle de formule II avec R² = un atome d'hydrogène par réaction des pyrazoles de formule III 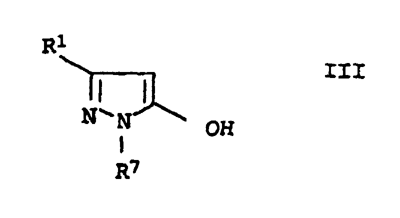 dans laquelle les substituants R¹ et R⁷ prennent la signification indiquée dans la revendication 1, avec un dérivé benzoyle de formule IV 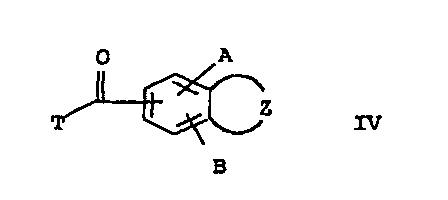 dans laquelle T représente un atome d'halogène et A, B et Z prennent la signification citée dans la revendication 1, suivi par un réarrangement du produit d'acylation V 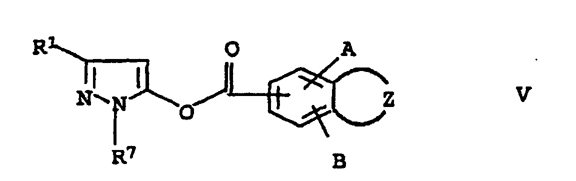 en présence d'un catalyseur.

3. Procédé selon la revendication 1 ou 2, **caractérisé en ce que** le substituant R⁷ dans la position α représente un groupe alkyle en C₃ à C6, ramifié.

4. Procédé selon la revendication 1 ou 2, **caractérisé en ce que** le substituant R⁷ représente un groupe benzyle, 4-chlorobenzyle, 4-cyanobenzyle, 4-nitrobenzyle, 4-trifluorométhylbenzyle, 4-méthylsulfonylbenzyle, ou 4-acylbenzyle.

5. Procédé selon la revendication 4, **caractérisé en ce que** le substituant R⁷ représente un groupe benzyle, 4-chlorobenzyle, ou 4-nitrobenzyle.

6. Procédé selon la revendication 1, **caractérisé en ce que** l'on emploie en tant qu'acide organique, l'acide trifluorométhanesulfonique ou l'acide trichloracétique.

7. Procédé selon la revendication 1, **caractérisé en ce que** l'on emploie en tant qu'acide inorganique, l'acide sulfurique, l'acide nitrique, l'acide chlorhydrique ou l'acide bromhydrique.

8. Procédé selon la revendication 1, **caractérisé en ce que** l'on réalise la réaction dans un solvant.

9. Procédé selon la revendication 8, **caractérisé en ce que** l'on emploie en tant que solvant, l'acétonitrile, le DMF, le dioxane, le tétrahydrofurane, le toluène ou le chlorobenzène.

10. Dérivés pyrazol-4-yl-benzoyle de formule I 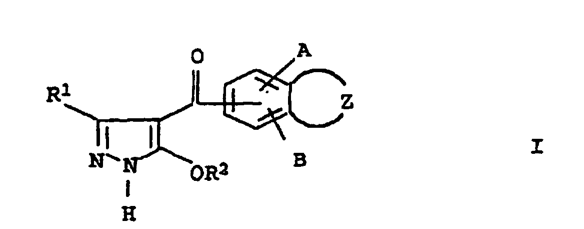 dans laquelle les substituants R¹, R², A, B et Z prennent la signification indiquée dans la revendication 1.

11. Dérivés pyrazol-4-yl-benzoyle de formule I selon la revendication 10, dans laquelle le substituant Z est un cycle condensé, saturé ou insaturé, hétérocyclique à 5 ou 6 maillons, de sorte qu'avec le noyau phényle condensé, il en résulte les hétérocycles bicycliques suivants :
benzofuranyle, isobenzofuranyle, benzothiényle, isobenzothiényle, thionaphtalényle, isothionaphtalényle, indolyle, isoindolyle, indazolyle, indolénynyle, isobenzazolyle, pyranopyrrolyle, isoindazolyle, indoxazinyle, benzoxazolyle, benzothiazolyle, benzo-thiazolyl-S,S-dioxide, benzopyranyle, quinoléinyle, isoquinoléinyle, phtalazinyle, quinoxaléinyle, quinazoléinyle, naphtyridinyle, pyridopyridinyle, pyranyle, thiopyranyle, chromanyle, thiachromanyle, chroményle, thiachroményle, benzoxazinyle et
benzisoxazinyle.

12. Dérivés pyrazol-4-yl-benzoyle de formule VI selon la revendication 10 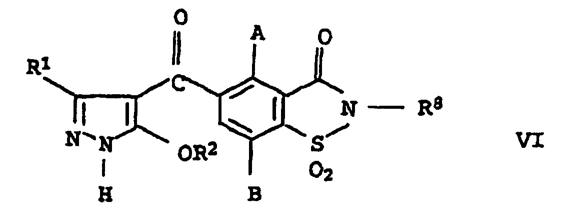 dans laquelle les substituants R¹, R², A, et B prennent les significations indiquées dans la revendication 10 et R⁸ représente un atome d'hydrogène, un groupe alkyle en C₁ à C₄, alcoxy en C₁ à C₄, ou halogénoalkyle en C₁ à C₄.

13. Dérivés pyrazol-4-yl-benzoyle de formule VII selon la revendication 10, 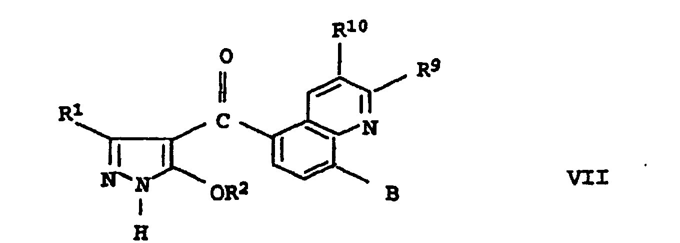 dans laquelle les substituants R¹, R², et B prennent la signification indiquée dans la revendication 10, et R⁹, R¹⁰ représentent indépendamment l'un de l'autre un atome d'hydrogène, un groupe alkyle en C₁ à C₄, alcoxy en C₁ à C₄, ou halogénoalkyle en C₁ à C₄.

14. Dérivés pyrazol-4-yl-benzoyle de formule VIII selon la revendication 10, 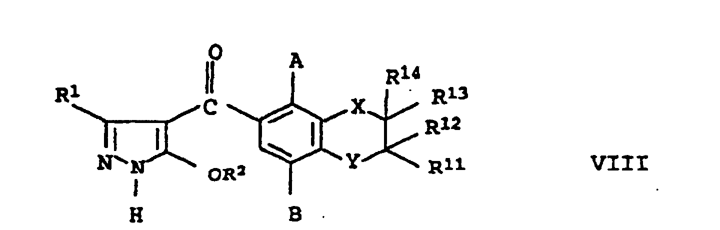 dans laquelle les substituants R¹, R², A et B prennent la signification précitée et
R¹¹, R¹², R¹³, R¹⁴ représentent indépendamment l'un de l'autre un atome d'hydrogène, un groupe alkyle en C₁ à C₄, alcoxy en C₁ à C₄, ou halogénoalkyle en C₁ à C₄;
R¹¹, R¹³ ou R¹¹, R¹⁴ ou R¹², R¹³, ou R¹², R¹⁴ peuvent former une liaison;
X, Y représentent indépendamment l'un de l'autre S(O)ₙ, C=O, CR¹⁵R¹⁶, NR¹⁷;
R¹⁵, R¹⁶ représentent indépendamment l'un de l'autre un atome d'hydrogène, un groupe alkyle en C₁ à C₄, alcoxy en C₁ à C₄, halogénoalkyle en C₁ à C₄, ou halogénoalcoxy en C₁ à C₄;
R¹⁵, R¹⁶ peuvent former ensemble un carbocycle de 5 à 6 maillons, qui peut contenir 2 atomes d'oxygène ou atomes de soufre;
R¹⁷ représente un atome d'hydrogène, ou un groupe alkyle en C₁ à C₄;
n vaut 0, 1 ou 2.

15. Dérivés pyrazol-4-yl-benzoyle de formule I selon l'une quelconque des revendications 10 à 14, dans laquelle les résidus R¹ et R² représentent indépendamment l'un de l'autre, un atome d'hydrogène, ou un groupe alkyle en C₁ à C₄, A et B représentent indépendamment l'un de l'autre un atome d'hydrogène, un groupe alkyle en C₁ à C₄, alcoxy en C₁ à C₄, alkylthio en C₁ à C₄, alkylsulfonyle en C₁ à C₄, halogénoalkyle en C₁ à C₄, un atome d'halogène, un groupe hydroxy, cyano ou nitro.

16. Dérivés pyrazol-4-yl-benzoyle de formule I selon la revendication 14, dans laquelle les résidus A et B représentent indépendamment l'un de l'autre, un atome d'hydrogène, un groupe méthyle, méthoxy, méthylthio, méthylsulfonyle, trifluorométhyle, un atome de chlore, un groupe cyano ou nitro.

17. Dérivés pyrazol-4-yl-benzoyle de formule I selon la revendication 14, dans laquelle les résidus R¹, R² représentent un atome d'hydrogène, et A et B représentent indépendamment l'un de l'autre, un atome d'hydrogène, un groupe méthyle, méthoxy, méthylthio, méthylsulfonyle ou un atome de chlore.

18. Dérivés pyrazol-4-yl-benzoyle de formule I selon la revendication 14, dans laquelle les résidus R¹, R² représentent un atome d'hydrogène, A représente un groupe méthyle ou un atome de chlore et B représente un groupe méthylthio ou méthylsulfonyle.

19. Agent herbicide, contenant au moins un dérivé pyrazol-4-yl-benzoyle de formule I selon la revendication 10 et des additifs inertes.

20. Procédé pour lutter contre un développement non souhaité de plantes, **caractérisé en ce que** l'on laisse agir une quantité efficace en tant qu'herbicide d'un dérivé pyrazol-4-yl-benzoyle de formule I selon la revendication 10 sur les plantes ou leur espace vital.
